# EUROPEAN PATENT APPLICATION

(11) **EP 4 378 928 A1**
(43) Date of publication of application: **05.06.2024**
(21) Application number: 22878320.5
(22) Date of filing: 21.09.2022
(51) Int. Cl.: C07C 321/26, C07C 319/14, C07C 321/28, C07C 323/21, C07C 323/62, C07C 323/64, C07C 327/22, C07D 303/34, C08F 20/38, C08F 28/04, C08G 59/30, C08G 59/40, C08G 75/0204, C08K 5/36, C08L 101/00

(54) **NAPHTHALENE DITHIOL AND DERIVATIVE THEREOF, AND PRODUCTION METHODS AND USES FOR SAME**

(30) Priority: 05.10.2021 JP 2021164338; 14.04.2022 JP 2022067062
(71) Applicant: SUGAI CHEMICAL INDUSTRY CO., LTD., Wakayama-shi, Wakayama 6410043 (JP)
(72) Inventor: MINEYAMA Kenji, Wakayama-shi, Wakayama 641-0043 (JP); KAZEKAMI Yutaka, Wakayama-shi, Wakayama 641-0043 (JP); KONO Ryota, Wakayama-shi, Wakayama 641-0043 (JP)
(74) Representative: Grünecker Patent- und Rechtsanwälte PartG mbB
(86) International application number: PCT/JP2022/035048
(87) International publication number: WO 2023/058449

(57) **Abstract**

Provided are a naphthalenedithiol or a derivative thereof that can achieve both high refractive index and high dissolubility (compatibility), and a process for producing the same and a use therefor. A naphthalene compound of the present invention is represented by the formula (1): 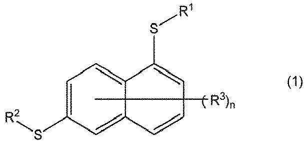 wherein R¹ and R² independently represent a hydrogen atom or a substituent, R³ represents a substituent, and n denotes an integer of 0 to 6.

At least one of R¹ and R² may represent the substituent. The naphthalene compound of the formula (1) may be dissolved in or mixed with an organic solvent to prepare a mixture (or blend); a curable composition containing the naphthalene compound of the formula (1) may be prepared; a resin of the present invention at least contains a constituent unit represented by the formula (1P): 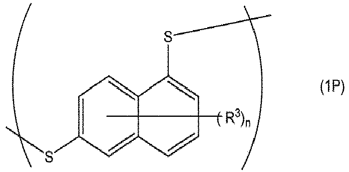 wherein R³ represents a substituent, and n denotes an integer of 0 to 6.

## Description

### TECHNICAL FIELD

The present invention relates to a naphthalene compound having sulfur atoms bonded to the 1,6-positions (a 1,6-naphthalenedithiol and a derivative thereof), and a production process and a use or an application for the same.

### BACKGROUND ART

A naphthalenedithiol or a derivative thereof is used as a functional material, or a raw material or a reaction intermediate thereof in various fields including an optical material, an electric and electronic material, and a medicine.

For example, Japanese Patent Application Laid-Open Publication No. 2008-527413 (JP 2008-527413 A, Patent Document 1) discloses a display including a light transmissible substrate and a specified hardcoat layer bonded to the substrate, and discloses that examples of an aromatic sulfur (meth)acrylate monomer as a material of the hardcoat layer include compounds represented by the following formulae:

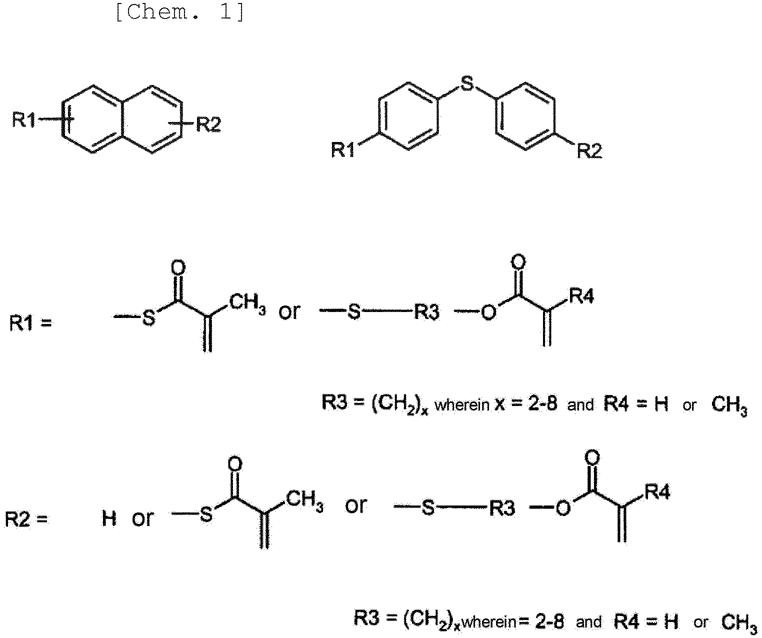

Example 12 of the Patent Document 1 specifically describes the preparation of the following compounds (9) to (11), each having sulfur atoms bonded to a naphthalene skeleton thereof at the 2,7-positions.

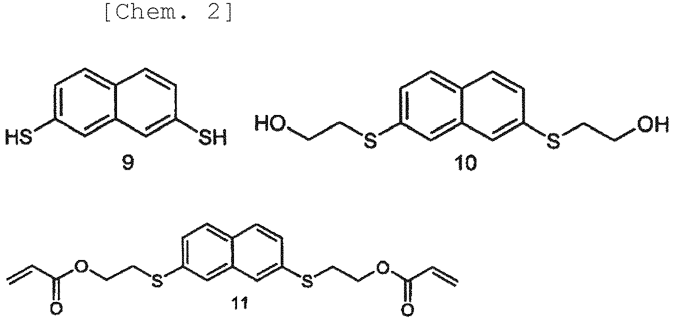

Japanese Patent Application Laid-Open Publication No. H6-256299 (JP H6-256299 A, Patent Document 2) discloses a process for producing a thio(meth)acrylate compound represented by the following general formula (I) and discloses that used as a material for preparing the thio(meth)acrylate compound is a thiol compound represented by the following general formula (II): 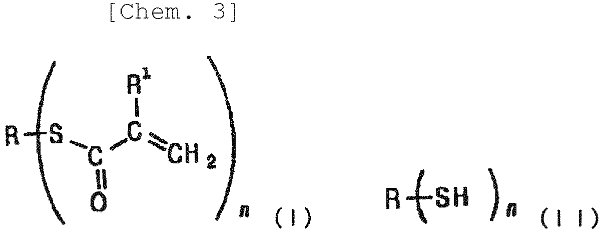 wherein R represents an organic residue, R¹ represents a hydrogen atom or a methyl group, and n denotes an integer of 1 to 10.

The Patent Document 2 describes naphthalenedithiols each having mercapto groups on 1,4-positions, 1,5-positions, 2,6-positions, or 2,7-positions as the thiol compound represented by the formula (II). However, thiol compounds used in Examples of the document are quite different from these naphthalenedithiols.

Japanese Patent Application Laid-Open Publication Nos. 2004-536933 (JP 2004-536933 A, Patent Document 3) and 2004-536934 (JP 2004-536934 A, Patent Document 4) disclose a specified polymerizable composition containing a thio(meth)acrylate functional monomer, and describe, as the thio(meth)acrylate functional monomer, a compound represented by the following general formula: 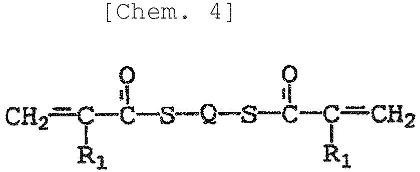 wherein R₁ represents a hydrogen or a methyl, Q represents a divalent linking group selected from a straight chain or branched C₂ to C₁₂ alkylene, a C₄ to C₁₂ cyclic alkylene, a C₆ to C₁₄ arylene, and a C₇ to C₂₆ alkarylene, and the carbon chain of Q may optionally contain at least one linkage selected from the group consisting of an ether, a thioether, and a combination thereof.

The Patent Documents 3 and 4 disclose that examples of the C₆ to C₁₄ arylene represented by Q in the general formula include 1,4-, 1,5-, 2,6-, and 2,7-naphthalenylenes, although a compound in which Q is 1,2-ethylene is used in Examples.

"Cross-Linked Polythiomethacrylate Esters Based on Naphthalene-Synthesis, Properties and Reprocessing", Materials, 2020, Vol. 13, issue 13, 3021 (Nonpatent Document 1) discloses S,S'-naphthalene-1,5-diyl bis(2-methyl prop-2-enethioate) [1,5-NAF-S-Met] represented by the following formula, and a copolymer thereof [a copolymer with methyl (meth)acrylate (MMA) or styrene (ST)].

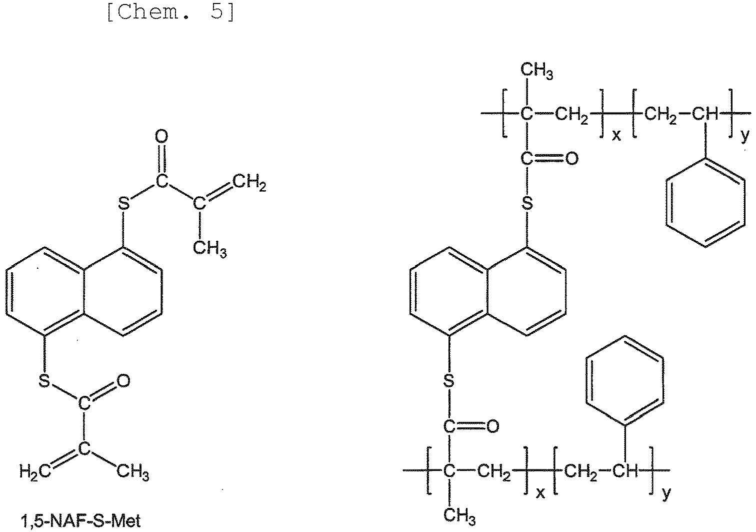

U.S. Patent No. 2463219 specification (Patent Document 5) discloses a method for improving a millability of a synthetic elastomer, the method comprising adding an arylmercaptan and oxygen at a specific ratio to a latex of a synthetic elastomer containing a 1,3-butadiene-styrene copolymer prepared by emulsion polymerization. This document describes various thiol compounds exemplified as the arylmercaptan (a benzene- and naphthalene-based mercaptan), including 2,5-dimercapto-naphthalene.

U.S. Patent No. 9170495 specification (Patent Document 6) discloses that a phenol monomer represented by the following formula 1 for forming a resist under-layer is synthesized according to the following reaction scheme: 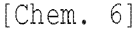 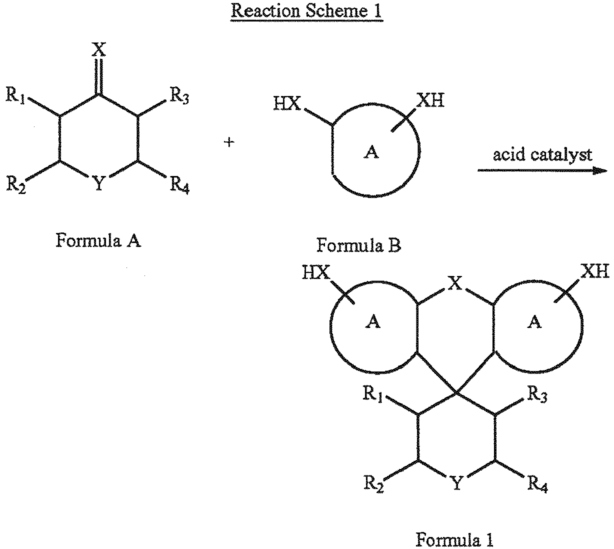 wherein R₁, R₂, R₃, and R₄ independently represent a hydrogen atom or a specified C₁₋₂₀hydrocarbon group, A represents a monocyclic or polycyclic C₄₋₂₀aromatic hydrocarbon group, X represents an oxygen atom or a sulfur atom, and Y represents a single bond or others.

This document describes Synthesis Examples 10 and 11, in which 9-fluorenone or thioxanthone is allowed to react with naphthalene-1,6-dithiol in the presence of sulfuric acid as an acid catalyst to synthesize the corresponding phenol monomer.

"Orbital Views of the Electron Transport in Molecular Devices", Journal of the American Chemical Society, 2008, Vol. 130, issue 29, p. 9406-9413 (Nonpatent Document 2), which describes electron transport properties of π-conjugated molecules, discloses that qualitative predictions based on frontier orbital analysis about the site-dependent electron transport in naphthalene, phenanthrene, and anthracene are compared with density functional theory calculations for the molecular junctions of their dithiolate derivatives, in which two gold electrodes have strong contact with a molecule through two Au-S bonds. This document describes 1,4-, 1,5-, 2,6-, and 2,7-naphthalenedithiolates as examples of the dithiolate derivatives.

"Three Consecutive Allylic Sigmatropic [S-O, S-S, S-C] Rearrangements of 1,8-Bis(Allylthio)Naphthalene Monooxides Via Transannular Interaction", Phosphorus, Sulfur, and Silicon and the Related Elements, 1994, Vol. 95, issue 1-4, p. 385-386 (Nonpatent Document 3) and "Rearrangements of the Allyl Group in the Thermolysis of 1,8-Bis(allylthio)- and 1,8-Bis(allylseleno)naphthalene Monooxides via Through-space Interaction between Two Sulfur and Selenium Atoms", Tetrahedron, 1995, Vol. 51, issue 45, p. 12239-12256 (Nonpatent Document 4) disclose 1,8-bis(allylthio)naphthalene or a monoxide thereof.

"Thioether Glycidyl Resins. V. Products of Condensation of 1,4-, 1,5-Di(mercapto)naphthalene and 4,4'-Di(mercapto)diphenyl with Epichlorohydrin", Journal of Applied Polymer Science, 1985, Vol. 30, issue 1, p. 411-421 (Nonpatent Document 5) discloses that 1,4- or 1,5-dimercaptonaphthalene (1,4- or 1,5-DMN) is allowed to react with epichlorohydrin to synthesize 1,4- or 1,5-di(glycidylthio)naphthalene (1,4- or 1,5-DGTN) represented by the following formulae and that 1,4- and 1,5-DGTN, and cured products thereof are evaluated for their thermal or heat characteristics and mechanical characteristics.

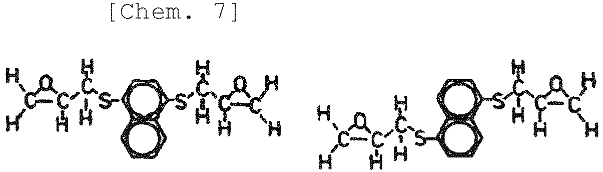

"Studies on Chemotherapeutics for Acid-fast Bacilli. XIII. On Some Compounds Related to Vinyl Sulfone and their Bacteriostatic and Chemical Activities. (2)", YAKUGAKU ZASSHI, 1955, Vol. 75, issue 12, p. 1560-1564 (Nonpatent Document 6) discloses that aliphatic or aromatic compounds each having two β-chloroethylsulfonyl groups are synthesized and are examined for their antibacterial properties. Fig. 1 of this document shows that, as an example of the above-mentioned compounds, 1,5-bis(β-chloroethylsulfonyl)naphthalenes each having β-chloroethylsulfonyl groups on the 1,5-positions of the naphthalene nucleus are prepared or synthesized via 1,5-dimercaptonaphthalene, 1,5-bis(β-hydroxyethylthio)naphthalene, and 1,5-bis(β-chloroethylthio)naphthalene as raw material(s) or reaction intermediate(s).

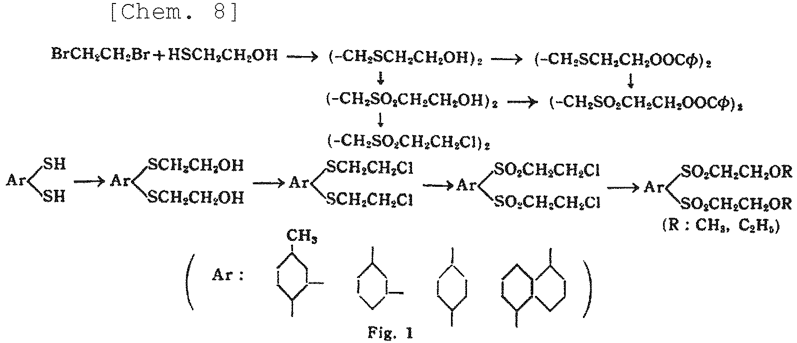

"Study of Energy Migration and Trapping in a Poly(ethylene 2,6-naphthalenedicarboxylate) Matrix by Fluorescence Spectroscopy", Macromolecules, 2000, Vol. 33, issue 17, p. 6344-6352 (Nonpatent Document 7) discloses that the efficiency of quenchers quenching the fluorescence of a poly(ethylene-2,6-naphthalenedicarboxylate) (PEN) matrix are investigated and that 2,6-bis(2-hydroxyethylthio)naphthalene (NSEG) and others are used as the quenchers and NSEG quenches PEN fluorescence best.

Japanese Patent Application Laid-Open Publication No. H10-511412 (JP H10-511412 A, Patent Document 7) discloses a composition containing an aromatic thioether compound represented by the following formula in order to quench fluorescence in PEN-containing polymers without deleteriously affecting the physical properties of the PEN-containing polymers:

Ar(SR)ₙ

wherein n denotes 1 or more; R represents a group such as -L-X, wherein L represents an organic divalent linking group and X represents a polyester reactive group; and Ar represents a group such as benzene and naphthalene.

This document discloses that a preferred aromatic thioether compound is a compound in which Ar represents naphthalene and which contains two polymerizable groups X, wherein L represents ethylene or an arylene (in particular, 1,2-, 1,3-, and 1,4-phenylene) and X is a group selected from hydroxy, carboxy, and other groups. Examples 4 to 7 of the document disclose specific preparations of aromatic thioether compounds each having specified groups at the 2,6-positions of naphthalene as shown below.

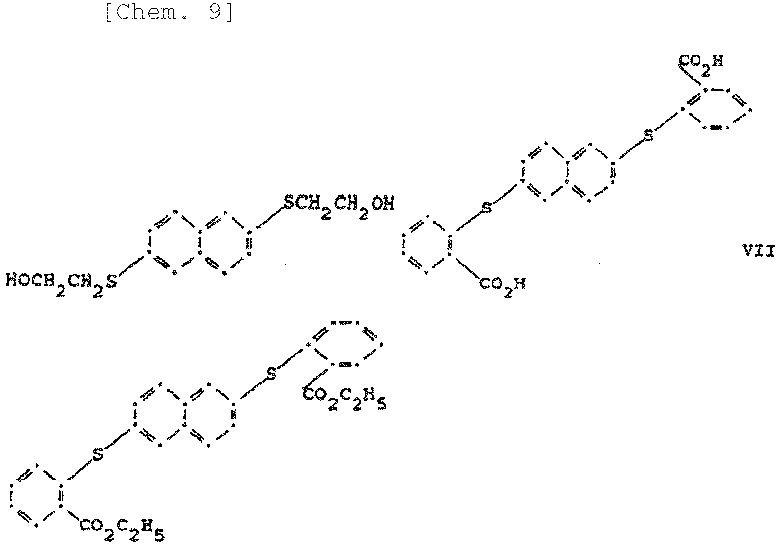

### CITATION LIST

### PATENT LITERATURE

Patent Document 1: JP 2008-527413 A (Claims 1 and 6, [0031], [0038], and Example 12)
Patent Document 2: JP H6-256299 A (Claim 1, [0007], and Examples)
Patent Document 3: JP 2004-536933 A (Claim 1, [0023], and Examples)
Patent Document 4: JP 2004-536934 A (Claim 1, [0019], and Examples)
Patent Document 5: U.S. Patent No. 2463219 specification (Claim 1, and column 5, line 12)
Patent Document 6: U.S. Patent No. 9170495 specification (Reaction Scheme 1, and Synthesis Examples 10 and 11)
Patent Document 7: JP H10-511412 A (Claims, Background of the Invention, the 9th to the 3rd lines from the bottom of page 14, and Examples 4 to 7)

### NONPATENT LITERATURE

Nonpatent Document 1: Karolina Fila and two others, "Cross-Linked Polythiomethacrylate Esters Based on Naphthalene-Synthesis, Properties and Reprocessing", Materials, 2020, Vol. 13, issue 13, 3021 (Figures 1 and 2, Table 1, and others)
Nonpatent Document 2: Kazunari Yoshizawa and two others, "Orbital Views of the Electron Transport in Molecular Devices", Journal of the American Chemical Society, 2008, Vol. 130, issue 29, p. 9406-9413 (Figure S1, Tables S1-S4, and others)
Nonpatent Document 3: Naomichi Furukawa and one other, "Three Consecutive Allylic Sigmatropic [SO, S-S, S-C] Rearrangements of 1,8-Bis(Allylthio)Naphthalene Monooxides Via Transannular Interaction", Phosphorus, Sulfur, and Silicon and the Related Elements, 1994, Vol. 95, issue 1-4, p. 385-386
Nonpatent Document 4: Hidetaka Shima and one other, "Rearrangements of the Allyl Group in the Thermolysis of 1,8-Bis(allylthio)-and 1,8-Bis(allylseleno)naphthalene Monooxides via Through-space Interaction between Two Sulfur and Selenium Atoms", Tetrahedron, 1995, Vol. 51, issue 45, p. 12239-12256
Nonpatent Document 5: Wladyslaw Charmas, "Thioether Glycidyl Resins. V. Products of Condensation of 1,4-, 1,5-Di(mercapto)naphthalene and 4,4'-Di(mercapto)diphenyl with Epichlorohydrin", Journal of Applied Polymer Science, 1985, Vol. 30, issue 1, p. 411-421
Nonpatent Document 6: Toshio Nambara, "Studies on Chemotherapeutics for Acid-fast Bacilli. XIII. On Some Compounds Related to Vinyl Sulfone and their Bacteriostatic and Chemical Activities. (2)", YAKUGAKU ZASSHI, 1955, Vol. 75, issue 12, p. 1560-1564 (Fig. 1, Experimental, and others)
Nonpatent Document 7: Jean Duhamel and two others, "Study of Energy Migration and Trapping in a Poly(ethylene 2,6-naphthalenedicarboxylate) Matrix by Fluorescence Spectroscopy", Macromolecules, 2000, Vol. 33, issue 17, p. 6344-6352

### SUMMARY OF INVENTION

### TECHNICAL PROBLEM

The inclusion of a fused or condensed polycyclic arene ring such as a naphthalene ring in a chemical structure typically tends to decrease a dissolubility (or solubility) while increasing a refractive index; none of the above-mentioned documents also discloses or suggests that a naphthalenedithiol and a derivative thereof have both high refractive index and high dissolubility (or compatibility) [in particular, a dissolubility in an organic compound (such as an organic solvent and another reaction component such as a copolymerizable component) or a resin (or a polymer)].

The Nonpatent Document 6 suggests that difunctional compounds derived from 1,5-naphthalenedithiol and others each have significantly decreased dissolubility.

It is therefore an object of the present invention to provide a naphthalenedithiol or a derivative thereof having both a high refractive index and a high dissolubility (compatibility), and a production process and a use for the same.

### SOLUTION TO PROBLEM

The inventors of the present invention made intensive studies to achieve the above object and finally found that a compound having a chemical structure with sulfur atoms bonded to a naphthalene skeleton thereof at the 1,6-positions achieves both high refractive index and high dissolubility. The present invention was accomplished based on the above findings. The present invention may include the following aspects and other aspects.

The present invention includes aspect [1]: a naphthalene compound represented by the following formula (1): 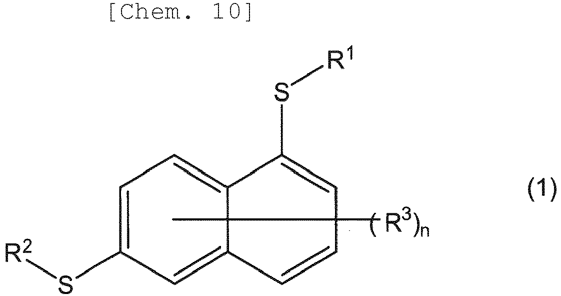
wherein R¹ and R² independently represent a hydrogen atom or a substituent, and
R³ represents a substituent, and n denotes an integer of 0 to 6.

Aspect [1-1]; In the aspect [1], at least one of R¹ and R² in the naphthalene compound may represent the substituent.

Aspect [2]; In the formula (1) in the aspect [1] or [1-1], each of R¹ and R² may represent at least one member selected from the group consisting of a hydrogen atom, a substituted or unsubstituted hydrocarbon group [a hydrocarbon group which may have a substituent (or which may be substituted)], and a group containing an element in group 16 of the Periodic Table, and
R³ may represent a hydrocarbon group, and n may denote an integer of 0 to 3.

Aspect [3]; In the formula (1) in the aspect [1], [1-1], or [2], at least one of R¹ and R² may represent at least one member selected from the group consisting of the groups defined by the following formulae (2B) to (2G): 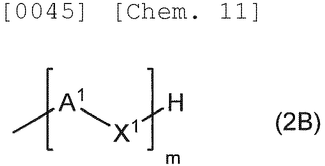
wherein A¹ represents an alkylene group, X¹ represents an oxygen atom or a sulfur atom, and m denotes 0 or an integer of not less than 1; 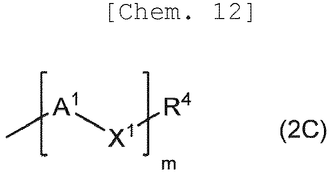
wherein A¹ represents an alkylene group, X¹ represents an oxygen atom or a sulfur atom, and m denotes 0 or an integer of not less than 1, and
R⁴ represents a saturated hydrocarbon group which may have a halogen atom (a saturated hydrocarbon group which may be substituted by a halogen atom); 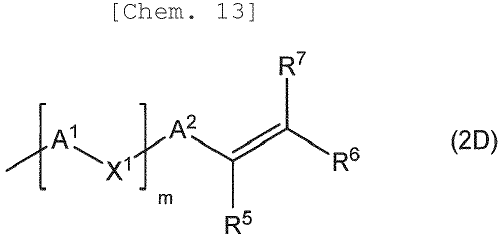
wherein A¹ represents an alkylene group, X⁴ represents an oxygen atom or a sulfur atom, and m denotes 0 or an integer of not less than 1,
A² represents a direct bond (a single bond) or an alkylene group, and
R⁵, R⁶ and R⁷ independently represent a hydrogen atom, or a substituted or unsubstituted hydrocarbon group [a hydrocarbon group which may have a substituent (or which may be substituted)]; 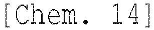 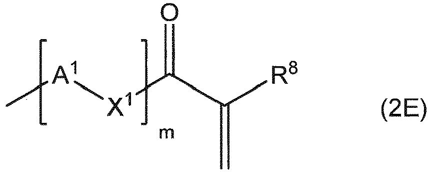
wherein A¹ represents an alkylene group, X¹ represents an oxygen atom or a sulfur atom, and m denotes 0 or an integer of not less than 1, and
R⁸ represents a hydrogen atom or a methyl group; 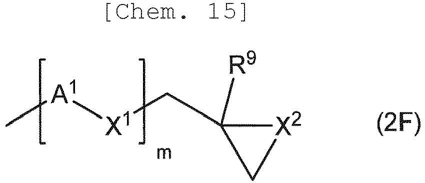
wherein A¹ represents an alkylene group, X¹ represents an oxygen atom or a sulfur atom, and m denotes 0 or an integer of not less than 1,
X² represents an oxygen atom or a sulfur atom, and
R⁹ represents a hydrogen atom or a methyl group; 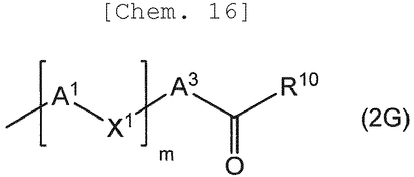
wherein A¹ represents an alkylene group, X¹ represents an oxygen atom or a sulfur atom, and m denotes 0 or an integer of not less than 1,
A³ represents an alkylene group, and
R¹⁰ represents a hydroxyl group, a group [-OR¹¹] [wherein R¹¹ represents a substituted or unsubstituted hydrocarbon group (a hydrocarbon group which may have a substituent or which may be substituted)], or a halogen atom.

Aspect [4]; In the formula (1) in the aspect [1], [1-1], [2], or [3], at least one of R¹ and R² may represent at least one member selected from the group consisting of a hydrogen atom, an alkyl group, a haloalkyl group, an alkenyl group (such as vinyl group and allyl group), (meth)acryloyl group, a (meth)acryloyloxyalkyl group, a glycidyl group, a β-methylglycidyl group, a glycidyloxyalkyl group, a β-methylglycidyloxyalkyl group, a hydroxyalkyl group, a carboxyalkyl group, an alkoxycarbonylalkyl group, and a halocarbonylalkyl group, and
R³ may represent an alkyl group, and n may denote an integer of 0 to 2.

Aspect [5]; The naphthalene compound in the aspect [1], [1-1], [2], [3], or [4] may have a refractive index of not less than 1.6 at a temperature of 25°C and a wavelength of 589 nm.

Aspect [6]; In the aspect [1], [1-1], [2], [3], [4], or [5], the naphthalene compound may be dissolvable at a concentration of about not less than 20% by mass at a temperature of 25°C in at least one organic solvent selected from the group consisting of toluene, tetrahydrofuran, propylene glycol monomethyl ether acetate, ethyl acetate, and N,N-dimethylformamide.

The present invention includes aspect [7]: a process for producing the naphthalene compound of the aspect [1], [1-1], [2], [3], [4], [5], or [6], the process comprising a step of replacing, with the substituent (the substituent corresponding to R¹ and/or R²), at least one hydrogen atom of mercapto groups on 1-position and 6-position of a naphthalene compound represented by the following formula (1A): 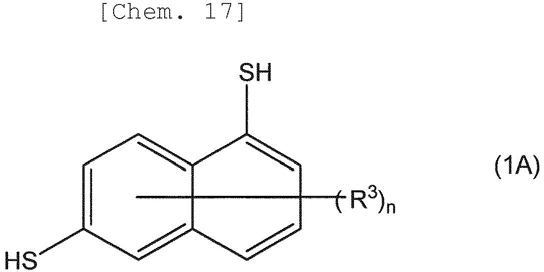 wherein R³ and n each have the same meanings as the formula (1).

The present invention also includes aspect [8]: a mixture (a blend, a solution, or a liquid composition) containing a naphthalene compound represented by the formula (1) of the aspect [1], [1-1], [2], [3], [4], [5], or [6], and an organic solvent.

Aspect [9]; In the aspect [8], the organic solvent may contain at least one member selected from the group consisting of a hydrocarbon, a halogenated hydrocarbon, an alcohol, an ether, a glycol ether acetate, an ester, an amide, and a urea.

Aspect [10]; In the aspect [8] or [9], the naphthalene compound may have a concentration of about not less than 15% by mass relative to the whole mixture.

The present invention further includes aspect [11]: a curable composition containing a naphthalene compound represented by the formula (1) of the aspect [1], [1-1], [2], [3], [4], [5], or [6].

Aspect [12]; In the aspect [11], the curable composition may contain the naphthalene compound in which at least one of R¹ and R² in the formula (1) represents a group defined by the formula (2D), (2E), or (2F).

Aspect [13]; In the aspect [11] or [12], the curable composition may contain the naphthalene compound in which at least one of R¹ and R² in the formula (1) represents, for example, at least one member selected from the group consisting of an alkenyl group (such as vinyl group and allyl group), (meth)acryloyl group, a (meth)acryloyloxyalkyl group, a glycidyl group, a β-methylglycidyl group, a glycidyloxyalkyl group, and a β-methylglycidyloxyalkyl group. Further, the curable composition may contain the naphthalene compound and a polymerizable compound having at least one ethylenically unsaturated bond.

Aspect [14]; In any of the aspects [11] to [13], the curable composition may at least contain the naphthalene compound in which both R¹ and R² in the formula (1) represent a group (preferably a hydrogen atom) defined by the formula (2B) or formula (2G), and an epoxy resin.

Aspect [15]; In any of the aspects [11] to [14], the naphthalene compound in the curable composition may have a concentration of about not less than 25% by mass relative to the whole curable composition.

The present invention also includes aspect [16]: a cured product of the curable composition recited in the aspect [11], [12], [14], or [15].

The present invention also includes aspect [17]: a cured product of the curable composition recited in the aspect [13].

The present invention includes aspect [18]: a curing agent for curing an epoxy resin, wherein the curing agent is represented by the formula (1) of the aspect [1], [1-1], [2], [3], [4], [5], or [6], wherein R¹ and R² independently represent a group defined by the formula (2B) or formula (2G) recited in the aspect [3], preferably a curing agent represented by the formula (1A).

The present invention also includes aspect [19]: a resin containing a constituent unit represented by the following formula (1P): 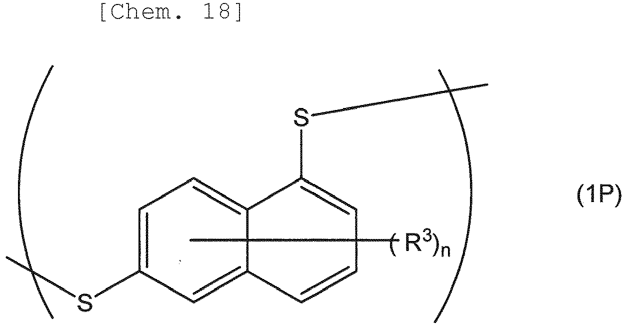 wherein R³ represents a substituent, and n denotes an integer of 0 to 6.

Aspect [20]; In the aspect [19], the resin may be a thermoplastic resin.

Aspect [21]; In the aspect [19] or [20], the resin may be a polymer of a polymerizable component at least containing a compound represented by the formula (1) of the aspect [1], in particular, a compound in which R¹ and R² independently represent a group defined by the formula (2B), (2D), (2E), (2F), or (2G) recited in the aspect [3], wherein the polymerizable component may be any of the following polymerizable components (a) to (c):
(a) a polymerizable component containing a dithiol component and a component having two ethylenically unsaturated bonds;
   wherein the dithiol component contains the compound in which each of R¹ and R² in the formula (1) represents a group (such as a hydrogen atom and a mercaptoalkyl group) defined by the formula (2B), where X¹ represents a sulfur atom, and/or
   the component having two ethylenically unsaturated bonds (such as a diallyl component, a di(meth)acrylate component, and a diene component) contains the compound in which each of R¹ and R² in the formula (1) represents a group defined by the formula (2D) or (2E),
(b) a polymerizable component containing a diol component and/or a dithiol component, and at least one component selected from the group consisting of a dicarboxylic acid component, a diisocyanate component, and a carbonyl component;
   wherein the diol component or the dithiol component contains the compound in which each of R¹ and R² in the formula (1) represents a group defined by the formula (2B), and/or
   the dicarboxylic acid component contains the compound in which each of R¹ and R² in the formula (1) represents a group defined by the formula (2G),
(c) a polymerizable component containing
   a diol component and/or a dithiol component containing the compound in which each of R¹ and R² in the formula (1) represents a group defined by the formula (2B), and
   an epihalohydrin component, or
   a polymerizable component containing
   an epoxy component containing the compound in which each of R¹ and R² in the formula (1) represents a group defined by the formula (2F), and
   a bisphenol component.

The present invention further includes aspect [22]: a method for improving a dissolubility or compatibility of a naphthalene compound in an organic compound (preferably a liquid organic compound), the naphthalene compound having sulfur atoms directly bonded to a naphthalene ring thereof at least at two positions, wherein the improvement comprises including the sulfur atoms at least at 1,6-positions (or preparing or adjusting the positions of the sulfur atoms so as to be at least the 1,6-positions), specifically a method for improving the dissolubility or compatibility, wherein the improvement comprises introducing a constituent unit represented by the formula (1P) in a chemical structure thereof.

Moreover, the present invention includes aspect [23]: a resin composition at least containing a compound represented by the formula (1) of the aspect [1], [1-1], [2], [3], [4], [5], or [6], and a resin.

Further, the present invention includes aspect [24]: a refractive index increasing agent for increasing (or improving) a refractive index, wherein the refractive index increasing agent is represented by the formula (1) of the aspect [1], [1-1], [2], [3], [4], [5], or [6] .

The present invention also includes aspect [25]: a method for increasing (or improving) a refractive index, which comprises adding the refractive index increasing agent of the aspect [24] to a resin.

In this description and claims, the term "(meth)acryloyl" is used as a general term for (or is used to mean both) "acryloyl" and "methacryloyl".

### ADVANTAGEOUS EFFECTS OF INVENTION

According to the present invention, the naphthalenedithiol or the derivative thereof has the chemical structure having sulfur atoms bonded to the naphthalene skeleton at the 1,6-positions and thus achieves both high refractive index and high dissolubility (or compatibility) [in particular, a dissolubility in an organic compound (such as an organic solvent, another reaction component such as a copolymerizable component, and a resin)]. In particular, the naphthalenedithiol or the derivative thereof is dissolvable (compatible) at a high concentration in various organic solvents, copolymerizable components, or resins. Thus, use of the naphthalenedithiol or the derivative thereof as a raw material (such as a polymerizable component) for a functional material (such as an optical material) or as a reaction intermediate allows the easy or efficient preparation of a homogeneous composition [such as a reaction solution, a reaction mixture or a reactive composition (such as a polymerizable composition or a curable composition), and a coating composition or a coating film (a coating)]. Further, use of the naphthalenedithiol or the derivative thereof itself as a functional material (such as an additive such as a fluorescence quencher, a refractive index increasing agent, a curing agent, a crosslinking agent, or a crosslinking co-agent) also allows the preparation of a homogeneous composition (such as a curable composition and a cured product thereof, a resin composition, and a molded product containing the composition(s)), since the naphthalenedithiol or the derivative thereof is easily or efficiently dissolvable in or compatible with a resin and others.

### DESCRIPTION OF EMBODIMENTS

### [Naphthalene compound represented by the formula (1)]

The naphthalene compound according to the present invention has a naphthalene-1,6-dithio skeleton [that is, a chemical structure represented by the above-mentioned formula (1P)] and is represented by the following formula (1): 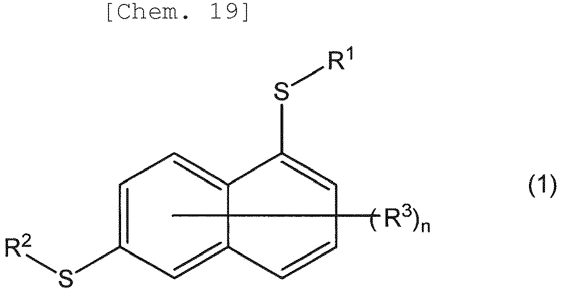
wherein R¹ and R² independently represent a hydrogen atom or a substituent, and
R³ represents a substituent, and n denotes an integer of 0 to 6.

In the formula (1), R¹ and R² each may represent a hydrogen atom, or at least one (particularly, both) of R¹ and R² may represent the substituent. A monovalent substituent represented by each of R¹ and R² is not particularly limited to a specific one, and examples of the monovalent substituent may include a substituted or unsubstituted hydrocarbon group [a hydrocarbon group which may have a substituent (or which may be substituted)], a heterocyclic group, and a group containing an element in group 16 of the Periodic Table.

Examples of the hydrocarbon group may include a (straight- or branched-chain) aliphatic hydrocarbon group, an alicyclic hydrocarbon group, an aromatic hydrocarbon group, and a group having two or more of these groups in combination.

The aliphatic hydrocarbon group may be a straight- or branched-chain group. Moreover, the aliphatic hydrocarbon group may be a saturated aliphatic hydrocarbon group such as an alkyl group or may be an unsaturated aliphatic hydrocarbon group such as an alkenyl group.

Examples of the alkyl group may include a C₁₋₃₀alkyl group (such as a C₁₋₂₄alkyl group) such as methyl group, ethyl group, propyl group (n-propyl group, isopropyl group), butyl group (n-butyl group, isobutyl group, sec-butyl group, tert-butyl group), pentyl group (such as n-pentyl group, isopentyl group, and neopentyl group), hexyl group (such as n-hexyl group), heptyl group (such as n-heptyl group), octyl group (such as n-octyl group and 2-ethylhexyl group), nonyl group, decyl group, undecyl group, dodecyl group, tetradecyl group, hexadecyl group, octadecyl group, and icosyl group, preferably a C₁₋₁₈alkyl group (such as a C₁₋₁₂alkyl group), further preferably a C₁₋₈alkyl group (such as a C₁₋₆alkyl group), particularly a C₁₋₄alkyl group (such as a C₁₋₃alkyl group such as methyl group).

Examples of the unsaturated aliphatic hydrocarbon group may include a group corresponding to the above-exemplified alkyl group and having at least one ethylenically unsaturated bond (C=C double bond). The unsaturated aliphatic hydrocarbon group may, for example, be an alkenyl group. Examples of the alkenyl group may include a C₂₋₁₀alkenyl group such as vinyl group, propenyl group (such as allyl group and isopropenyl group), and butenyl group (such as 2-methylallyl group and crotyl group).

The alicyclic hydrocarbon group may be a saturated group or an unsaturated group. The alicyclic hydrocarbon group may be a monocyclic group (such as a cycloalkyl group, a cycloalkenyl group, a cycloalkadienyl group, and a cyclopolyenyl group) or may be a polycyclic group [such as a ring-assembly (ring-aggregated) group, a bridged cyclic (crosslinked cyclic) group, and a spirocyclic group].

As examples of the saturated monocyclic cycloalkyl group, there may be mentioned a C₃₋₂₀cycloalkyl group such as cyclopropyl group, cyclobutyl group, cyclopentyl group, cyclohexyl group, cycloheptyl group, cyclooctyl group, and cyclodecyl group, preferably a C₄₋₁₂cycloalkyl group, and further preferably a C₅₋₁₀cycloalkyl group.

Examples of the unsaturated monocyclic cycloalkenyl group and cycloalkadienyl group (or cyclopolyenyl group) may include a group corresponding to the above-exemplified cycloalkyl group and having at least one ethylenically unsaturated bond (double bond), and specific examples thereof may include a C₃₋₂₀cycloalkenyl group such as cyclopentenyl group and cyclohexenyl group, and a C₃₋₂₀cycloalkadienyl group such as cyclopentadienyl group.

The polycyclic alicyclic hydrocarbon group may be a ring-assembly group that has two or more aliphatic rings directly bonded to each other, or may be a bridged cyclic (crosslinked cyclic) group that has two or more aliphatic rings sharing one or more atom(s), a spirocyclic group, or other groups. Examples of the aliphatic ring may include an aliphatic ring corresponding to the above-mentioned monocyclic group. Representative examples of the polycyclic alicyclic hydrocarbon group may include a C₆₋₂₀ring-assembly alicyclic hydrocarbon group such as bicyclohexyl group, and a C₃₋₂₀bridged cyclic alicyclic hydrocarbon group such as norbornyl group and adamantyl group.

Examples of the aromatic hydrocarbon group may include an aryl group. The aromatic hydrocarbon group may be a monocyclic aryl group (such as phenyl group) or a polycyclic aryl group. The polycyclic aryl group may be a condensed cyclic group that has two or more rings (such as benzene rings) sharing two or more atoms (such as a C₁₀₋₂₄condensed cyclic aryl group such as naphthyl group, anthryl group, and phenanthryl group) or may be a ring-assembly group that has two or more rings (such as benzene rings) directly bonded to each other (such as a C₁₂₋₂₈ring-assembly aryl group such as biphenyl group, phenylnaphthyl group, naphthylphenyl group, binaphthyl group, and terphenyl group).

As examples of the group having two or more of these hydrocarbon groups in combination, there may be mentioned a cycloalkyl-alkyl group (such as a C₃₋₂₀cycloalkylC₁₋₆alkyl group such as cyclohexylmethyl group); an alkylaryl group [such as a mono- to pentaC₁₋₆alkylC₆₋₂₀aryl group such as methylphenyl group (tolyl group) and dimethylphenyl group (xylyl group)]; an aralkyl group (such as a C₆₋₂₀arylC₁₋₆alkyl group such as benzyl group and phenethyl group); and an arylalkenyl group [such as a C₆₋₂₀arylC₂₋₆alkenyl group such as phenylvinyl group (styryl group), cinnamyl group, and ethylcinnamyl group].

These hydrocarbon groups may have a substituent (or may be substituted). For example, the hydrocarbon group may or may not have one or more (such as 1 to 3, preferably 1 to 2, further preferably 1) substituent(s) (such as a group free from an element in group 16 of the Periodic Table) such as a halogen atom (such as a fluorine atom, a chlorine atom, a bromine atom, and an iodine atom), a cyano group, an amino group, and a substituted amino group (such as a mono- or dialkylamino group such as methylamino group and diisopropylamino group). The hydrocarbon group may have such a substituent alone or in combination of two or more. As a preferred substituent which the hydrocarbon group may have, there may be mentioned a halogen atom, more preferably a chlorine atom, a bromine atom, and an iodine atom, further preferably a chlorine atom and a bromine atom, and particularly a chlorine atom.

Examples of the heterocyclic group may include a heterocyclic group having one or more heteroatom(s) different from an element in group 16 of the Periodic Table, and may specifically include a heterocyclic group having nitrogen atom(s), such as pyrrolyl group, pyrrolidinyl group, imidazolyl group, pyridyl group, quinolyl group, and carbazolyl group.

The group containing an element in group 16 of the Periodic Table contains at least one element in group 16 of the Periodic Table [such as an oxygen atom (O), a sulfur atom (S), a selenium atom (Se), and a tellurium atom (Te)]. A preferred element in group 16 of the Periodic Table may be an oxygen atom (O) and/or a sulfur atom (S), and a further preferred element may be an oxygen atom (O).

The group containing an element in group 16 of the Periodic Table may be a straight-chain, branched-chain, or ring-shaped group, and may be a saturated group or an unsaturated group. Representative examples of the group containing an element in group 16 of the Periodic Table may include an oxygen atom-containing group, a sulfur atom-containing group, and a group having the oxygen atom- or sulfur atom-containing group bonded to the above-mentioned hydrocarbon group (the hydrocarbon group which may have a substituent) or heterocyclic group.

Example of the oxygen atom-containing group may include the following:
a hydroxyl group-containing group [such as hydroxyl group; a hydroxyalkyl group (such as a hydroxyC₁₋₆alkyl group such as hydroxymethyl group and hydroxyethyl group); and a hydroxy(poly)alkoxyalkyl group (such as a hydroxy(poly)C₁₋₆alkoxyC₁₋₆alkyl group such as a hydroxy-mono- to decaethoxy-ethyl group)];
an ether bond-containing group [such as an alkoxyalkyl group (such as a C₁₋₆alkoxyC₁₋₆alkyl group such as methoxymethyl group); a cycloalkoxyalkyl group (such as a C₃₋₂₀cycloalkyloxyC₁₋₆alkyl group such as cyclohexyloxymethyl group); an aryloxyalkyl group (such as a C₆₋₂₄aryloxyC₁₋₆alkyl group such as phenoxymethyl group); an aralkyloxyalkyl group (such as a C₆₋₂₄arylC₁₋₆alkyloxyC₁₋₆alkyl group such as benzyloxymethyl group); an epoxy-containing group (such as epoxy group, glycidyl group, β-methylglycidyl group, and epoxidized cyclohexenyl group); and an oxetane-containing group (such as oxetanyl group)];
a carbonyl group-containing group [such as formyl group; carboxyl group; an alkylcarbonyl group (such as a C₁₋₂₀alkyl-carbonyl group such as methylcarbonyl group); an alkoxycarbonyl group (such as a C₁₋₆alkoxycarbonyl group such as methoxycarbonyl group, ethoxycarbonyl group, and tert-butoxycarbonyl group);
(meth)acryloyl group; a (meth)acryloyloxyalkyl group [such as a (meth)acryloyloxyC₁₋₆alkyl group such as (meth)acryloyloxymethyl group]; an amide group (or an aminocarbonyl group); and a substituted aminocarbonyl group (such as an alkylaminocarbonyl group, specifically a mono- or diC₁₋₆alkylamino-carbonyl group such as N-ethylaminocarbonyl group)];
a nitro group-containing group (such as the above-mentioned hydrocarbon group having nitro group, such as nitromethyl group, nitrocyclohexyl group, and nitrophenyl group); and
an oxygen atom-containing heterocyclic group (such as furyl group, tetrahydrofuryl group, pyranyl group, tetrahydropyranyl group, isobenzofuranyl group, xanthonyl group, and morpholinyl group).

Examples of the sulfur atom-containing group may include the following:
a thiol group (mercapto group)-containing group [such as thiol group; a mercaptoalkyl group (such as a mercaptoC₁₋₆alkyl group such as mercaptomethyl group and mercaptoethyl group); a mercapto(poly)alkoxyalkyl group (such as a mercapto(poly)C₁₋₆alkoxyC₁₋₆alkyl group such as a mercapto-mono- to decaethoxy-ethyl group); and a group corresponding to such a group and having a mercapto(poly)thio group (or a mercapto(poly)sulfanyl group) [-Sₙ-H] instead of mercapto group, wherein n denotes an integer of not less than 2];
a sulfide bond-containing group [such as an alkylthio group (such as a C₁₋₆alkylthio group such as methylthio group); a cycloalkylthio group (such as a C₃₋₂₀cycloalkylthio group such as cyclohexylthio group); an arylthio group (such as a C₆₋₂₄arylthio group such as phenylthio group); an aralkylthio group (such as a C₆₋₂₄arylC₁₋₆alkylthio group such as benzylthio group); an alkylthioalkyl group (such as a C₁₋₆alkylthioC₁₋₆alkyl group such as methylthiomethyl group); a cycloalkylthioalkyl group (such as a C₃₋₂₀cycloalkylthioC₁₋₆alkyl group such as cyclohexylthiomethyl group); an arylthioalkyl group (such as a C₆₋₂₄arylthioC₁₋₆alkyl group such as phenylthiomethyl group); an aralkylthioalkyl group (such as a C₆₋₂₄arylC₁₋₆alkylthioC₁₋₆alkyl group such as benzylthiomethyl group); a thiirane-containing group [such as thiiranyl group (or 1,2-epithioethyl group) and thiiranylmethyl group (or 2,3-epithiopropyl group)]; and a group corresponding to such a group and having a polythio group (a polysulfide bond) [-Sₙ-] instead of thio group (sulfide bond) [-S-], wherein n denotes an integer of not less than 2];
a thiocarbonyl (C=S) group-containing group [such as an alkyl(thiocarbonyl) group (such as a C₁₋₂₀alkyl-(thiocarbonyl) group such as methyl(thiocarbonyl) group); an alkoxy(thiocarbonyl) group (such as a C₁₋₆alkoxy-(thiocarbonyl) group such as methoxy(thiocarbonyl) group); a thio(meth)acryloyl group; a thio(meth)acryloyloxyalkyl group [such as a (meth)acryloyloxyC₁₋₆alkyl group such as thio(meth)acryloyloxymethyl group]; a thioamide group (or a thiamide group); and a substituted thioamide group (such as an alkyl(thioamide) group, specifically a mono- or diC₁₋₆alkyl-(thioamide) group such as N-ethyl(thioamide) group)]; and a sulfur atom-containing heterocyclic group (such as thienyl group, tetrahydrothienyl group, and thianthrenyl group).

Preferred examples of the substituent represented by each of R¹ and R² may include a substituted or unsubstituted hydrocarbon group [a hydrocarbon group which may have a substituent (or which may be substituted)], and a group containing an element in group 16 of the Periodic Table, and further preferred examples may include a substituted or unsubstituted hydrocarbon group, an oxygen atom-containing group, and a sulfur atom-containing group.

Preferred examples of each of R¹ and R² may include at least one member selected from the group consisting of a hydrogen atom, a substituted or unsubstituted hydrocarbon group, and a group containing an element in group 16 of the Periodic Table. Further preferably, at least one (preferably both) of R¹ and R² may contain at least one member selected from the group consisting of the monovalent groups represented by the after-mentioned formulae (2B) to (2G). The species or kinds of R¹ and R² may be different from each other, and are preferably the same.

In this description and claims, the monovalent group represented by the formula (2B) may simply be referred to as a group (2B); in the same manner, each of the monovalent groups represented by the formulae (2C) to (2G) may be referred to as the corresponding one of the groups (2C) to (2G).

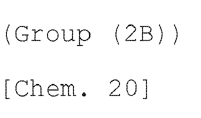

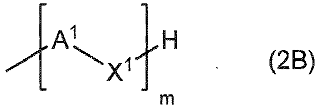

In the formula, A¹ represents an alkylene group, X¹ represents an oxygen atom or a sulfur atom, and m denotes 0 or an integer of not less than 1.

In the group (2B), as examples of the alkylene group represented by A¹, there may be mentioned a C₁₋₁₀alkylene group such as methylene group, ethylene group, propane-1,3-diyl group, propylene group, butane-1,4-diyl group, butane-1,2-diyl group, and hexane-1,6-diyl group. A preferred alkylene group A¹ may be a C₁₋₆alkylene group (such as a C₂₋₆alkylene group), further preferably a C₂₋₄alkylene group (a C₂₋₃alkylene group such as ethylene group and propylene group), and particularly ethylene group.

X¹ may represent either of an oxygen atom or a sulfur atom, and is preferably an oxygen atom.

The repeating number m of the group [-A¹-X¹-] is 0 or an integer of not less than 1. When the repeating number is not less than 1, the repeating number may, for example, be an integer of about 1 to 20 (such as about 1 to 15) and preferably an integer of about 1 to 10 (such as about 1 to 5). The number m in the group (2B) may preferably be an integer of about 0 to 10 (such as about 0 to 5), and further preferably an integer of about 0 to 2 (such as 0 or 1); in particular, the number m may be 0 or may be 1. When the number m is not less than 2, the species of two or more groups [-A¹-X¹-] (the species of A¹ and X¹) may be different from each other, and are preferably the same.

Representative examples of the group (2B) may include a hydrogen atom, a hydroxyalkyl group [such as a hydroxyC₂₋₆alkyl group such as hydroxyethyl group and hydroxypropyl group], a hydroxy-(poly)alkoxyalkyl group [such as a hydroxy-mono- to decaC₂₋₆alkoxy-C₂₋₆alkyl group such as hydroxyethoxyethyl group and hydroxyethoxyethoxyethyl group], a mercaptoalkyl group [such as a mercaptoC₂₋₆alkyl group such as mercaptoethyl group and mercaptopropyl group], and a mercapto-(poly)alkylthio-alkyl group [such as a mercapto-mono- to decaC₂₋₆alkylthio-C₂₋₆alkyl group such as mercaptoethylthioethyl group and mercaptoethylthioethylthioethyl group].

Preferred examples of the group (2B) may include a hydrogen atom, a hydroxyalkyl group [such as a hydroxyC₂₋₄alkyl group], a hydroxy-(poly)alkoxyalkyl group [such as a hydroxy-mono- to pentaC₂₋₄alkoxy-C₂₋₄alkyl group such as hydroxyethoxyethyl group], a mercaptoalkyl group [such as a mercaptoC₂₋₄alkyl group], and a mercapto-(poly)alkylthio-alkyl group [such as a mercapto-mono- to pentaC₂₋₄alkylthio-C₂₋₄alkyl group such as mercaptoethylthioethyl group]; further preferred examples may include a hydrogen atom, a hydroxyalkyl group [such as a hydroxyC₂₋₃alkyl group such as hydroxyethyl group], and a mercaptoalkyl group [such as a mercaptoC₂₋₃alkyl group such as mercaptoethyl group]; particularly preferred examples may include a hydrogen atom and a hydroxyalkyl group (such as hydroxyethyl group); the group (2B) may particularly be a hydroxyalkyl group (such as hydroxyethyl group); from the viewpoint of effective use as a curing agent for an epoxy resin, the group (2B) is preferably a hydrogen atom.

The group (2B) may form a salt. The group (2B) may form an alkali metal salt (such as a sodium salt and a potassium salt), an onium salt (such as an ammonium salt), and other salts.

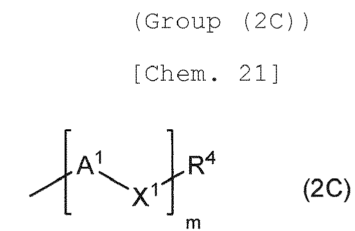

In the formula, A¹ represents an alkylene group, X¹ represents an oxygen atom or a sulfur atom, and m denotes 0 or an integer of not less than 1, and
R⁴ represents a saturated hydrocarbon group which may have a halogen atom.

In the group (2C), A¹ and X¹, including preferred embodiments, each have the same meanings as the alkylene group and the atom described in the section of the group (2B).

The repeating number m of the group [-A¹-X¹-] in the group (2C) may, for example, be within the same range as the numerical range described in the section of the group (2B). The number m in the group (2C) may preferably be an integer of about 0 to 10 (such as about 0 to 5), further preferably an integer of about 0 to 2 (such as 0 or 1), and particularly preferably 0. When the number m is not less than 2, the species of two or more groups [-A¹-X¹-] (the species of A¹ and X¹) may be different from each other, and are preferably the same.

Examples of the saturated hydrocarbon group represented by R⁴ may include a saturated hydrocarbon group among the hydrocarbon groups exemplified in the above-mentioned R¹, R² (such as the above-mentioned saturated aliphatic hydrocarbon group, the above-mentioned saturated alicyclic hydrocarbon group, and a group having two or more of these groups in combination). The saturated hydrocarbon group represented by R⁴ may preferably be an alkyl group, further preferably a C₁₋₆alkyl group (such as a C₁₋₄alkyl group), and particularly a C₁₋₃alkyl group (such as a C₁₋₂alkyl group such as methyl group).

The saturated hydrocarbon group represented by R⁴ may or may not have one or more halogen atom(s) (such as a fluorine atom, a chlorine atom, a bromine atom, and an iodine atom) or may or may not be substituted by a halogen atom. In a case where the saturated hydrocarbon group have a halogen atom, a preferred halogen atom may be a chlorine atom, a bromine atom, and an iodine atom, further preferably a chlorine atom and a bromine atom, and particularly a chlorine atom. The number of halogen atoms (the number of substitutions) may, for example, be about 0 to 10, preferably about 1 to 3, further preferably about 1 to 2, and particularly 1. When the number of halogen atoms (the number of substitutions) is not less than 2, the species of two or more halogen atoms may be the same or different from each other.

Representative examples of the group (2C) may include an alkyl group (such as a C₁₋₁₂alkyl group such as methyl group, ethyl group, propyl group, and butyl group), an alkoxyalkyl group [such as a C₁₋₆alkoxyC₂₋₆alkyl group such as methoxyethyl group, ethoxyethyl group, and methoxypropyl group], a polyalkoxy-alkyl group [such as a C₁₋₆alkoxy-mono- to decaC₂₋₆alkoxy-C₂₋₆alkyl group such as methoxyethoxyethyl group, ethoxyethoxyethyl group, and methoxyethoxyethoxyethyl group], an alkylthioalkyl group [such as a C₁₋₆alkylthioC₂₋₆alkyl group such as methylthioethyl group, ethylthioethyl group, and methylthiopropyl group], a polyalkylthio-alkyl group [such as a C₁₋₆alkylthio-mono- to decaC₂₋₆alkylthio-C₂₋₆alkyl group such as methylthioethylthioethyl group and methylthioethylthioethylthioethyl group], a group in which any of the above-mentioned groups has one or more halogen atom(s), such as chlorine atom(s) [such as a haloalkyl group such as a chloroalkyl group (such as chloroethyl group) and a bromoalkyl group (such as bromoethyl group), and a haloalkoxyalkyl group such as a chloroalkoxyalkyl group (such as chloroethoxyethyl group)].

Preferred examples of the group (2C) may include an alkyl group (such as a C₁₋₈alkyl group), an alkoxyalkyl group [such as a C₁₋₄alkoxyC₂₋₄alkyl group], a polyalkoxy-alkyl group [such as a C₁₋₄alkoxy-mono- to pentaC₂₋₄alkoxy-C₂₋₄alkyl group], a (poly)alkylthio-alkyl group [such as a C₁₋₄alkylthioC₂₋₄alkyl group and a C₁₋₄alkylthio-mono- to pentaC₂₋₄alkylthio-C₂₋₄alkyl group], and a haloalkyl group (such as a haloC₁₋₈alkyl group); further preferred examples may include an alkyl group (such as a C₁₋₄alkyl group), an alkoxyalkyl group [such as a C₁₋₃alkoxyC₂₋₃alkyl group], an alkylthioalkyl group [such as a C₁₋₃alkylthioC₂₋₃alkyl group], and a haloalkyl group (such as a haloC₁₋₄alkyl group); particularly preferred examples may include an alkyl group (such as a C₁₋₃alkyl group), an alkoxyalkyl group [such as a C₁₋₂alkoxyC₂₋₃alkyl group], and a haloalkyl group (such as a haloC₁₋₃alkyl group); the group (2C) may particularly be an alkyl group (such as a C₁₋₂alkyl group such as methyl group) and a haloalkyl group (such as a haloC₁₋₂alkyl group such as chloroethyl group); among them, the group (2C) may be an alkyl group.

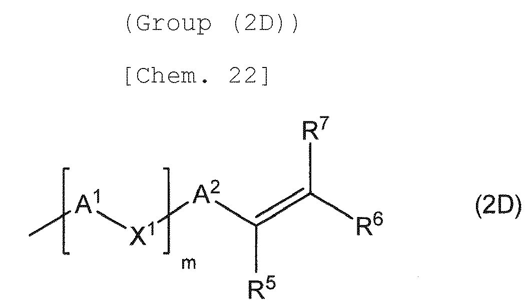

In the formula, A¹ represents an alkylene group, X¹ represents an oxygen atom or a sulfur atom, and m denotes 0 or an integer of not less than 1,

A² represents a direct bond (a single bond) or an alkylene group,

R⁵, R⁶, and R⁷ independently represent a hydrogen atom, or a substituted or unsubstituted hydrocarbon group [a hydrocarbon group which may have a substituent (or which may be substituted)].

In the group (2D), A¹ and X¹, including preferred embodiments, each have the same meanings as the alkylene group and the atom described in the section of the group (2B).

The repeating number m of the group [-A¹-X¹-] in the group (2D) may, for example, be within the same range as the numerical range described in the section of the group (2B). The number m in the group (2D) may preferably be an integer of about 0 to 10 (such as about 0 to 5), further preferably an integer of about 0 to 2 (such as 0 or 1), and particularly preferably 0. When the number m is not less than 2, the species of two or more groups [-A¹-X¹-] (the species of A¹ and X¹) may be different from each other, and are preferably the same.

A² may be either of a direct bond (a single bond) or an alkylene group, and is preferably an alkylene group. From the point of view of more improvement in refractive index, A² preferably represents a direct bond (a single bond). Examples of the alkylene group represented by A² may include a C₁₋₁₀alkylene group such as methylene group, ethylene group, ethane-1,1-diyl group, propane-1,3-diyl group, propylene group, butane-1,4-diyl group, and hexane-1,6-diyl group. The alkylene group represented by A² may preferably be a C₁₋₆alkylene group (such as a C₁₋₄alkylene group), further preferably a C₁₋₃alkylene group (such as a C₁₋₂alkylene group such as methylene group and ethylene group), and particularly methylene group.

Preferred examples of A² may include a direct bond or a C₁₋₃alkylene group, further preferably a direct bond or a C₁₋₂alkylene group (such as methylene group and ethylene group), and particularly preferably a direct bond or methylene group (in particular, methylene group). From the point of view of more improvement in refractive index, A² preferably represents a direct bond (a single bond).

Each of R⁵, R⁶, and R⁷ may represent either of a hydrogen atom, or a substituted or unsubstituted hydrocarbon group [a hydrocarbon group which may be substituted]. Examples of the substituted or unsubstituted hydrocarbon group [the hydrocarbon group which may be substituted] may include the same group as the substituted or unsubstituted hydrocarbon group [the hydrocarbon group which may be substituted] represented by the above-mentioned R¹, R².

Preferred examples of R⁵, R⁶, R⁷ may include a hydrogen atom or a hydrocarbon group (such as an alkyl group); further preferred examples may include a hydrogen atom or a C₁₋₆alkyl group (such as a C₁₋₄alkyl group). Among them, preferred is a hydrogen atom or a C₁₋₃alkyl group (such as methyl group), particularly a hydrogen atom. The species of R⁵, R⁶, R⁷ may be the same or different from one another.

Representative examples of the group (2D) may include an alkenyl group [such as a C₂₋₁₀alkenyl group such as vinyl group, a propenyl group (such as allyl group and isopropenyl group), and a butenyl group (such as 2-methylallyl group and crotyl group)], an alkenyloxyalkyl group [such as a C₂₋₆alkenyloxyC₂₋₆alkyl group such as vinyloxyethyl group, allyloxyethyl group, and allyloxypropyl group], an alkenyl-(poly)alkoxy-alkyl group [such as a C₂₋₆alkenyloxy-mono- to decaC₂₋₆alkoxy-C₂₋₆alkyl group such as vinyloxyethoxyethyl group, allyloxyethoxyethyl group, and allyloxyethoxyethoxyethyl group], an alkenylthioalkyl group [such as a C₂₋₆alkenylthioC₂₋₆alkyl group such as vinylthioethyl group, allylthioethyl group, and allylthiopropyl group], and an alkenyl-(poly)alkylthio-alkyl group [such as a C₂₋₆alkenylthio-mono- to decaC₂₋₆alkylthio-C₂₋₆alkyl group such as vinylthioethylthioethyl group, allylthioethylthioethyl group, and allylthioethylthioethylthioethyl group].

Preferred examples of the group (2D) may include an alkenyl group [such as a C₂₋₄alkenyl group], an alkenyloxyalkyl group [such as a C₂₋₄alkenyloxyC₂₋₄alkyl group], an alkenyl-(poly)alkoxyalkyl group [such as a C₂₋₄alkenyloxy-mono- to pentaC₂₋₄alkoxy-C₂₋₄alkyl group], an alkenylthioalkyl group [such as a C₂₋₄alkenylthioC₂₋₄alkyl group], and an alkenyl-(poly)alkylthio-alkyl group [such as a C₂₋₄alkenylthio-mono- to pentaC₂₋₄alkylthio-C₂₋₄alkyl group]; further preferred examples may include an alkenyl group [such as a C₂₋₆alkenyl group], an alkenyloxyalkyl group [such as a C₂₋₃alkenyloxyC₂₋₃alkyl group], and an alkenylthioalkyl group [such as a C₂₋₃alkenylthioC₂₋₃alkyl group]; among them, the group (2D) may be an alkenyl group (such as a C₂₋₃alkenyl group such as vinyl group, allyl group, and isopropenyl group); in particular, the group (2D) may be vinyl group or allyl group (particularly allyl group). From the point of view of more improvement in refractive index or polymerizability, the group (2D) is preferably vinyl group.

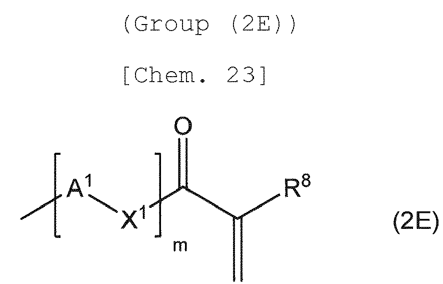

In the formula, A¹ represents an alkylene group, X¹ represents an oxygen atom or a sulfur atom, and m denotes 0 or an integer of not less than 1, and
R⁸ represents a hydrogen atom or a methyl group.

In the group (2E), A¹ and X¹, including preferred embodiments, each have the same meanings as the alkylene group and the atom described in the section of the group (2B).

The repeating number m of the group [-A¹-X¹-] in the group (2E) may, for example, be within the same range as the numerical range described in the section of the group (2B). The number m in the group (2E) may preferably be an integer of about 0 to 10 (such as about 0 to 5), further preferably an integer of about 0 to 2 (such as 0 or 1), and particularly preferably 0. When the number m is not less than 2, the species of two or more groups [-A¹-X¹-] (the species of A¹ and X¹) may be different from each other, and are preferably the same.

R⁸ may represent either of a hydrogen atom or a methyl group. In particular, when the number m is 0, R⁸ preferably represents a methyl group from the point of view of productivity or storage stability.

Representative examples of the group (2E) may include (meth)acryloyl group, a (meth)acryloyloxyalkyl group [such as a (meth)acryloyloxyC₂₋₆alkyl group such as (meth)acryloyloxyethyl group and (meth)acryloyloxypropyl group], a (meth)acryloyloxy-(poly)alkoxy-alkyl group [such as a (meth)acryloyloxy-mono- to decaC₂₋₆alkoxy-C₂₋₆alkyl group such as (meth)acryloyloxyethoxyethyl group and (meth)acryloyloxyethoxyethoxyethyl group], a (meth)acryloylthioalkyl group [such as a (meth)acryloylthioC₂₋₆alkyl group such as (meth)acryloylthioethyl group and (meth)acryloylthiopropyl group], and a (meth)acryloylthio-(poly)alkylthio-alkyl group [such as a (meth)acryloylthio-mono- to decaC₂₋₆alkylthio-C₂₋₆alkyl group such as (meth)acryloylthioethylthioethyl group and (meth)acryloylthioethylthioethylthioethyl group].

Preferred examples of the group (2E) may include (meth)acryloyl group, a (meth)acryloyloxyalkyl group [such as a (meth)acryloyloxyC₂₋₄alkyl group], and a (meth)acryloyloxy-(poly)alkoxy-alkyl group [such as a (meth)acryloyloxy-mono- to pentaC₂₋₄alkoxy-C₂₋₄alkyl group such as (meth)acryloyloxyethoxyethyl group]; further preferred examples may include (meth)acryloyl group and a (meth)acryloyloxyalkyl group [such as a (meth)acryloyloxyC₂₋₃alkyl group such as (meth)acryloyloxyethyl group]; in particular, (meth)acryloyl group (especially methacryloyl group) is preferred.

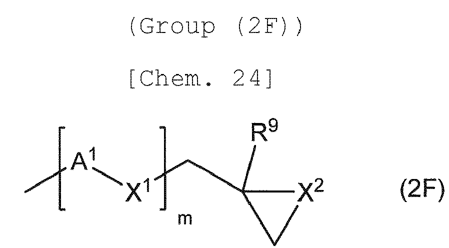

In the formula, A¹ represents an alkylene group, X¹ represents an oxygen atom or a sulfur atom, and m denotes 0 or an integer of not less than 1,
X² represents an oxygen atom or a sulfur atom, and
R⁹ represents a hydrogen atom or a methyl group.

In the group (2F), A¹ and X¹, including preferred embodiments, each have the same meanings as the alkylene group and the atom described in the section of the group (2B).

The repeating number m of the group [-A¹-X¹-] in the group (2F) may, for example, be within the same range as the numerical range described in the section of the group (2B). The number m in the group (2F) may preferably be an integer of about 0 to 10 (such as about 0 to 5), further preferably an integer of about 0 to 2 (such as 0 or 1), and particularly preferably 0. When the number m is not less than 2, the species of two or more groups [-A¹-X¹-] (the species of A¹ and X¹) may be different from each other, and are preferably the same.

X² may represent either of an oxygen atom or a sulfur atom, and is preferably an oxygen atom.

R⁹ may represent either of a hydrogen atom or a methyl group, and is preferably a hydrogen atom.

Representative examples of the group (2F) may include glycidyl group, a glycidyloxyalkyl group [such as a glycidyloxyC₂₋₆alkyl group such as glycidyloxyethyl group and glycidyloxypropyl group], a glycidyloxy-(poly)alkoxy-alkyl group [such as a glycidyloxy-mono- to decaC₂₋₆alkoxy-C₂₋₆alkyl group such as glycidyloxyethoxyethyl group and glycidyloxyethoxyethoxyethyl group], a glycidylthioalkyl group [such as a glycidylthioC₂₋₆alkyl group such as glycidylthioethyl group and glycidylthiopropyl group], a glycidylthio-(poly)alkylthio-alkyl group [such as a glycidylthio-mono- to decaC₂₋₆alkylthio-C₂₋₆alkyl group such as glycidylthioethylthioethyl group and glycidylthioethylthioethylthioethyl group], and a group corresponding to such a group and having β-methylglycidyl group, 2,3-epithiopropyl group, or 2-methyl-2,3-epithiopropyl group instead of glycidyl group.

Preferred examples of the group (2F) may include glycidyl group, a glycidyloxyalkyl group [such as a glycidyloxyC₂₋₄alkyl group], a glycidyloxy-(poly)alkoxy-alkyl group [such as a glycidyloxy-mono-to pentaC₂₋₄alkoxy-C₂₋₄alkyl group such as glycidyloxyethoxyethyl group], and a group corresponding to such a group and having β-methylglycidyl group or 2,3-epithiopropyl group instead of glycidyl group; further preferred examples may include glycidyl group, a glycidyloxyalkyl group [such as a glycidyloxyC₂₋₃alkyl group such as glycidyloxyethyl group], and a group corresponding to such a group and having β-methylglycidyl group or 2,3-epithiopropyl group instead of glycidyl group; particularly preferred examples may include glycidyl group, β-methylglycidyl group, and 2,3-epithiopropyl group (such as glycidyl group and β-methylglycidyl group, preferably glycidyl group).

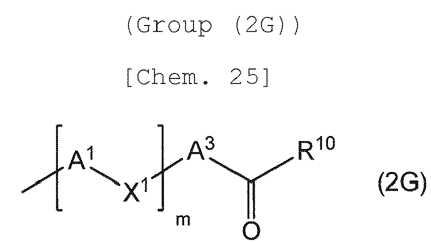

In the formula, A¹ represents an alkylene group, X¹ represents an oxygen atom or a sulfur atom, and m denotes 0 or an integer of not less than 1,
A³ represents an alkylene group, and
R¹⁰ represents a hydroxyl group, a group [-OR¹¹] (wherein R¹¹ represents a substituted or unsubstituted hydrocarbon group), or a halogen atom.

In the group (2G), A¹ and X¹, including preferred embodiments, each have the same meanings as the alkylene group and the atom described in the section of the group (2B).

The repeating number m of the group [-A¹-X¹-] in the group (2G) may, for example, be within the same range as the numerical range described in the section of the group (2B). The number m in the group (2G) may preferably be an integer of about 0 to 10 (such as about 0 to 5), further preferably an integer of about 0 to 2 (such as 0 or 1), and particularly preferably 0. When the number m is not less than 2, the species of two or more groups [-A¹-X¹-] (the species of A¹ and X¹) may be different from each other, and are preferably the same.

Examples of the alkylene group represented by A³ may include a C₁₋₁₀alkylene group such as methylene group, ethylene group, ethane-1,1-diyl group, propane-1,3-diyl group, propylene group, butane-1,4-diyl group, and hexane-1,6-diyl group. The alkylene group represented by A³ may preferably be a C₁₋₆alkylene group (such as a C₁₋₄alkylene group), further preferably a C₁₋₃alkylene group (such as a C₁₋₂alkylene group such as methylene group and ethylene group), and particularly preferably methylene group.

Preferred examples of A³ may include a C₁₋₃alkylene group, further preferably a C₁₋₂alkylene group (such as methylene group and ethylene group), and particularly preferably methylene group.

R¹⁰ may represent a group [-OR¹¹], wherein R¹¹ represents a substituted or unsubstituted hydrocarbon group; examples of the substituted or unsubstituted hydrocarbon group represented by R¹¹ may include the same group as the substituted or unsubstituted hydrocarbon group [the hydrocarbon group which may be substituted] represented by the above-mentioned R¹, R². Representative examples of the group [-OR¹¹] may include an alkoxy group, a cycloalkyloxy group (such as a C₃₋₁₀cycloalkyloxy group such as cyclohexyloxy group), and an aryloxy group (such as a C₆₋₁₂aryloxy group such as phenoxy group), and may preferably include an alkoxy group. Examples of the alkoxy group (a straight- or branched-chain alkoxy group) may include a C₁₋₁₀alkoxy group such as methoxy group, ethoxy group, n-propyloxy group, isopropyloxy group, n-butoxy group, t-butoxy group, and hexyloxy group. The alkoxy group may preferably be a C₁₋₆alkoxy group, further preferably a C₁₋₄alkoxy group, and particularly a C₁₋₃alkoxy group such as methoxy group and ethoxy group.

R¹⁰ may represent any of a hydroxyl group, a group [-OR¹¹], or a halogen atom; R¹⁰ may preferably represent a hydroxyl group or a group [-OR¹¹] (such as an alkoxy group) and may further preferably be a hydroxyl group.

Representative examples of the group (2G) may include the following:
a carboxyalkyl group (such as a carboxyC₁₋₆alkyl group such as carboxymethyl group);
an alkoxycarbonylalkyl group (such as a C₁₋₆alkoxycarbonyl-C₁₋₆alkyl group such as methoxycarbonylmethyl group and ethoxycarbonylmethyl group);
a halocarbonylalkyl group (such as a halocarbonylC₁₋₆alkyl group such as chlorocarbonylmethyl group and bromocarbonylmethyl group);
a carboxy-(poly)alkoxy-alkyl group [such as a carboxyC₁₋₆alkoxyC₂₋₆alkyl group such as carboxymethoxyethyl group and carboxymethoxypropyl group; and a carboxyC₁₋₆alkoxy-mono- to decaC₂₋₆alkoxy-C₂₋₆alkyl group such as carboxymethoxyethoxyethyl group and carboxymethoxyethoxyethoxyethyl group];
an alkoxycarbonyl-(poly)alkoxy-alkyl group [such as a C₁₋₆alkoxy-carbonyl-C₁₋₆alkoxyC₂₋₆alkyl group such as methoxycarbonylmethoxyethyl group, ethoxycarbonylmethoxyethyl group, and methoxycarbonylmethoxypropyl group; and a C₁₋₆alkoxycarbonyl-C₁₋₆alkoxy-mono- to decaC₂₋₆alkoxy-C₂₋₆alkyl group such as methoxycarbonylmethoxyethoxyethyl group and methoxycarbonylmethoxyethoxyethoxyethyl group];
a halocarbonyl-(poly)alkoxy-alkyl group [such as a halocarbonyl-C₁₋₆alkoxyC₂₋₆alkyl group such as chlorocarbonylmethoxyethyl group, bromocarbonylmethoxyethyl group, and chlorocarbonylmethoxypropyl group; and a halocarbonyl-C₁₋₆alkoxy-mono- to decaC₂₋₆alkoxy-C₂₋₆alkyl group such as chlorocarbonylmethoxyethoxyethyl group and chlorocarbonylmethoxyethoxyethoxyethyl group];
a carboxy-(poly)alkylthio-alkyl group [such as a carboxyC₁₋₆alkylthioC₂₋₆alkyl group such as carboxymethylthioethyl group and carboxymethylthiopropyl group; and a carboxyC₁₋₆alkylthio-mono- to decaC₂₋₆alkylthio-C₂₋₆alkyl group such as carboxymethylthioethylthioethyl group and carboxymethylthioethylthioethylthioethyl group];
an alkoxycarbonyl-(poly)alkylthio-alkyl group [such as a C₁₋₆alkoxy-carbonyl-C₁₋₆alkylthioC₂₋₆alkyl group such as methoxycarbonylmethylthioethyl group, ethoxycarbonylmethylthioethyl group, and methoxycarbonylmethylthiopropyl group; and a C₁₋₆alkoxy-carbonyl-C₁₋₆alkylthio-mono- to decaC₂₋₆alkylthio-C₂₋₆alkyl group such as methoxycarbonylmethylthioethylthioethyl group and methoxycarbonylmethylthioethylthioethylthioethyl group]; and
a halocarbonyl-(poly)alkylthio-alkyl group [such as a halocarbonyl-C₁₋₆alkylthioC₂₋₆alkyl group such as chlorocarbonylmethylthioethyl group, bromocarbonylmethylthioethyl group, and chlorocarbonylmethylthiopropyl group; and a halocarbonyl-C₁₋₆alkylthio-mono- to decaC₂₋₆alkylthio-C₂₋₆alkyl group such as chlorocarbonylmethylthioethylthioethyl group and chlorocarbonylmethylthioethylthioethylthioethyl group].

Preferred examples of the group (2G) may include the following:
a carboxyalkyl group (such as a carboxyC₁₋₄alkyl group such as carboxymethyl group),
an alkoxycarbonylalkyl group (such as a C₁₋₄alkoxycarbonyl-C₁₋₄alkyl group such as methoxycarbonylmethyl group),
a halocarbonylalkyl group (such as a halocarbonylC₁₋₄alkyl group such as chlorocarbonylmethyl group),
a carboxyalkoxyalkyl group [such as a carboxyC₁₋₄alkoxyC₂₋₄alkyl group such as carboxymethoxyethyl group],
an alkoxycarbonylalkoxyalkyl group [such as a C₁₋₄alkoxy-carbonyl-C₁₋₄alkoxyC₂₋₄alkyl group such as methoxycarbonylmethoxyethyl group],
a halocarbonylalkoxyalkyl group [such as a halocarbonyl-C₁₋₄alkoxyC₂₋₄alkyl group such as chlorocarbonylmethoxyethyl group],
a carboxyalkylthioalkyl group [such as a carboxyC₁₋₄alkylthioC₂₋₄alkyl group such as carboxymethylthioethyl group],
an alkoxycarbonylalkylthioalkyl group [such as a C₁₋₄alkoxy-carbonyl-C₁₋₄alkylthioC₂₋₄alkyl group such as methoxycarbonylmethylthioethyl group], and
a halocarbonylalkylthioalkyl group [such as a halocarbonyl-C₁₋₄alkylthioC₂₋₄alkyl group such as chlorocarbonylmethylthioethyl group].

Further preferred examples of the group (2G) may include a carboxyalkyl group (such as a carboxyC₁₋₃alkyl group such as carboxymethyl group), an alkoxycarbonylalkyl group (such as a C₁₋₃alkoxycarbonyl-C₁₋₃alkyl group such as methoxycarbonylmethyl group), a halocarbonylalkyl group (such as a halocarbonylC₁₋₃alkyl group such as chlorocarbonylmethyl group). In particular, the group (2G) may be a carboxyalkyl group (such as a carboxyC₁₋₂alkyl group such as carboxymethyl group).

The group (2G) may form a salt. For example, when R¹⁰ represents a hydroxyl group, the group (2G) may form an alkali metal salt (such as a sodium salt and a potassium salt), an onium salt (such as an ammonium salt), and other salts.

In a case where R¹ and R² each represent at least one member selected from the group consisting of the monovalent groups represented by the groups (2B) to (2G), more preferred R¹ and R² each may represents at least one member selected from the group consisting of a hydrogen atom, an alkyl group, a haloalkyl group (such as 2-chloroethyl group), an alkenyl group (such as vinyl group and allyl group), (meth)acryloyl group, a (meth)acryloyloxyalkyl group, a glycidyl group, a β-methylglycidyl group, a glycidyloxyalkyl group, a β-methylglycidyloxyalkyl group, a hydroxyalkyl group, a carboxyalkyl group, an alkoxycarbonylalkyl group, and a halocarbonylalkyl group.

In the formula (1), examples of the monovalent substituent represented by R³ may include the same as the group exemplified as the substituent represented by the above-mentioned R¹, R², or may include a halogen atom (such as a fluorine atom, a chlorine atom, a bromine atom, and an iodine atom), a hydroxyl group, an alkoxy group (such as methoxy group), a cycloalkyloxy group (such as cyclohexyloxy group), an aryloxy group (such as phenoxy group), an aralkyloxy group (such as benzyloxy group), a hydroxyalkoxy group (such as 2-hydroxyethoxy group), a vinyloxy group, an allyloxy group, a glycidyloxy group, a β-methylglycidyloxy group, an amino group, a substituted amino group (such as a mono- or dialkylamino group and a mono- or diacylamino group), a nitro group, and a cyano group.

Preferred examples of R³ may include a hydrocarbon group (a hydrocarbon group which may be substituted), more preferably a hydrocarbon group which is not substituted, such as a straight- or branched-chain alkyl group, and further preferably a C₁₋₆alkyl group (such as a C₁₋₄alkyl group) such as methyl group.

The number of substitutions n is an integer of 0 to 6, and may, for example, be an integer of 0 to 5 (such as 0 to 4), preferably an integer of 0 to 3 (such as 0 to 2), and further preferably 0 or 1 (particularly 0).

When the number n is not less than 1, the substitution position of R³ is not particularly limited to a specific one. When the number n is not less than 2, the species of two or more groups R³ may be the same or different from each other.

Representative examples of the compound represented by the formula (1) may include the following embodiments (A) to (G) (in particular, the compounds represented by the following formulae (1A) to (1G)).

In this description and claims, the compound represented by the formula (1) may simply be referred to as a compound (1); in the same manner, each of the compounds represented by the formulae (1A) to (1G) may be referred to as the corresponding one of the compounds (1A) to (1G).

### (Embodiment (A))

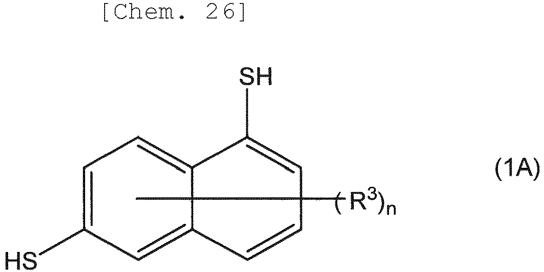

In the formula, R³ and n, including preferred embodiments, each have the same meanings as defined in the formula (1).

The embodiment (A) [the compound (1A)] is a 1,6-naphthalenedithiol compound represented by the formula (1) in which each of R¹ and R² represents a hydrogen atom, and examples of the embodiment (A) may include 1,6-naphthalenedithiol. The compound (1A) may be in the form of a salt (or a complex), and may, for example, be an alkali metal salt such as a sodium salt and a potassium salt. The compound (1A) in the form of a salt has a high dissolubility in water.

A process for producing the compound (1A) is not particularly limited to a specific one. For example, the compound (1A) may be prepared by (i) allowing a salt of a 1,6-naphthalenedisulfonic acid corresponding to the compound (1A) to react with a halogenating agent to prepare a 1,6-bis(halosulfonyl)naphthalene and (ii) allowing the resulting 1,6-bis(halosulfonyl)naphthalene to react with an acid and a reducing agent.

In the above-mentioned (i), examples of the salt of the 1,6-naphthalenedisulfonic acid corresponding to the compound (1A) may include an alkali metal salt (such as preferably a sodium salt) of 1,6-naphthalenedisulfonic acid.

As examples of the halogenating agent, there may be mentioned a thionyl halide (such as preferably thionyl chloride). The halogenating agent may be used alone or in combination of two or more. The ratio of the halogenating agent relative to 1 mol of the salt of the 1,6-naphthalenedisulfonic acid may, for example, be about 2 to 10 mol, preferably about 2.1 to 5 mol, and further preferably about 2.3 to 3 mol.

The reaction may be carried out in the presence or absence of a catalyst. Examples of the catalyst may include an amide such as N,N-dimethylformamide (DMF) and N,N-dimethylacetamide (DMAc) (preferably DMF). The catalyst may be used alone or in combination of two or more. In a case where the catalyst is used, the ratio of the catalyst relative to 1 mol of the salt of the 1,6-naphthalenedisulfonic acid may, for example, be about 0.01 to 1 mol, preferably about 0.1 to 0.7 mol, and further preferably about 0.3 to 0.5 mol.

The reaction may be carried out in the presence or absence of a solvent. Examples of the solvent may include an inert or inactive solvent to the reaction, for example, a hydrocarbon, a halide of a hydrocarbon (such as a chloride (or a chlorinated matter)), preferably an aromatic hydrocarbon such as toluene and xylene, and a hydrocarbon chloride such as dichloromethane, chloroform, 1,2-dichloroethane, chlorobenzene, and dichlorobenzene. The solvent may be used alone or as a mixed solvent in combination of two or more. The ratio of the solvent is not particularly limited to a specific ratio, and may, for example, be about 10 to 1000 parts by mass, preferably about 100 to 800 parts by mass, and further preferably about 300 to 600 parts by mass, relative to 100 parts by mass of the salt of the 1,6-naphthalenedisulfonic acid.

The reaction temperature may, for example, be about 50 to 150°C, preferably about 80 to 120°C, and further preferably about 90 to 110°C. The reaction time may, for example, be about 1 to 24 hours, preferably about 2 to 18 hours, and further preferably about 4 to 12 hours.

The reaction may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, and drying, and a combination of these means, or may directly be subjected (such as, in a solution state) to the above-mentioned reaction (ii) without separation-purification.

In the above-mentioned (ii), examples of the 1,6-bis(halosulfonyl)naphthalene may include a 1,6-bis(chlorosulfonyl)naphthalene such as 1,6-bis(chlorosulfonyl)naphthalene [or 1,6-naphthalenedisulfonyl chloride].

The acid may include, for example, an inorganic acid such as hydrochloric acid and sulfuric acid, and is preferably hydrochloric acid. The acid may be used alone or in combination of two or more. The ratio of the acid relative to 1 mol of the 1,6-bis(halosulfonyl)naphthalene may, for example, be about 10 to 100 mol, preferably about 40 to 60 mol, and further preferably about 45 to 55 mol.

As examples of the reducing agent, there may be mentioned zinc (such as zinc powder) and iron (such as iron powder), and the reducing agent is preferably zinc. The reducing agent may be used alone or in combination of two or more. The ratio of the reducing agent relative to 1 mol of the 1,6-bis(halosulfonyl)naphthalene may, for example, be about 5 to 20 mol, preferably about 8 to 17 mol, and further preferably about 10 to 15 mol.

The reaction may be carried out in the presence of a solvent. The solvent may include the same as the solvent in the above-mentioned (i). The solvent may be used alone or as a mixed solvent in combination of two or more. The amount of the solvent is not particularly limited to a specific one, and may, for example, be about 100 to 10000 parts by mass, preferably about 1000 to 5000 parts by mass, and further preferably about 2000 to 4000 parts by mass, relative to 100 parts by mass of the 1,6-bis(halosulfonyl)naphthalene.

The reaction temperature may, for example, be about 30 to 120°C, preferably about 40 to 100°C, and further preferably about 50 to 80°C. The reaction time may, for example, be about 0.5 to 24 hours, preferably about 1 to 12 hours, and further preferably about 2 to 5 hours.

The reaction may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, drying, and distillation (such as distillation under a reduced pressure), and a combination of these means.

In the present invention, the compound represented by the formula (1) (provided that the compound (1A) is excluded) may be produced by a step of replacing at least one hydrogen atom selected from mercapto groups on 1-position and 6-position of the compound (1A) with a specified substituent (a substituent corresponding to R¹ and/or R²) according to a conventional method. For example, the compounds of the embodiments (B) to (G) [such as the compounds (1B) to (1G)] may be prepared by allowing a specified reagent (or a reaction component) to react with the compound (1A), as described below.

For the preparation of the compound represented by the formula (1) (provided that the compound (1A) is excluded) from the compound (1A), the reaction may be carried out in the presence of an agent such as a conventional reducing agent and/or polymerization inhibitor from the viewpoint of the prevention of the reaction between the compounds (1A) (disulfidation).

Examples of the reducing agent may include a metal hydride [such as an alkali metal borohydride such as lithium borohydride, sodium borohydride, lithium triethylborohydride, lithium tri(s-butyl)borohydride, sodium tri(s-butyl)borohydride, potassium tri(s-butyl)borohydride, sodium triacetoxyborohydride, and sodium cyanoborohydride; an aluminum hydride such as lithium aluminum hydride, sodium bis(2-methoxyethoxy)aluminum hydride, and diisobutylaluminum hydride; nickel borohydride; zinc borohydride; and tributyltin hydride], a borane complex (such as borane-dimethyl sulfide complex and borane-tetrahydrofuran complex), a silane (such as triethylsilane), a phosphine (such as tris(2-carboxyethyl)phosphine hydrochloride), and a sulfite (such as sodium sulfite).

Examples of the polymerization inhibitor may include a quinone [such as hydroquinone, p-benzoquinone, t-butylhydroquinone, methoquinone (or p-methoxyphenol), toluquinone (or methyl-p-benzoquinone), and 2,5-diphenyl-p-benzoquinone], a catechol (such as t-butylcatechol), an amine (such as diphenylamine and diphenylpicrylhydrazyl), and a nitro compound (such as nitrobenzene).

Such a reducing agent and/or polymerization inhibitor may be used alone or in combination of two or more. Among these reducing agents and/or polymerization inhibitors, a metal hydride (such as an alkali metal borohydride such as sodium borohydride), a quinone (such as hydroquinone and methoquinone), and others are preferred.

### (Embodiment (B))

The embodiment (B) is a compound represented by the formula (1) in which at least one (preferably both) of R¹ and R² represents a group having a hydroxyl group or thiol group (mercapto group) [such as a hydroxyalkyl group and a hydroxy(poly)alkoxyalkyl group]. The compound especially preferably includes a compound in which both R¹ and R² represent the above-mentioned group [particularly, the group (2B)], such as the following compound (1B): 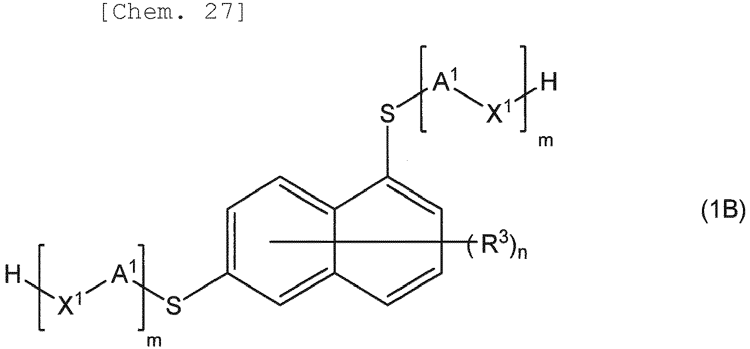
wherein A¹'s independently represent an alkylene group, X¹'s independently represent an oxygen atom or a sulfur atom, and m's independently denote 0 or an integer of not less than 1,
at least one m denotes not less than 1, and
R³ and n, including preferred embodiments, each have the same meanings as defined in the formula (1) .

In the formula (1B), preferred embodiments of each of A¹, X¹, and m may be the same as the corresponding preferred embodiments in the group (2B). A¹, X¹, and m in 1-position each may be different from those in 6-position, and are preferably the same as those in 6-position.

At least one of m's in the formula (1B) is not less than 1. Preferably, both of m's may be not less than 1 (such as an integer of 1 to 5), and further preferably, both of m's may be 1 or 2 (such as 1).

Preferred examples of the compound (1B) may include a 1,6-bis(hydroxyalkylthio)naphthalene [such as a 1,6-bis(hydroxyC₂₋₄alkylthio)naphthalene], a 1,6-bis[hydroxy-(poly)alkoxy-alkylthio]naphthalene [such as a 1,6-bis(hydroxy-mono- to pentaC₂₋₄alkoxy-C₂₋₄alkylthio)naphthalene such as 1,6-bis(hydroxyethoxyethylthio)naphthalene], a 1,6-bis(mercaptoalkylthio)naphthalene [such as a 1,6-bis(mercaptoC₂₋₄alkylthio)naphthalene], and a 1,6-bis[mercapto-(poly)alkylthio-alkylthio]naphthalene [such as a 1,6-bis(mercapto-mono- to pentaC₂₋₄alkylthio-C₂₋₄alkylthio)naphthalene such as 1,6-bis(mercaptoethylthioethylthio)naphthalene]; further preferred examples may include a 1,6-bis(hydroxyalkylthio)naphthalene [such as a 1,6-bis(hydroxyC₂₋₃alkylthio)naphthalene] and a 1,6-bis(mercaptoalkylthio)naphthalene [such as a 1,6-bis(mercaptoC₂₋₃alkylthio)naphthalene]; in particular, the compound (1B) may be a 1,6-bis(hydroxyalkylthio)naphthalene [such as a 1,6-bis(hydroxyC₂₋₃alkylthio)naphthalene such as 1,6-bis(hydroxyethylthio)naphthalene].

The compound (1B) may form a salt. The compound (1B) may form an alkali metal salt (such as a sodium salt and a potassium salt), and other salts.

As examples of a process for producing the compound (1B), there may be mentioned a process which includes a reaction (an addition reaction or a ring-opening reaction) of the compound (1A) with a reaction component corresponding to the group [-A¹-X¹-].

Examples of the reaction component corresponding to the group [-A¹-X¹-] wherein X¹ represents an oxygen atom may include an alkylene oxide, alkylene carbonate, and halogenated alcohol corresponding to the alkylene group A¹; examples of the reaction component corresponding to the group [-A¹-X¹-] wherein X¹ represents a sulfur atom may include an alkylene sulfide and alkylene trithiocarbonate corresponding to the alkylene group A¹. Such a reaction component may be used alone or in combination of two or more. In particular, preferred examples may include an alkylene carbonate and a halogenated alcohol, and further preferred examples may include a C₂₋₄alkylene-carbonate such as ethylene carbonate and propylene carbonate, and a halogenated C₂₋₄alcohol such as 2-chloroethanol.

In these methods, used as the compound (1A) may, for example, be the above-mentioned compound. Depending on the type of the reaction, the compound (1A) may be used as it is, or may be used in the form of an alkali metal salt such as a sodium salt (in the form of an alkali metal salt aqueous solution). For example, in the reaction with the alkylene carbonate, the compound (1A) may be subjected as it is to the reaction; in the reaction with the halogenated alcohol, the compound (1A) may be subjected in the form of an alkali metal salt such as a sodium salt (in the form of an alkali metal salt aqueous solution) to the reaction.

The alkali metal salt of the compound (1A) is prepared by a conventional method, for example, may be prepared by allowing the above-mentioned compound to react with an alkali metal hydroxide (such as sodium hydroxide). In this reaction, the ratio of the alkali metal hydroxide relative to 1 mol of the compound (1A) may, for example, be about 2 to 10 mol, preferably about 2.2 to 5 mol, and further preferably about 2.5 to 3 mol.

It is preferred that the alkali metal hydroxide be added in the form of an aqueous solution. The ratio of water may, for example, be about 100 to 1000 parts by mass, preferably about 200 to 800 parts by mass, and further preferably about 400 to 600 parts by mass, relative to 100 parts by mass of the compound (1A).

In order to prevent the disulfidation of the compound (1A), the preparation reaction of the alkali metal salt may be carried out in the presence of the conventional reducing agent and/or polymerization inhibitor for inhibiting disulfidation exemplified in the section of the embodiment (A). The reaction may preferably be carried out in the presence of the reducing agent (such as an alkali metal borohydride such as sodium borohydride). The ratio of the reducing agent relative to 1 mol of the compound (1A) may, for example, be about 0.0001 to 0.5 mol, preferably about 0.001 to 0.3 mol, and further preferably about 0.01 to 0.1 mol.

The reaction temperature of the preparation reaction of the alkali metal salt may, for example, be about 30 to 100°C, preferably about 40 to 80°C, and further preferably about 50 to 70°C. The reaction time may, for example, be about 0.1 to 12 hours, preferably about 0.3 to 3 hours, and further preferably about 0.5 to 2 hours.

The preparation reaction of the alkali metal salt may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, and drying, and a combination of these means, or may be subjected to the subsequent reaction (such as a reaction with a halogenated alcohol) without separation-purification.

The ratio of the reaction component of corresponding to the group [-A¹-X¹-] is suitably adjusted according to the repeating number m, and others. When both m's of the compound (1B) are 1, the above-mentioned ratio relative to 1 mol of the compound (1A) may, for example, be about 2 to 10 mol and preferably about 2.1 to 5 mol; for the reaction with the alkylene carbonate as the reaction component corresponding to the group [-A¹-X¹-], the ratio may further preferably be about 2.3 to 3 mol; for the reaction with the halogenated alcohol as the reaction component corresponding to the group [-A¹-X¹-], the ratio may further preferably be about 2.05 to 2.3 mol.

The reaction may be carried out in the presence or absence of a solvent. Examples of the solvent may include an inert or inactive organic solvent to the reaction, for example, an amide such as DMF and DMAc (preferably DMF), and water. For the reaction with the alkylene carbonate, an amide such as DMF may be used; for the reaction with the halogenated alcohol, water may be used. The solvent may be used alone or as a mixed solvent in combination of two or more. The ratio of the solvent is not particularly limited to a specific ratio, and may, for example, be about 100 to 2000 parts by mass, preferably about 300 to 1500 parts by mass, and further preferably about 500 to 1000 parts by mass, relative to 100 parts by mass of the compound (1A) or the salt thereof.

In order to prevent the disulfidation of the compound (1A), the reaction may be carried out in the presence of the conventional reducing agent and/or polymerization inhibitor for inhibiting disulfidation exemplified in the section of the embodiment (A). The ratio of the reducing agent and/or polymerization inhibitor may, for example, be about 0.01 to 10 parts by mass, preferably about 0.05 to 5 parts by mass, and further preferably about 0.1 to 1 part by mass, relative to 100 parts by mass of the compound (1A).

The reaction temperature may, for example, be about 50 to 150°C, preferably about 80 to 120°C, and further preferably about 90 to 110°C. The reaction time may, for example, be about 0.1 to 12 hours, preferably about 0.3 to 6 hours, and further preferably about 0.5 to 2 hours.

The reaction may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, and drying, and a combination of these means. For example, in a case where the alkylene carbonate (such as ethylene carbonate) is used as the reaction component corresponding to the group [-A¹-X¹-], the reaction product may be washed with an alkaline aqueous solution (such as a sodium hydroxide aqueous solution) in order to remove unreacted components.

### (Embodiment (C))

The embodiment (C) is a compound represented by the formula (1) in which at least one (preferably both) of R¹ and R² represents a group having a saturated hydrocarbon group which may have a halogen atom [such as a saturated hydrocarbon group which may have a halogen atom, including an alkyl group and a haloalkyl group, and a (poly)alkoxy-alkyl group]. The compound especially preferably includes a compound in which both R¹ and R² represent the above-mentioned group [particularly, the group (2C)], such as the following compound (1C): 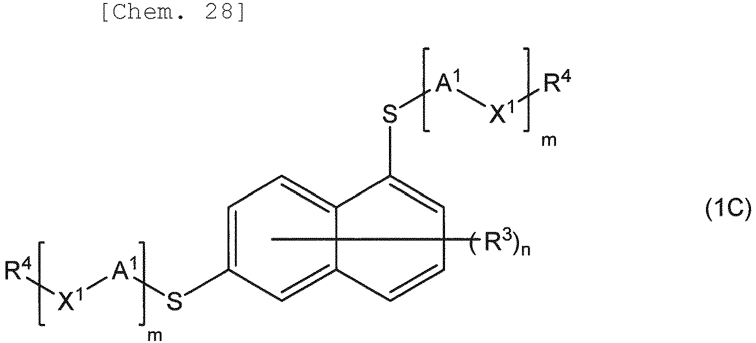
wherein A¹'s independently represent an alkylene group, X¹'s independently represent an oxygen atom or a sulfur atom, and m's independently denote 0 or an integer of not less than 1,
R⁴'s independently represent a saturated hydrocarbon group which may have a halogen atom, and
R³ and n, including preferred embodiments, each have the same meanings as defined in the formula (1) .

In the formula (1C), preferred embodiments of each of A¹, X¹, m, and R⁴ may be the same as the corresponding preferred embodiments in the group (2C). A¹, X¹, m, and R⁴ in 1-position each may be different from those in 6-position, and are preferably the same as those in 6-position.

Preferred examples of the compound (1C) may include the following:
a 1,6-bis(alkylthio)naphthalene [such as a 1,6-bis(C₁₋galkylthio)naphthalene],
a 1,6-bis(haloalkylthio)naphthalene [such as a 1,6-bis(haloC₁₋₈alkylthio)naphthalene],
a 1,6-bis(alkoxyalkylthio)naphthalene [such as a 1,6-bis(C₁₋₄alkoxyC₂₋₄alkylthio)naphthalene],
a 1,6-bis(haloalkoxyalkylthio)naphthalene [such as a 1,6-bis(haloC₁₋₄alkoxyC₂₋₄alkylthio)naphthalene],
a 1,6-bis(polyalkoxy-alkylthio)naphthalene [such as a 1,6-bis(C₁₋₄alkoxy-mono- to pentaC₂₋₄alkoxy-C₂₋₄alkylthio)naphthalene],
a 1,6-bis(haloalkoxy(poly)alkoxy-alkylthio)naphthalene [such as a 1,6-bis(haloC₁₋₄alkoxy-mono- to pentaC₂₋₄alkoxy-C₂₋₄alkylthio)naphthalene],
a 1,6-bis[(poly)alkylthio]naphthalene [such as a 1,6-bis(C₁₋₄alkylthioC₂₋₄alkylthio)naphthalene and a 1,6-bis(C₁₋₄alkylthio-di- to hexaC₂₋₄alkylthio)naphthalene], and
a 1,6-bis[haloalkylthio(poly)alkylthio]naphthalene [such as a 1,6-bis(haloC₁₋₄alkylthioC₂₋₄alkylthio)naphthalene and a 1,6-bis(haloC₁₋₄alkylthio-di- to hexaC₂₋₄alkylthio)naphthalene].

Further preferred examples may include a 1,6-bis(alkylthio)naphthalene [such as a 1,6-bis(C₁₋₄alkylthio)naphthalene], a 1,6-bis(haloalkylthio)naphthalene [such as a 1,6-bis(haloC₁₋₄alkylthio)naphthalene], a 1,6-bis(alkoxyalkylthio)naphthalene [such as a 1,6-bis(C₁₋₃alkoxyC₂₋₃alkylthio)naphthalene], a 1,6-bis(haloalkoxyalkylthio)naphthalene [such as a 1,6-bis(haloC₁₋₃alkoxyC₂₋₃alkylthio)naphthalene], a 1,6-bis(alkylthio)naphthalene [such as a 1,6-bis(C₁₋₃alkylthioC₂₋₃alkylthio)naphthalene], and a 1,6-bis(haloalkylthio)naphthalene [such as a 1,6-bis(haloC₁₋₃alkylthioC₂₋₃alkylthio)naphthalene]. Particularly preferred examples may include a 1,6-bis(alkylthio)naphthalene [such as a 1,6-bis(C₁₋₃alkylthio)naphthalene], a 1,6-bis(haloalkylthio)naphthalene [such as a 1,6-bis(haloC₁₋₃alkylthio)naphthalene], a 1,6-bis(alkoxyalkylthio)naphthalene [such as a 1,6-bis(C₁₋₂alkoxyC₂₋₃alkylthio)naphthalene], and a 1,6-bis(haloalkoxyalkylthio)naphthalene [such as a 1,6-bis(haloC₁₋₂alkoxyC₂₋₃alkylthio)naphthalene]. The compound (C) may particularly be a 1,6-bis(alkylthio)naphthalene [such as a 1,6-bis(C₁₋₂alkylthio)naphthalene such as 1,6-bis(methylthio)naphthalene], a 1,6-bis(haloalkylthio)naphthalene [such as a 1,6-bis(chloroC₁₋₂alkylthio)naphthalene such as 1,6-bis(2-chloroethylthio)naphthalene]. Among them, preferred is a 1,6-bis(alkylthio)naphthalene.

As examples of a process for producing the compound (1C), there may be mentioned a process which includes allowing the compound (1A) or the compound (1B) to react with a reaction component corresponding to the group [-R⁴] (such as an alkylating agent and a halogenating agent), and a process which includes allowing the compound (1A) to react with a halide (such as an iodide) having a halogen atom (such as an iodine atom) bonded to a corresponding group [-(A¹-X¹)ₘ-R⁴]. The former method is preferred, in particular, a process which includes allowing the compound (1A) or the compound (1B) [particularly the compound (1A)] to react with an alkylating agent corresponding to the group [-R⁴].

In these methods, used as the compound (1A) or the compound (1B) [particularly the compound (1A)] may for example be the above-mentioned compound. Depending on the type of the reaction (such as, for the reaction with the alkylating agent), the compound (1A) or the compound (1B) may be subjected in the form of an alkali metal salt such as a sodium salt (in the form of an alkali metal salt aqueous solution) to the reaction.

The alkali metal salt of the compound (1A) or the compound (1B) [particularly the compound (1A)] is prepared by a conventional method, for example, may be prepared by allowing the above-mentioned compound to react with an alkali metal hydroxide (such as sodium hydroxide). In this reaction, the ratio of the alkali metal hydroxide relative to 1 mol of the compound (1A) or the compound (1B) [particularly the compound (1A)] may, for example, be about 2 to 10 mol, preferably about 2.2 to 5 mol, and further preferably about 2.5 to 3 mol.

It is preferred that the alkali metal hydroxide be added in the form of an aqueous solution. The ratio of water may, for example, be about 100 to 1000 parts by mass, preferably about 200 to 800 parts by mass, and further preferably about 400 to 600 parts by mass, relative to 100 parts by mass of the compound (1A) or the compound (1B) [particularly the compound (1A)].

In order to prevent the disulfidation of the compound (1A) or the compound (1B) [particularly the compound (1A)], the preparation reaction of the alkali metal salt may be carried out in the presence of the conventional reducing agent and/or polymerization inhibitor for inhibiting disulfidation exemplified in the section of the embodiment (A). The reaction may preferably be carried out in the presence of the reducing agent (such as an alkali metal borohydride such as sodium borohydride). The ratio of the reducing agent relative to 1 mol of the compound (1A) or the compound (1B) [particularly the compound (1A)] may, for example, be about 0.0001 to 0.5 mol, preferably about 0.001 to 0.3 mol, and further preferably about 0.01 to 0.1 mol.

The reaction temperature of the preparation reaction of the alkali metal salt may, for example, be about 30 to 100°C, preferably about 40 to 80°C, and further preferably about 50 to 70°C. The reaction time may, for example, be about 0.1 to 12 hours, preferably about 0.3 to 3 hours, and further preferably about 0.5 to 2 hours.

The preparation reaction of the alkali metal salt may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, and drying, and a combination of these means, or may be subjected to the subsequent reaction (such as a reaction with an alkylating agent) without separation-purification.

In the reaction with the alkylating agent, examples of the alkylating agent corresponding to the group [-R⁴] may include an alkyl halide such as an alkyl iodide, and preferably a C₁₋₁₂alkyl iodide (such as a C₁₋₆alkyl iodide such as methyl iodide and ethyl iodide). The alkylating agent may be used alone or in combination of two or more.

The ratio of the alkylating agent relative to 1 mol of the compound (1A) or the compound (1B) [particularly the compound (1A)] (or an alkali metal salt thereof such as a sodium salt thereof) may, for example, be about 2 to 10 mol, preferably about 2.1 to 2.5 mol, and further preferably about 2.1 to 2.3 mol.

In order to prevent the disulfidation of the compound (1A) or the compound (1B) [particularly the compound (1A)], the reaction with the alkylating agent may be carried out in the presence of the conventional reducing agent and/or polymerization inhibitor for inhibiting disulfidation exemplified in the section of the embodiment (A). The reaction may preferably be carried out in the presence of the polymerization inhibitor (such as a quinone such as methoquinone). The ratio of the polymerization inhibitor may, for example, be about 0.01 to 10 parts by mass, preferably about 0.05 to 5 parts by mass, and further preferably about 0.1 to 1 part by mass, relative to 100 parts by mass of the compound (1A) or the compound (1B) [particularly the compound (1A)].

The reaction with the alkylating agent may be carried out in the presence of a solvent. Examples of the solvent may include an inert or inactive organic solvent to the reaction, for example, a chlorinated hydrocarbon (such as preferably methylene chloride). Such a solvent may be used alone or as a mixed solvent in combination of two or more. For the addition of the compound (1A) or the compound (1B) [particularly the compound (1A)] in the form of an alkali metal salt aqueous solution, the solvent may contain water. Specifically, the solvent may contain a chlorinated hydrocarbon such as methylene chloride and water, and the ratio of the former/the latter (mass ratio) may, for example, be about 30/70 to 90/10, preferably about 40/60 to 80/20, and further preferably about 50/50 to 70/30. The total amount of the solvent may, for example, be about 100 to 3000 parts by mass, preferably about 1000 to 2000 parts by mass, and further preferably about 1400 to 1800 parts by mass, relative to 100 parts by mass of the compound (1A) or the compound (1B) [particularly the compound (1A)].

The reaction temperature of the reaction with the alkylating agent may, for example, be about -30 to 30°C, preferably about -10 to 20°C, and further preferably about 0 to 10°C. The reaction time may, for example, be about 6 to 48 hours, preferably about 12 to 36 hours, and further preferably about 16 to 24 hours.

The reaction with the alkylating agent may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, drying, and distillation (such as distillation under a reduced pressure), and a combination of these means.

For the purification by distillation (such as distillation under a reduced pressure), the distillation may, for example, be carried out at pressure of about 1.5 to 2.5 hPa and preferably about 1.8 to 2.1 hPa, and a temperature of about 100 to 250°C (such as about 150 to 220°C) and preferably about 170 to 195°C. Moreover, a fraction may be obtained at a column top temperature (a top temperature) of, for example, about 120 to 230°C (about 160 to 210°C) and preferably about 165 to 185°C.

In a case where the group [-R⁴] is a saturated hydrocarbon group having a halogen atom (such as a haloalkyl group such as a chloroalkyl group), for example, the compound (1B) may be allowed to react with a halogenating agent (such as thionyl chloride and thionyl bromide) corresponding to the halogen atom, preparing the compound (1C). As the compound (1B) for the reaction, there may be used a compound prepared by the production process described in the embodiment (B). For example, the compound (1B) may be used after purification by crystallization or other means or may be used without purification by crystallization. For example, after extraction and washing of the obtained compound (1B), the resulting organic layer (a solution containing the compound (1B)) may be subjected to the reaction. The alkylating agent may be used alone or in combination of two or more.

The ratio of the halogenating agent relative to 1 mol of the compound (1B) may, for example, be about 2 to 10 mol, preferably about 2.1 to 2.5 mol, and further preferably about 2.2 to 2.4 mol.

The reaction with the halogenating agent may be carried out in the presence or absence of a solvent, and may preferably be carried out in the absence of a solvent. The reaction temperature of the reaction with the halogenating agent may, for example, be about 50 to 100°C, preferably about 60 to 80°C, and further preferably about 65 to 75°C. The reaction time may, for example, be about 1 to 48 hours, preferably about 6 to 36 hours, and further preferably about 12 to 24 hours.

The reaction with the halogenating agent may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as neutralization, washing, extraction, concentration, filtration, crystallization, centrifugal separation, column chromatography, activated carbon treatment, and drying, and a combination of these means.

### (Embodiment D)

The embodiment (D) is a compound represented by the formula (1) in which at least one of R¹ and R² represents a group having an unsaturated hydrocarbon group [such as a hydrocarbon group having one or more ethylenically unsaturated bond(s) (such as an alkenyl group such as vinyl group and allyl group), and an alkenyloxyalkyl group]. The compound especially preferably includes a compound in which both R¹ and R² represent the above-mentioned group [particularly the group (2D)], such as the following compound (1D): 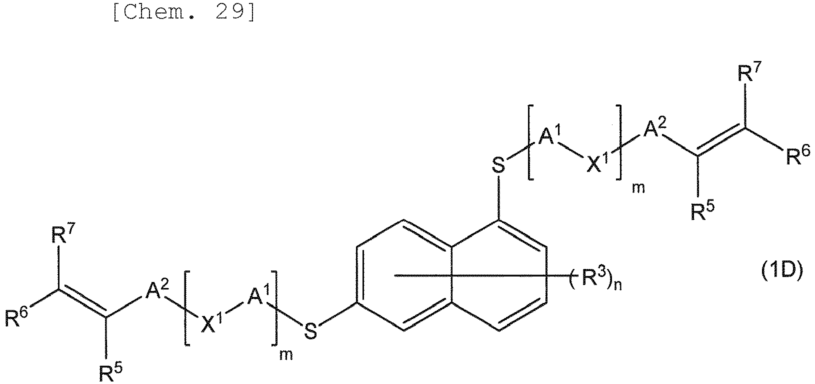
wherein A¹'s independently represent an alkylene group, X¹'s independently represent an oxygen atom or a sulfur atom, and m's independently denote 0 or an integer of not less than 1,
A²'s independently represent a direct bond (a single bond) or an alkylene group,
R⁵'s, R⁶'s and R⁷'s independently represent a hydrogen atom, or a substituted or unsubstituted hydrocarbon group [a hydrocarbon group which may be substituted],
R³ and n, including preferred embodiments, each have the same meanings as defined in the formula (1) .

In the formula (1D), preferred embodiments of each of A¹, X¹, m, A², R⁵, R⁶, and R⁷ may be the same as the corresponding preferred embodiments in the group (2D). A¹, X¹, m, A², R⁵, R⁶, and R⁷ in 1-position each may be different from those in 6-position, and are preferably the same as those in 6-position.

Preferred examples of the compound (1D) may include a 1,6-bis(alkenylthio)naphthalene [such as a 1,6-bis(C₂₋₆alkenylthio)naphthalene], a 1,6-bis(alkenyloxyalkylthio)naphthalene [such as a 1,6-bis(C₂₋₄alkenyloxyC₂₋₄alkylthio)naphthalene], a 1,6-bis[alkenyl-(poly)alkoxy-alkylthio]naphthalene [such as a 1,6-bis(C₂₋₄alkenyloxy-mono- to pentaC₂₋₄alkoxy-C₂₋₄alkylthio)naphthalene], and a 1,6-bis[alkenylthio-(poly)alkylthio]naphthalene [such as a 1,6-bis(C₂₋₄alkenylthioC₂₋₄alkylthio)naphthalene and a 1,6-bis(C₂₋₄alkenylthio-di- to hexaC₂₋₄alkylthio)naphthalene]. Further preferred examples may include a 1,6-bis(alkenylthio)naphthalene [such as a 1,6-bis(C₂₋₄alkenylthio)naphthalene], a 1,6-bis(alkenyloxyalkylthio)naphthalene [such as a 1,6-bis(C₂₋₃alkenyloxyC₂₋₃alkylthio)naphthalene], and a 1,6-bis(alkenylthioalkylthio)naphthalene [such as a 1,6-bis(C₂₋₃alkenylthioC₂₋₃alkylthio)naphthalene]. Particularly preferred examples may include a 1,6-bis(alkenylthio)naphthalene [such as a 1,6-bis(C₂₋₃alkenylthio)naphthalene such as 1,6-bis(vinylthio)naphthalene, 1,6-bis(allylthio)naphthalene, and 1,6-bis(isopropenylthio)naphthalene]. The compound (D) may particularly be 1,6-bis(vinylthio)naphthalene and 1,6-bis(allylthio)naphthalene [particularly 1,6-bis(allylthio)naphthalene]. From the point of view of more improvement in refractive index, 1,6-bis(vinylthio)naphthalene is preferred.

As examples of a process for producing the compound (1D), there may be mentioned the following:
a process which includes allowing the compound (1A) or the compound (1B) to react with a reaction component corresponding to the group [-A²-CR⁵=CR⁶R⁷] (such as an alkenylating agent);
a process which includes allowing the compound (1A) to react with a corresponding halide (such as an iodide) of the group [-(A¹-X¹)ₘ-A²-CR⁵=CR⁶R⁷] or a corresponding compound having a halogen atom (such as an iodine atom) bonded to the group [-(A¹-X¹)ₘ-A²-CR⁵=CR⁶R⁷]; and
in a case where A² represents a direct bond (a single bond), for example, the group [-A²-CR⁵=CR⁶R⁷] is vinyl group, a process which includes allowing the compound (1B) [such as a 1,6-bis(2-hydroxyethylthio)naphthalene] to react with a halogenating agent (such as thionyl chloride), and treating (dehydrohalogenating such as dehydrochlorinating) the resulting compound (1C) [specifically, a compound (1C) in which the group [-R⁴] is a haloalkyl group such as chloroethyl group, including a 1,6-bis(2-haloethylthio)naphthalene (such as 1,6-bis(2-chloroethylthio)naphthalene)] with a base (such as an aqueous solution of an alkali metal hydroxide such as sodium hydroxide and potassium hydroxide, and an alcoholic alkali metal hydroxide such as ethanolic potassium hydroxide).

Among these processes, in a case where A² represents an alkylene group, preferred is a process which includes allowing the compound (1A) or the compound (1B) [particularly the compound (1A)] to react with the alkenylating agent corresponding to the group [-A²-CR⁵=CR⁶R⁷]. As examples of the compound (1A) or the compound (1B) [particularly the compound (1A)], there may be mentioned the compound described above, and the compound (1A) or the compound (1B) is preferably subjected in the form of an alkali metal salt such as a sodium salt (in the form of an alkali metal salt aqueous solution) to the above-mentioned reaction. The alkali metal salt may be prepared according to the same manner as in the method described in the embodiment (C).

In the reaction with the alkenylating agent corresponding to the group [-A²-CR⁵=CR⁶R⁷], examples of the alkenylating agent may include an alkenyl halide such as an alkenyl iodide, and preferably a C₁₋₁₂alkenyl iodide (such as a C₁₋₆alkenyl iodide such as allyl iodide and isopropenyl iodide). The alkenylating agent may be used alone or in combination of two or more.

The ratio of the alkenylating agent relative to 1 mol of the compound (1A) or the compound (1B) [particularly the compound (1A)] (or an alkali metal salt thereof such as a sodium salt thereof) may, for example, be about 2 to 10 mol, preferably about 2.1 to 2.5 mol, and further preferably about 2.1 to 2.3 mol.

In order to prevent the disulfidation of the compound (1A) or the compound (1B) [particularly the compound (1A)], the reaction with the alkenylating agent may be carried out in the presence of the conventional reducing agent and/or polymerization inhibitor for inhibiting disulfidation exemplified in the section of the embodiment (A). The reaction may preferably be carried out in the presence of the polymerization inhibitor (such as a quinone such as methoquinone). The ratio of the polymerization inhibitor may, for example, be about 0.01 to 10 parts by mass, preferably about 0.05 to 5 parts by mass, and further preferably about 0.1 to 1 part by mass, relative to 100 parts by mass of the compound (1A) or the compound (1B) [particularly the compound (1A)].

The reaction with the alkenylating agent may be carried out in the presence of a solvent. Examples of the solvent may include an inert or inactive organic solvent to the reaction, for example, a chlorinated hydrocarbon (such as preferably methylene chloride). Such a solvent may be used alone or as a mixed solvent in combination of two or more. For the addition of the compound (1A) or the compound (1B) [particularly the compound (1A)] in the form of an alkali metal salt aqueous solution, the solvent may contain water. Specifically, the solvent may contain a chlorinated hydrocarbon such as methylene chloride and water, and the ratio of the former/the latter (mass ratio) may, for example, be about 40/60 to 90/10, preferably about 50/50 to 80/20, and further preferably about 60/40 to 70/30. The total amount of the solvent may, for example, be about 100 to 3000 parts by mass, preferably about 1000 to 2000 parts by mass, and further preferably about 1300 to 1700 parts by mass, relative to 100 parts by mass of the compound (1A) or the compound (1B) [particularly the compound (1A)].

The reaction temperature of the reaction with the alkenylating agent may, for example, be about -30 to 30°C, preferably about -10 to 20°C, and further preferably about 0 to 10°C. The reaction time may, for example, be about 1 to 48 hours, preferably about 6 to 36 hours, and further preferably about 12 to 24 hours.

The reaction with the alkenylating agent may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, and drying, and a combination of these means.

In the above-mentioned reaction (or dehydrohalogenation treatment such as dehydrochlorination) in a case where A² represents a direct bond (a single bond) (such as, in a case where the group [-A²-CR⁵=CR⁶R⁷] is vinyl group), that is, in the reaction of the compound (1C) in which the group [-R⁴] is a saturated hydrocarbon group which is substituted by a halogen atom (a haloalkyl group such as a chloroalkyl group) [such as a 1,6-bis(2-haloethylthio)naphthalene such as 1,6-bis(2-chloroethylthio)naphthalene] with a base, examples of the base may include an alkali metal hydroxide such as sodium hydroxide and potassium hydroxide. The base may be in the form of an aqueous solution or may be in the form of an alcohol solution (the form of a C₁₋₄alcohol solution such as an ethanol solution), and may preferably be in an aqueous solution.

In the reaction of the compound (1C) with the base, the ratio of the base relative to 1 mol of the compound (1C) may, for example, be, about 2 to 10 mol, preferably about 2.2 to 5 mol, and further preferably about 2.5 to 3 mol.

The reaction of the compound (1C) with the base may be carried out in the presence or absence of a phase transfer catalyst. Examples of the phase transfer catalyst may include a conventional quaternary ammonium salt, including a tetraalkylammonium salt (such as a tetramethylammonium salt, a tetraethylammonium salt, a tetra-n-propylammonium salt, a tetra-n-butylammonium salt, a trioctylmethylammonium salt, a trilaurylmethylammonium salt, and a cetyltrimethylammonium salt), an aryltrialkylammonium salt (such as a phenyltrimethylammonium salt), and an aralkyltrialkylammonium salt (such as a benzyltrimethylammonium salt, a benzyltriethylammonium salt, and a benzyltri-n-butylammonium salt). Examples of the alkylammonium salt may include a halide (a halogenide) such as a chloride, a bromide, and an iodide, a hydroxide, and a hydrogen sulfate.

Such a phase transfer catalyst may be used alone or in combination of two or more. Among these phase transfer catalysts, a tetraalkylammonium halide such as tetrabutylammonium bromide is preferred. The ratio of the phase transfer catalyst relative to 1 mol of the compound (1C) may, for example, be about 0.001 to 0.5 mol, preferably about 0.01 to 0.1 mol, and further preferably about 0.03 to 0.07 mol.

The reaction of the compound (1C) with the base may be carried out in the presence of a solvent. Examples of the solvent may include an inert or inactive organic solvent to the reaction, for example, a hydrocarbon (such as an aliphatic hydrocarbon, an alicyclic hydrocarbon such as cyclohexane, and an aromatic hydrocarbon such as toluene, preferably an aromatic hydrocarbon such as toluene). The solvent may be used alone or as a mixed solvent in combination of two or more. For the addition of the base in the form of an aqueous solution, the solvent may contain water. Specifically, the solvent may contain an aromatic hydrocarbon such as toluene and water, and the ratio of the former/the latter (mass ratio) may, for example, be about 50/50 to 99/1, preferably about 70/30 to 97/3, and further preferably about 80/20 to 95/5. The total amount of the solvent may, for example, be about 100 to 3000 parts by mass, preferably about 100 to 1000 parts by mass, and further preferably about 300 to 500 parts by mass, relative to 100 parts by mass of the compound (1C).

The reaction temperature of the reaction of the compound (1C) with the base may, for example, be about 60 to 100°C, preferably about 70 to 90°C, and further preferably about 75 to 85°C. The reaction time may, for example, be about 1 to 48 hours, preferably about 6 to 24 hours, and further preferably about 12 to 18 hours.

The reaction of the compound (1C) with the base may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, and drying, and a combination of these means.

### (Embodiment E)

The embodiment (E) is a compound represented by the formula (1) in which at least one of R¹ and R² represents a group having (meth)acryloyl group or a thio(meth)acryloyl group [such as (meth)acryloyl group, a (meth)acryloyloxyalkyl group, and a (meth)acryloyloxy(poly)alkoxyalkyl group]. The compound especially preferably includes a compound in which both R¹ and R² represent the above-mentioned group [particularly the group (2E)], such as the following compound (1E): 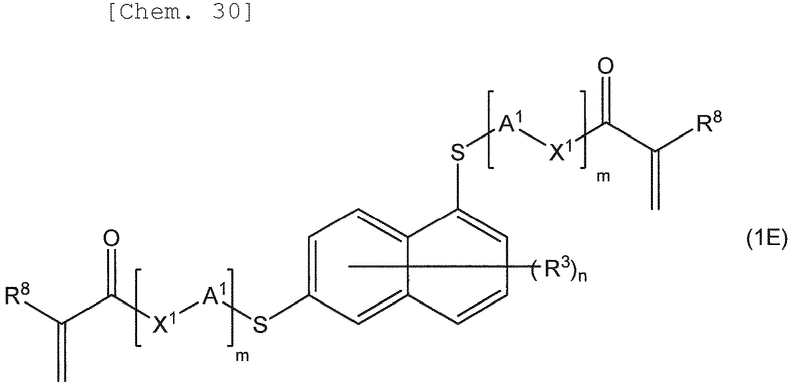
wherein A¹'s independently represent an alkylene group, X¹'s independently represent an oxygen atom or a sulfur atom, and m's independently denote 0 or an integer of not less than 1,
R⁸'s independently represent a hydrogen atom or a methyl group, and
R³ and n, including preferred embodiments, each have the same meanings as defined in the formula (1) .

In the formula (1E), preferred embodiments of each of A¹, X¹, m, and R⁸ may be the same as the corresponding preferred embodiments in the group (2E). A¹, X¹, m, and R⁸ in 1-position each may be different from those in 6-position, and are preferably the same as those in 6-position.

Preferred examples of the compound (1E) may include a 1,6-bis[(meth)acryloylthio]naphthalene, a 1,6-bis[(meth)acryloyloxyalkylthio]naphthalene [such as a 1,6-bis[(meth)acryloyloxyC₂₋₄alkylthio]naphthalene], and a 1,6-bis[(meth)acryloyloxy-(poly)alkoxy-alkylthio]naphthalene [such as a 1,6-bis[(meth)acryloyloxy-mono- to pentaC₂₋₄alkoxy-C₂₋₄alkylthio]naphthalene such as 1,6-bis[(meth)acryloyloxyethoxyethylthio]naphthalene]; further preferred examples may include a 1,6-bis[(meth)acryloylthio]naphthalene (such as 1,6-bis[(meth)acryloylthio]naphthalene) and a 1,6-bis[(meth)acryloyloxyalkylthio]naphthalene [such as a 1,6-bis[(meth)acryloyloxyC₂₋₃alkylthio]naphthalene such as 1,6-bis[(meth)acryloyloxyethylthio]naphthalene]; particularly preferred examples may include a 1,6-bis[(meth)acryloylthio]naphthalene (particularly, such as 1,6-bis(methacryloylthio)naphthalene).

As examples of a process for producing the compound (1E), there may be mentioned a process which includes allowing the compound (1A) or the compound (1B) [particularly the compound (1A)] to react with a reaction component corresponding to the (meth)acryloyl group [-C(=O)-CR⁸=CH₂].

As examples of the compound (1A) or the compound (1B) [particularly the compound (1A)], there may be mentioned the compound described above, and the compound (1A) or the compound (1B) is preferably subjected in the form of an alkali metal salt such as a sodium salt (in the form of an alkali metal salt aqueous solution) to the above-mentioned reaction. The alkali metal salt may be prepared according to the same manner as in the method described in the embodiment (C).

Examples of the reaction component corresponding to the (meth)acryloyl group [-C(=O)-CR⁸=CH₂] may include a (meth)acryloyl halide such as (meth)acryloyl chloride, (meth)acryloyl bromide, and (meth)acryloyl iodide (preferably a methacryloyl halide). The reaction component may preferably be (meth)acryloyl chloride (particularly methacryloyl chloride). Such a reaction component may be used alone or in combination of two or more.

The ratio of the reaction component corresponding to the (meth)acryloyl group [-C(=O)-CR⁸=CH₂] relative to 1 mol of the compound (1A) or the compound (1B) [particularly the compound (1A)] (or an alkali metal salt thereof such as a sodium salt thereof) may, for example, be about 2 to 10 mol, preferably about 2.1 to 2.5 mol. For a methacryloyl halide (such as methacryloyl chloride), the ratio may further preferably be about 2.3 to 2.5 mol; for an acryloyl halide (such as acryloyl chloride), the ratio may further preferably be about 2.1 to 2.3 mol.

In order to prevent the disulfidation of the compound (1A) or the compound (1B) [particularly the compound (1A)], the reaction with the reaction component corresponding to the (meth)acryloyl group [-C(=O)-CR⁸=CH₂] may be carried out in the presence of the conventional reducing agent and/or polymerization inhibitor for inhibiting disulfidation exemplified in the section of the embodiment (A). The reaction may preferably be carried out in the presence of the polymerization inhibitor (such as a quinone such as methoquinone). The ratio of the polymerization inhibitor may, for example, be about 0.01 to 10 parts by mass, preferably about 0.05 to 5 parts by mass, relative to 100 parts by mass of the compound (1A) or the compound (1B) [particularly the compound (1A)]. For a methacryloyl halide (such as methacryloyl chloride), the ratio may further preferably be about 0.1 to 1 part by mass; for an acryloyl halide (such as acryloyl chloride), the ratio may further preferably be about 0.5 to 1.5 parts by mass.

The reaction with the reaction component corresponding to the (meth)acryloyl group [-C(=O)-CR⁸=CH₂] may be carried out in the presence of a solvent. Examples of the solvent may include an inert or inactive organic solvent to the reaction, for example, a chlorinated hydrocarbon (such as preferably methylene chloride). Such a solvent may be used alone or as a mixed solvent in combination of two or more. For the addition of the compound (1A) or the compound (1B) [particularly the compound (1A)] in the form of an alkali metal salt aqueous solution, the solvent may contain water. Specifically, the solvent may contain a chlorinated hydrocarbon such as methylene chloride and water. For a methacryloyl halide (such as methacryloyl chloride), the ratio of the former/the latter (mass ratio) may, for example, be about 40/60 to 90/10, preferably about 50/50 to 80/20, and further preferably about 60/40 to 70/30; for an acryloyl halide (such as acryloyl chloride), the ratio of the former/the latter (mass ratio) may, for example, be about 60/40 to 99/1, preferably about 70/30 to 95/5, and further preferably about 80/20 to 90/10. For a methacryloyl halide (such as methacryloyl chloride), the total amount of the solvent relative to 100 parts by mass of the compound (1A) or the compound (1B) [particularly the compound (1A)] may, for example, be about 100 to 3000 parts by mass, preferably about 1000 to 2000 parts by mass, and further preferably about 1300 to 1700 parts by mass; for an acryloyl halide (such as acryloyl chloride), the total amount of the solvent relative to 100 parts by mass of the compound (1A) or the compound (1B) [particularly the compound (1A)] may, for example, be about 1000 to 5000 parts by mass, preferably about 2000 to 4000 parts by mass, and further preferably about 3000 to 3500 parts by mass.

The reaction temperature of the reaction with the reaction component corresponding to the (meth)acryloyl group [-C(=O)-CR⁸=CH₂] may, for example, be about -30 to 30°C, preferably about -10 to 20°C, and further preferably about -5 to 15°C (such as 0 to 10°C). The reaction time may, for example, be about 0.1 to 3 hours, preferably about 0.3 to 2 hours, and further preferably about 0.5 to 1.5 hours.

The reaction with the reaction component corresponding to the (meth)acryloyl group [-C(=O)-CR⁸=CH₂] may preferably be carried out under light shielding. The reaction may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as washing, extraction, concentration, filtration, crystallization, centrifugal separation, column chromatography, activated carbon treatment, and drying, and a combination of these means.

### (Embodiment F)

The embodiment (F) is a compound represented by the formula (1) in which at least one of R¹ and R² represents a group having an epoxy ring (an oxirane ring) or a thiirane ring [such as glycidyl group, β-methylglycidyl group, a glycidyloxyalkyl group, a glycidyloxy(poly)alkoxyalkyl group, and a group corresponding to such a group and having a thiirane ring instead of an epoxy ring]. The compound especially preferably includes a compound in which both R¹ and R² represent the above-mentioned group [particularly the group (2F)], such as the following compound (1F): 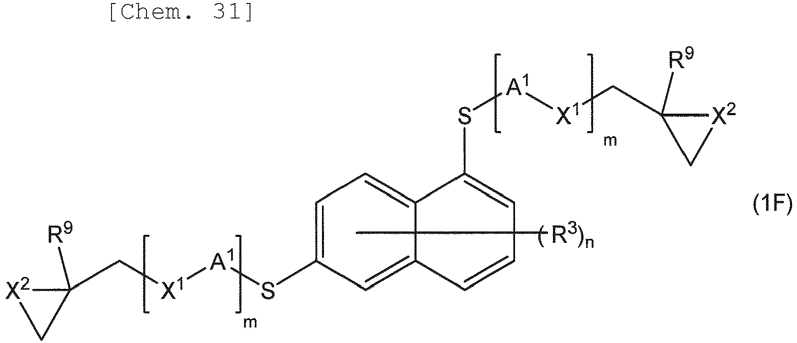
wherein A¹'s independently represent an alkylene group, X¹'s independently represent an oxygen atom or a sulfur atom, and m's independently denote 0 or an integer of not less than 1,
X²'s independently represent an oxygen atom or a sulfur atom,
R⁹'s independently represent a hydrogen atom or a methyl group, and
R³ and n, including preferred embodiments, each have the same meanings as defined in the formula (1) .

In the formula (1F), preferred embodiments of each of A¹, X¹, m, X², and R⁹ may be the same as the corresponding preferred embodiments in the group (2F) . A¹, X¹, m, X², and R⁹ in 1-position each may be different from those in 6-position, and are preferably the same as those in 6-position.

Preferred examples of the compound (1F) may include a 1,6-bis(glycidylthio)naphthalene, a 1,6-bis(glycidyloxyalkylthio)naphthalene [such as a 1,6-bis(glycidyloxyC₂₋₄alkylthio)naphthalene], a 1,6-bis[glycidyloxy-(poly)alkoxy-alkylthio]naphthalene [such as a 1,6-bis(glycidyloxy-mono- to pentaC₂-₄alkoxy-C₂₋₄alkylthio)naphthalene such as 1,6-bis(glycidyloxyethoxyethylthio)naphthalene], and a compound corresponding to such a compound and having β-methylglycidyl group or 2,3-epithiopropyl group instead of glycidyl group; further preferred examples may include a 1,6-bis(glycidylthio)naphthalene, a 1,6-bis(glycidyloxyalkylthio)naphthalene [such as a 1,6-bis(glycidyloxyC₂₋₃alkylthio) naphthalene such as 1,6-bis(glycidyloxyethylthio)naphthalene], and a compound corresponding to such a compound and having β-methylglycidyl group or 2,3-epithiopropyl group instead of glycidyl group; and particularly preferred examples may include 1,6-bis(glycidylthio)naphthalene, 1,6-bis(β-methylglycidylthio)naphthalene, and 1,6-bis(2,3-epithiopropylthio)naphthalene [such as 1,6-bis(glycidylthio)naphthalene, 1,6-bis(β-methylglycidylthio)naphthalene, preferably 1,6-bis(glycidylthio)naphthalene].

The compound (1F) may or may not contain a polymer produced in the production process [specifically, a polymer (or a polynuclear compound) having two or more naphthalene-1,6-dithio skeletons (that is, two or more constituent units represented by the formula (1P)) in a chemical structure thereof, such as a di- to decamer].

As examples of a process for producing the compound (1F), there may be mentioned a process which includes allowing the compound (1A) or the compound (1B) to react with a reaction component corresponding to X² and R⁹ (such as an epihalohydrin and an epithiohalohydrin); and in a case where X² represents a sulfur atom, a process which includes allowing a corresponding glycidyl compound having an oxygen atom as X² to react with a thiiranating agent for converting an epoxy ring into a thiirane ring (or converting X² into a sulfur atom), wherein the corresponding glycidyl compound may be obtained by the above-mentioned process [the reaction of the compound (1A) or (1B) with the epihalohydrin], and wherein examples of the thiiranating agent may include thiourea, and thiocyanic acid or a salt thereof (such as potassium thiocyanate).

Among these processes, preferred is a process which includes allowing the compound (1A) or the compound (1B) [particularly the compound (1A)] to react with the reaction component corresponding to X² and R⁹ (such as an epihalohydrin and an epithiohalohydrin).

Examples of the epihalohydrin may include an epihalohydrin (such as epichlorohydrin and epibromohydrin) and a β-methylepihalohydrin (or a 2-methyl-2-halomethyloxirane, such as β-methylepichlorohydrin).

Examples of the epithiohalohydrin may include an epithiohalohydrin [or a halomethylthiirane, including epithiochlorohydrin (or chloromethylthiirane)] and a β-methylepithiohalohydrin [or a 2-methyl-2-halomethylthiirane, including β-methylepithiochlorohydrin (or 2-methyl-2-chloromethylthiirane)].

Such a reaction component corresponding to X² and R⁹ may be used alone or in combination of two or more. Among these reaction components, an epihalohydrin and an epithiohalohydrin (such as epithiochlorohydrin) are preferred, and an epihalohydrin (such as an epichlorohydrin such as epichlorohydrin and β-methylepichlorohydrin), particularly an epihalohydrin (such as epichlorohydrin), is further preferred.

The ratio of the reaction component corresponding to X² and R⁹ relative to 1 mol of the compound (1A) or the compound (1B) [particularly the compound (1A)] may, for example, be, not less than 2 mol, and usually, the reaction component may preferably be added at an excess ratio (such as about 5 to 100 mol), such as about 10 to 80 mol, preferably about 20 to 50 mol, and further preferably about 30 to 40 mol, relative to 1 mol of the compound (1A) or the compound (1B). The excess reaction component may function as a solvent.

In order to fully accelerate the reaction of the compound (1A) or the compound (1B) [particularly the compound (1A)] with the reaction component corresponding to X² and R⁹, the reaction may preferably be carried out in the presence of a phase transfer catalyst. Examples of the phase transfer catalyst may include a conventional quaternary ammonium salt, including a tetraalkylammonium salt (such as a tetramethylammonium salt, a tetraethylammonium salt, a tetra-n-propylammonium salt, a tetra-n-butylammonium salt, a trioctylmethylammonium salt, a trilaurylmethylammonium salt, and a cetyltrimethylammonium salt), an aryltrialkylammonium salt (such as a phenyltrimethylammonium salt), and an aralkyltrialkylammonium salt (such as a benzyltrimethylammonium salt, a benzyltriethylammonium salt, and a benzyltri-n-butylammonium salt). Examples of the alkylammonium salt may include a halide (a halogenide) such as a chloride, a bromide, and an iodide, a hydroxide, and a hydrogen sulfate.

Such a phase transfer catalyst may be used alone or in combination of two or more. Among these phase transfer catalysts, an aralkyltrialkylammonium halide such as benzyltributylammonium chloride is preferred. The ratio of the phase transfer catalyst relative to 1 mol of the compound (1A) or the compound (1B) [particularly the compound (1A)] may, for example, be about 0.1 to 1 mol, preferably about 0.3 to 0.7 mol, and further preferably about 0.4 to 0.6 mol. The phase transfer catalyst may be used in the form of an aqueous solution, and the aqueous solution may, for example, have a concentration of about 10 to 90% by mass, preferably about 30 to 70% by mass, and further preferably about 40 to 60% by mass.

In order to prevent the disulfidation of the compound (1A) or the compound (1B) [particularly the compound (1A)], the reaction may be carried out in the presence of the conventional reducing agent and/or polymerization inhibitor for inhibiting disulfidation exemplified in the section of the embodiment (A). The ratio of the reducing agent and/or polymerization inhibitor may, for example, be about 0.01 to 10 parts by mass, preferably about 0.05 to 5 parts by mass, and further preferably about 0.1 to 1 part by mass, relative to 100 parts by mass of the compound (1A) or the compound (1B) [particularly the compound (1A)].

The reaction temperature may, for example, be about 50 to 150°C, preferably about 60 to 100°C, and further preferably about 70 to 90°C. The reaction time may, for example, be about 0.1 to 12 hours, preferably about 0.5 to 6 hours, and further preferably about 1 to 3 hours.

The reaction may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure.

After the completion of the reaction, the reaction product may be subjected to a treatment with a base (dehydrohalogenation, or ring closure treatment or cyclization). Examples of the base may include an inorganic base such as a metal hydroxide (such as an alkali metal hydroxide such as sodium hydroxide and potassium hydroxide). Such a base may be used alone or in combination of two or more. Among these bases, an alkali metal hydroxide such as sodium hydroxide is preferred. The ratio of the base relative to 1 mol of the compound (1A) or the compound (1B) [particularly the compound (1A)] may, for example, be about 2 to 10 mol, preferably about 2.3 to 5 mol, and further preferably about 2.5 to 3.5 mol. The base may be used in the form of an aqueous solution, and the aqueous solution may, for example, have a concentration of about 1 to 30% by mass, preferably about 5 to 20% by mass, and further preferably about 10 to 15% by mass.

The treatment temperature with the base may, for example, be about 10 to 100°C, preferably about 20 to 60°C, and further preferably about 30 to 50°C. The treatment time may, for example, be about 10 to 120 minutes, preferably about 20 to 90 minutes, and further preferably about 30 to 60 minutes.

After the completion of the treatment, the reaction product may be separated with purification by a conventional separation-purification means such as washing, extraction, concentration, filtration, crystallization, centrifugal separation, column chromatography, activated carbon treatment, and drying, and a combination of these means.

### (Embodiment G)

The embodiment (G) is a compound represented by the formula (1) in which at least one of R¹ and R² represents a group having a carboxyl group or a group derived from a carboxyl group (such as an alkoxycarbonyl group and a halocarbonyl group) [such as a carboxyalkyl group, an alkoxycarbonylalkyl group, a halocarbonylalkyl group, a carboxyalkoxyalkyl group, an alkoxycarbonylalkoxyalkyl group, and a halocarbonylalkoxyalkyl group]. The compound especially preferably includes a compound in which both R¹ and R² represent the above-mentioned group [particularly the group (2G)], such as the following compound (1G): 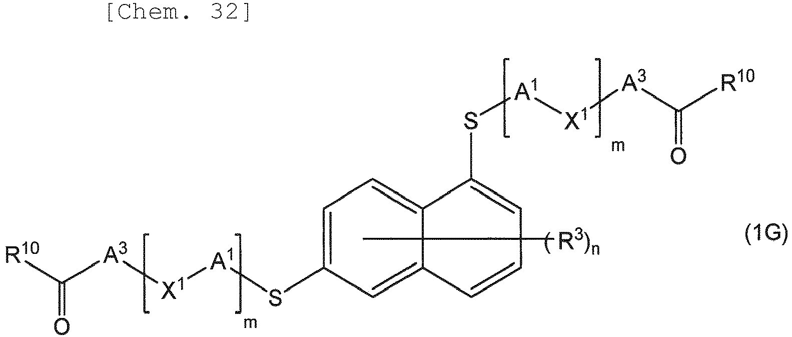
wherein A¹'s independently represent an alkylene group, X¹'s independently represent an oxygen atom or a sulfur atom, and m's independently denote 0 or an integer of not less than 1,
A³'s independently represent an alkylene group,
R¹⁰'s represent a hydroxyl group, a group [-OR¹¹] (wherein R¹¹ represents a substituted or unsubstituted hydrocarbon group), or a halogen atom, and
R³ and n, including preferred embodiments, each have the same meanings as defined in the formula (1) .

In the formula (1G), preferred embodiments of each of A¹, X¹, m, A³, R¹⁰, and R¹¹ may be the same as the corresponding preferred embodiments in the group (2G) . A¹, X¹, m, A³, R¹⁰, and R¹¹ in 1-position each may be different from those in 6-position, and are preferably the same as those in 6-position.

Preferred examples of the compound (1G) may include the following:
a 1,6-bis(carboxyalkylthio)naphthalene [such as a 1,6-bis(carboxyC₁₋₆alkylthio)naphthalene];
a 1, 6-bis (alkoxycarbonylalkylthio) naphthalene [such as a 1,6-bis(C₁₋₆alkoxy-carbonyl-C₁₋₆alkylthio)naphthalene];
a 1,6-bis(halocarbonylalkylthio)naphthalene [such as a 1,6-bis(halocarbonylC₁₋₆alkylthio)naphthalene];
a 1,6-bis[carboxy-(poly)alkoxy-alkylthio]naphthalene [such as a 1,6-bis(carboxyC₁₋₆alkoxyC₂₋₆alkylthio)naphthalene and a 1,6-bis(carboxyC₁₋₆alkoxy-mono- to decaC₂₋₆alkoxy-C₂₋₆alkylthio)naphthalene];
a 1,6-bis[alkoxycarbonyl-(poly)alkoxy-alkylthio]naphthalene [such as a 1,6-bis(C₁₋₆alkoxycarbonyl-C₁₋₆alkoxyC₂₋₆alkylthio)naphthalene and a 1,6-bis(C₁₋₆alkoxy-carbonyl-C₁₋₆alkoxy-mono- to decaC₂₋₆alkoxy-C₂₋₆alkylthio)naphthalene];
a 1,6-bis[halocarbonyl-(poly)alkoxy-alkylthio]naphthalene [such as a 1,6-bis(halocarbonyl-C₁₋₆alkoxyC₂₋₆alkylthio)naphthalene and a 1,6-bis(halocarbonyl-C₁₋₆alkoxy-mono- to decaC₂₋₆alkoxy-C₂₋₆alkylthio)naphthalene];
a 1,6-bis[carboxy-polyalkylthio]naphthalene [such as a 1,6-bis(carboxyC₁₋₆alkylthioC₂₋₆alkylthio)naphthalene and a 1,6-bis(carboxyC₁₋₆alkylthio-mono- to decaC₂-₆alkylthio)naphthalene];
a 1,6-bis[alkoxycarbonyl-polyalkylthio]naphthalene [such as a 1,6-bis(C₁₋₆alkoxy-carbonyl-C₁₋₆alkylthioC₂₋₆alkylthio)naphthalene and a 1,6-bis(C₁₋₆alkoxy-carbonyl-C₁₋₆alkylthio-mono- to decaC₂₋₆alkylthio)naphthalene]; and
a 1,6-bis[halocarbonyl-polyalkylthio]naphthalene [such as a 1,6-bis(halocarbonyl-C₁₋₆alkylthioC₂₋₆alkylthio)naphthalene and a 1,6-bis(halocarbonyl-C₁₋₆alkylthio-mono- to decaC₂₋₆alkylthio)naphthalene]. Further preferred examples may include a 1,6-bis(carboxyalkylthio)naphthalene [such as a 1,6-bis(carboxyC₁₋₄alkylthio)naphthalene]; a 1,6-bis(alkoxycarbonylalkylthio)naphthalene [such as a 1,6-bis(C₁₋₄alkoxy-carbonyl-C₁₋₄alkylthio)naphthalene]; a 1,6-bis(halocarbonylalkylthio)naphthalene [such as a 1,6-bis(halocarbonylC₁₋₄alkylthio)naphthalene]; a 1,6-bis(carboxyalkoxyalkylthio)naphthalene [such as a 1,6-bis(carboxyC₁₋₄alkoxyC₂₋₄alkylthio)naphthalene]; a 1, 6-bis (alkoxycarbonylalkoxyalkylthio) naphthalene [such as a 1,6-bis(C₁₋₄alkoxy-carbonyl-C₁₋₄alkoxyC₂₋₄alkylthio)naphthalene]; a 1,6-bis(halocarbonylalkoxyalkylthio)naphthalene [such as a 1,6-bis(halocarbonyl-C₁₋₄alkoxyC₂₋₄alkylthio)naphthalene]; a 1,6-bis[carboxydi(alkylthio)]naphthalene [such as a 1,6-bis(carboxyC₁₋₄alkylthioC₂₋₄alkylthio)naphthalene]; a 1,6-bis[alkoxycarbonyldi(alkylthio)]naphthalene [such as a 1,6-bis(C₁₋₄alkoxy-carbonyl-C₁₋₄alkylthioC₂₋₄alkylthio)naphthalene and a 1,6-bis(C₁₋₄alkoxycarbonyl-C₁₋₄alkylthio-mono- to decaC₂₋₄alkylthio)naphthalene]; and a 1,6-bis[halocarbonyldi(alkylthio)]naphthalene [such as a 1,6-bis(halocarbonyl-C₁₋₄alkylthioC₂₋₄alkylthio)naphthalene]. Particularly preferred examples may include a 1,6-bis(carboxyalkylthio)naphthalene [such as a 1,6-bis(carboxyC₁₋₃alkylthio)naphthalene]; a 1,6-bis(alkoxycarbonylalkylthio)naphthalene [such as a 1,6-bis(C₁₋₃alkoxy-carbonyl-C₁₋₃alkylthio)naphthalene]; a 1,6-bis(halocarbonylalkylthio)naphthalene [such as a 1,6-bis(halocarbonylC₁₋₃alkylthio)naphthalene]. The compound (1G) may particularly be a 1,6-bis(carboxyalkylthio)naphthalene [such as a 1,6-bis(carboxyC₁₋₂alkylthio)naphthalene such as 1,6-bis(carboxymethylthio)naphthalene].

The compound (1G) may form a salt. For example, when R¹⁰ represents a hydroxyl group, the group (1G) may form an alkali metal salt (such as a sodium salt and a potassium salt), an onium salt (such as an ammonium salt), and other salts.

As examples of a process for producing the compound (1G), there may be mentioned a process which includes allowing the compound (1A) or the compound (1B) to react with a reaction component corresponding to the group [-A³-C(=O)-R¹⁰] (such as a carboxyalkylating agent), and a process which includes allowing the compound (1A) to react with a corresponding halide (such as a chloride) in which a halogen atom (such as a chlorine atom) is bonded to the group [-(A¹-X¹)ₘ-A³-C(=O)-R¹⁰]; the former reaction process is preferred.

As examples of the compound (1A) or the compound (1B) [particularly the compound (1A)], there may be mentioned the compound described above, and the compound (1A) or the compound (1B) is preferably subjected in the form of an alkali metal salt such as a sodium salt (in the form of an alkali metal salt aqueous solution) to the above-mentioned reaction. The alkali metal salt may be prepared according to the same manner as in the method described in the embodiment (C); the alkali metal salt may be prepared in the same manner as the method described in the embodiment (C), or may preferably be prepared in the same manner as the method described in the embodiment (C) except that the ratio of the alkali metal hydroxide relative to 1 mol of the compound (1A) or the compound (1B) [particularly the compound (1A)] is, for example, about 3 to 10 mol, preferably about 5 to 7 mol, and further preferably about 5.5 to 6.5 mol.

The reaction component corresponding to the group [-A³-C(=O)-R¹⁰] (such as a carboxyalkylating agent) may include an alkyl ester of a haloalkanoic acid (such as a C₁₋₆alkyl ester of a haloC₂₋₆alkanoic acid, such as methyl chloroacetate, ethyl chloroacetate, and ethyl bromoacetate). Such a reaction component may be used alone or in combination of two or more.

The ratio of the carboxyalkylating agent relative to 1 mol of the compound (1A) or the compound (1B) [particularly the compound (1A)] (or an alkali metal salt thereof such as a sodium salt thereof) may, for example, be about 2 to 10 mol, preferably about 2.2 to 3 mol, and further preferably about 2.3 to 2.5 mol.

The reaction with the carboxyalkylating agent may be carried out in the presence of a solvent. Examples of the solvent may include an inert or inactive solvent to the reaction, for example, water. The water may be water used for the preparation of the compound (1A) or the compound (1B) [particularly the compound (1A)] into the form of an alkali metal salt, such as a sodium salt. The solvent may be used alone or as a mixed solvent in combination of two or more. The total amount of the solvent may, for example, be about 10 to 3000 parts by mass, preferably about 100 to 1000 parts by mass, and further preferably about 200 to 400 parts by mass, relative to 100 parts by mass of the compound (1A) or the compound (1B) [particularly the compound (1A)].

The reaction temperature of the reaction with the carboxyalkylating agent may, for example, be about 50 to 100°C, preferably about 70 to 85°C, and further preferably about 75 to 80°C. The reaction time may, for example, be about 0.1 to 12 hours, preferably about 0.5 to 6 hours, and further preferably about 1 to 3 hours.

The reaction with the carboxyalkylating agent may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure. After the completion of the reaction, the reaction product may be separated with purification by a conventional separation-purification means such as neutralization, washing, extraction, concentration, filtration, crystallization, centrifugal separation, column chromatography, activated carbon treatment, and drying, and a combination of these means.

In the reaction with the carboxyalkylating agent, the compound (1G) in which R¹⁰ represents a hydroxyl group, in other words, which has a carboxyl group, is usually obtained. The carboxyl groups may be changed or chemically modified by a conventional method such as esterification and halogenation.

Among these compounds (1), preferred are the embodiment (B) [the compound having a hydroxyl group or a mercapto group] and the embodiment (E) [the compound having (meth)acryloyl group], and further preferred may be the embodiment (E) [the compound having (meth)acryloyl group, particularly the compound (1E), especially the compound having methacryloyl group].

The compound (1) has an excellent compatibility with (or dissolubility in) various compounds [such as an organic solvent, a reaction component (such as a copolymerizable component), and a resin] and has a high refractive index. The compound (1) may, for example, have a refractive index at a temperature of 25°C and a wavelength of 589 nm of about not less than 1.6 (such as about 1.63 to 1.75), preferably about 1.64 to 1.74 (such as about 1.66 to 1.73), and further preferably about 1.65 to 1.725 (such as about 1.67 to 1.725).

In this description and claims, the refractive index can be measured in accordance with the method described in Examples below.

The compound (1A) (the dithiol compound) of the embodiment (A) may, for example, have a refractive index at a temperature of 25°C and a wavelength of 589 nm of about not less than 1.65 (such as about 1.69 to 1.75), preferably about 1.70 to 1.74 (such as about 1.71 to 1.735), and further preferably about 1.72 to 1.73.

The compound [the compound having a hydroxyl group or a mercapto group, particularly the compound (1B)] of the embodiment (B) may, for example, have a refractive index at a temperature of 25°C and a wavelength of 589 nm of about not less than 1.64 (such as about 1.65 to 1.7) and preferably about 1.66 to 1.69 (such as about 1.67 to 1.68).

The compound of the embodiment (C) [the compound having a saturated hydrocarbon group which may have a halogen atom, particularly the compound (1C)] may, for example, have a refractive index at a temperature of 25°C and a wavelength of 589 nm of about not less than 1.66 (such as about 1.67 to 1.71) and preferably about 1.68 to 1.7 (such as about 1.685 to 1.695).

The compound of the embodiment (D) [the compound having an unsaturated hydrocarbon group, particularly the compound (1D)] may, for example, have a refractive index at a temperature of 25°C and a wavelength of 589 nm of about not less than 1.63 (such as about 1.64 to 1.695) and preferably about 1.65 to 1.68 (such as about 1.66 to 1.67); the compound having the group (2D) [particularly the compound (1D)] in which A² is a direct bond may have such a refractive index of about 1.67 to 1.71 and preferably about 1.68 to 1.7.

The compound of the embodiment (E) [the compound having (meth)acryloyl group, particularly the compound (1E)] may, for example, have a refractive index at a temperature of 25°C and a wavelength of 589 nm of about not less than 1.61 (such as about 1.62 to 1.67) preferably about 1.63 to 1.66 (such as about 1.64 to 1.65) when the compound has methacryloyl group, and, for example, about not less than 1.63 (such as about 1.64 to 1.69) and preferably about 1.65 to 1.68 (such as about 1.66 to 1.67) when the compound has acryloyl group.

The compound of the embodiment (F) [the compound having an epoxy ring or a thiirane ring, particularly the compound (1F)] may, for example, have a refractive index at a temperature of 25°C and a wavelength of 589 nm of about not less than 1.63 (such as about 1.64 to 1.69) and preferably about 1.65 to 1.68 (such as about 1.66 to 1.67).

The compound of the embodiment (G) [the compound having a carboxyl group or a group derived from a carboxyl group, particularly the compound (1G)] may, for example, have refractive index at a temperature of 25°C and a wavelength of 589 nm of about not less than 1.63 (such as about 1.64 to 1.69) and preferably about 1.65 to 1.68 (such as about 1.66 to 1.67).

The compound (1) mostly has a relatively low melting point and can easily be molten without excessive heating in the reaction with another reaction component (or reagent), and thus the reaction may be easy to progress. The melting point of the compound (1) may, for example, be about not higher than 200°C (such as about not higher than 100°C, preferably about not higher than 80°C, and specifically about -30 to 70°C), preferably about not higher than 60°C (such as about -10 to 50°C), and further preferably about not higher than 40°C (such as about 0 to 30°C) .

In this description and claims, the melting point can be measured in accordance with the method described in Examples below.

The compound (1A) (the dithiol compound) of the embodiment (A) may, for example, have a melting point of about 10 to 60°C (such as about 20 to 50°C) and preferably about 25 to 45°C (such as about 30 to 40°C) .

The compound of the embodiment (B) [the compound having a hydroxyl group or a mercapto group, particularly the compound (1B)] may, for example, have a melting point of about 20 to 70°C (such as about 30 to 60°C) and preferably about 35 to 55°C (such as about 40 to 50°C) .

The compound of the embodiment (C) [the compound having a saturated hydrocarbon group which may have a halogen atom, particularly the compound (1C)] may, for example, have a melting point of about 25 to 75°C (such as about 30 to 70°C) and preferably about 40 to 60°C (such as about 45 to 55°C) .

The compound of the embodiment (D) [the compound having an unsaturated hydrocarbon group, particularly the compound (1D)] may, for example, have a melting point of a temperature not higher than a room temperature (such as not higher than 25°C), that is, the compound may be liquid under a room temperature.

The compound of the embodiment (E) [the compound having (meth)acryloyl group, particularly the compound (1E)] may, for example, have a melting point of about 50 to 90°C (such as about 55 to 85°C) and preferably about 60 to 80°C (such as about 65 to 75°C) when the compound has methacryloyl group, and, for example, about 40 to 90°C (such as about 50 to 80°C) and preferably about 55 to 75°C (such as about 60 to 70°C) when the compound has acryloyl group.

The compound of the embodiment (F) [the compound having an epoxy ring or a thiirane ring, particularly the compound (1F)] may, for example, have a melting point of a temperature not higher than a room temperature (such as not higher than 25°C), that is, the compound may be liquid under a room temperature.

The compound of the embodiment (G) [the compound having a carboxyl group or a group derived from a carboxyl group, particularly the compound (1G)] may, for example, have a melting point of about 100 to 250°C (such as about 150 to 200°C) and preferably about 160 to 190°C (such as about 170 to 185°C).

The compound (1) may, for example, have a molecular weight of about not more than 2000 (such as about 192 to 1000), preferably about not more than 500 (such as about 200 to 400), and further preferably about not more than 350.

### [Mixture (blend or dissolved mixture) containing naphthalene compound represented by the formula (1) and organic solvent]

The compound (1) has an excellent dissolubility in an organic solvent compared with corresponding other positional isomers (such as 1,5-, 2,6-, or 2,7-isomer), and thus can easily or efficiently be dissolved even at a high concentration and can easily form a homogeneous (or uniform) mixture (blend or dissolved mixture). The mixture (blend) may be a non-liquid composition such as a solid, and is preferably a solution or a liquid composition.

The organic solvent is not particularly limited to a specific one. Representative examples of the organic solvent may include the following:
a hydrocarbon (such as an aliphatic hydrocarbon such as hexane, heptane, octane, decane, and dodecane, an alicyclic hydrocarbon such as cyclohexane, an aromatic hydrocarbon such as benzene, toluene, xylene, and ethylbenzene);
a halogenated hydrocarbon corresponding to a halide of the hydrocarbon (such as a chlorinated hydrocarbon such as dichloromethane, chloroform, 1,2-dichloroethane, chlorobenzene, and dichlorobenzene);
an alcohol (such as a C₁₋₄alkanemonool such as methanol, ethanol, 1-propernol, and 2-propernol; and
a C₂₋₄alkanediol such as ethylene glycol);
an ether [such as a chain ether such as dimethyl ether, and a cyclic ether such as tetrahydrofuran (THF), 1,4-dioxane, and the epihalohydrin (such as epichlorohydrin) exemplified in the embodiment (F)];
a glycol ether [such as a cellosolve (such as a C₁₋₄alkyl cellosolve such as methyl cellosolve and ethyl cellosolve), a carbitol (such as a C₁₋₄alkyl carbitol such as methyl carbitol and ethyl carbitol), a (poly)C₂₋₄alkylene glycol monoC₁₋₄alkyl ether such as triethylene glycol monomethyl ether, propylene glycol monomethyl ether, and dipropylene glycol monomethyl ether; and a (poly)C₂₋₄alkylene glycol diC₁₋₄alkyl ether such as ethylene glycol dimethyl ether, propylene glycol dimethyl ether, diethylene glycol dimethyl ether, and dipropylene glycol dimethyl ether];
a glycol ether acetate [such as a cellosolve acetate (such as a C₁₋₄alkyl cellosolve acetate such as methyl cellosolve acetate), a carbitol acetate (such as a C₁₋₄alkyl carbitol acetate such as methyl carbitol acetate), and a (poly)C₂₋₄alkylene glycol monoC₁₋₄alkyl ether acetate such as propylene glycol monomethyl ether acetate (PGMEA) and dipropylene glycol monobutyl ether acetate];
a ketone (such as a chain ketone such as acetone and methyl ethyl ketone, and a cyclic ketone such as cyclohexanone);
an ester (such as an acetate ester such as methyl acetate, ethyl acetate, and butyl acetate, a lactate ester such as methyl lactate, and a lactone such as γ-butyrolactone and γ-valerolactone);
a carbonate (such as a chain carbonate such as dimethyl carbonate, and a cyclic carbonate such as ethylene carbonate and propylene carbonate);
a carboxylic acid (such as acetic acid and propionic acid);
a nitrile (such as acetonitrile, propionitrile, and benzonitrile);
an amide (such as a chain amide such as N,N-dimethylformamide (DMF) and N,N-dimethylacetamide (DMAc), and a cyclic amide such as N-methyl-2-pyrrolidone);
a urea (such as a chain urea such as tetramethylurea and tetraethylurea, and a cyclic urea such as 1,3-dimethyl-2-imidazolidinone (DMI) and dimethylpropyleneurea (DMPU)); and
a sulfoxide (such as dimethyl sulfoxide).

Such an organic solvent may be used alone or as a mixed solvent in combination of two or more.

Preferred examples of the organic solvent may include a hydrocarbon (such as an aromatic hydrocarbon such as toluene), a halogenated hydrocarbon (such as a chlorinated hydrocarbon such as methylene chloride and chloroform), an alcohol such as methanol, an ether (such as a cyclic ether such as THF, and an epihalohydrin such as epichlorohydrin), a glycol ether acetate (such as a (poly)C₂₋₄alkylene glycol monoC₁₋₄alkyl ether acetate such as PGMEA), an ester (such as an acetate ester such as ethyl acetate), an amide (such as a chain amide such as DMF), and a urea (such as a cyclic urea such as DMI). The compound (1) [particular the compound (1) that is not in the form of a salt] seems to relatively hard to dissolve in water.

The compound (1) is dissolvable at a high concentration in an organic solvent even under a room temperature (such as about 25°C). Thus, the compound (1) can form a mixture (blend) with the above-mentioned organic solvent [in particular, for example, at least one organic solvent selected from the group consisting of the above-mentioned preferred organic solvents; further preferably an alcohol (such as methanol), a hydrocarbon, an ether, a glycol ether acetate, an ester, and an amide; particularly a hydrocarbon (such as an aromatic hydrocarbon such as toluene), an ether (such as a cyclic ether such as THF), a glycol ether acetate (such as a (poly)C₂₋₄alkylene glycol monoC₁₋₄alkyl ether acetate such as PGMEA), an ester (such as an acetate ester such as ethyl acetate), and an amide (such as a chain amide such as DMF); and especially toluene, THF, PGMEA, ethyl acetate, and DMF]. The compound (1) in the mixture (blend) may, for example, have a concentration of not less than 15% by mass (such as not less than 20% by mass), preferably about not less than 30% by mass (such as about 40 to 99% by mass), further preferably about not less than 50% by mass (such as about 60 to 99% by mass), particularly about not less than 70% by mass (such as about 80 to 97% by mass), and especially about not less than 90% by mass (such as about 90 to 95% by mass). The concentration [the concentration of the compound (1)] may be a concentration at a temperature of 25°C, and may be a concentration in the whole mixture (blend).

In this description and claims, the concentration (the maximum dissolvable concentration) can be measured in accordance with the method described in the section "Dissolubility or Compatibility" of Examples below.

Preferred examples of the organic solvent for the compound (1A) (the dithiol compound) of the embodiment (A) may include a hydrocarbon, a halogenated hydrocarbon (such as a chlorinated hydrocarbon such as chloroform), an alcohol, an ether, a glycol ether acetate, an ester, an amide, and a urea (such as a cyclic urea such as DMI). Further preferred examples may include a hydrocarbon, an alcohol such as methanol, an ether, a glycol ether acetate, an ester, and an amide; among them, a hydrocarbon, an ether, a glycol ether acetate (such as a (poly)C₂₋₄alkylene glycol monoC₁₋₄alkyl ether acetate such as PGMEA), an ester, and an amide; and particularly a hydrocarbon (such as an aromatic hydrocarbon such as toluene), an ether (such as a cyclic ether such as THF), an ester (such as an acetate ester such as ethyl acetate), and an amide (such as a chain amide such as DMF).

The compound (1A) (the dithiol compound) of the embodiment (A) can be mixed (blended) with an alcohol such as methanol to prepare a mixture (blend) having at a concentration of the compound of, for example, 10 to 30% by mass and preferably 15 to 25% by mass; can be mixed (blended) with a hydrocarbon (such as an aromatic hydrocarbon such as toluene), an ether (such as a cyclic ether such as THF), an amide (such as a chain amide such as DMF), or an ester (such as an acetate ester such as ethyl acetate) to prepare a mixture (blend) having at a concentration of the compound of, for example, not less than 80% by mass (such as 85 to 99% by mass) and preferably not less than 90% by mass (such as 90 to 95% by mass); or can be mixed (blended) with a glycol ether acetate (such as a (poly)C₂₋₄alkylene glycol monoC₁₋₄alkyl ether acetate such as PGMEA) to prepare a mixture (blend) having at a concentration of the compound of, for example, 70 to 95% by mass and preferably 80 to 90% by mass.

The concentration [the concentration of the compound (1A)] may be a concentration at a temperature of 25°C, and may be a concentration in the whole mixture (blend). The same applies to the compounds of the following embodiments (B) to (G) [particularly the compounds (1B) to (1G)].

Preferred examples of the organic solvent for the compound of the embodiment (B) [the compound having a hydroxyl group or a mercapto group, particularly the compound (1B)] may include a hydrocarbon, a halogenated hydrocarbon (such as a chlorinated hydrocarbon such as chloroform), a glycol ether acetate, an ester, and an amide (such as a chain amide such as DMF). Further preferred examples may include a hydrocarbon (such as an aromatic hydrocarbon such as toluene), a glycol ether acetate, an ester; and particularly a glycol ether acetate (such as a (poly)C₂₋₄alkylene glycol monoC₁₋₄alkyl ether acetate such as PGMEA), and an ester (such as an acetate ester such as ethyl acetate).

The compound of the embodiment (B) [the compound having a hydroxyl group or a mercapto group, particularly the compound (1B)] can be mixed (blended) with a hydrocarbon (such as an aromatic hydrocarbon such as toluene) to prepare a mixture (blend) having at a concentration of the compound of, for example, 1 to 2% by mass; or can be mixed (blended) with an ester (such as an acetate ester such as ethyl acetate) or a glycol ether acetate (such as a (poly)C₂₋₄alkylene glycol monoC₁₋₄alkyl ether acetate such as PGMEA) to prepare a mixture (blend) having at a concentration of the compound of, for example, not less than 80% by mass (such as 85 to 99% by mass) and preferably not less than 90% by mass (such as 90 to 95% by mass).

Preferred examples of the organic solvent for the compound of the embodiment (C) [the compound having a saturated hydrocarbon group which may have a halogen atom, particularly the compound (1C)] may include a hydrocarbon, a halogenated hydrocarbon (such as a chlorinated hydrocarbon such as methylene chloride and chloroform), an ether (such as a cyclic ether such as THF), a glycol ether acetate, an ester, and a urea (such as a cyclic urea such as DMI). Further preferred examples may include a hydrocarbon, a glycol ether acetate (such as a (poly)C₂₋₄alkylene glycol monoC₁₋₄alkyl ether acetate such as PGMEA), an ester; and particularly a hydrocarbon (such as an aromatic hydrocarbon such as toluene), and an ester (such as an acetate ester such as ethyl acetate).

The compound of the embodiment (C) [the compound having a saturated hydrocarbon group, particularly the compound (1C)] can be mixed (blended) with a hydrocarbon (such as an aromatic hydrocarbon such as toluene) or an ester (such as an acetate ester such as ethyl acetate) to prepare a mixture (blend) having at a concentration of the compound of, for example, not less than 80% by mass (such as 85 to 99% by mass) and preferably not less than 90% by mass (such as 90 to 95% by mass); can be mixed (blended) with a glycol ether acetate (such as a (poly)C₂₋₄alkylene glycol monoC₁₋₄alkyl ether acetate such as PGMEA) to prepare a mixture (blend) having at a concentration of the compound of, for example, 20 to 60% by mass (such as 25 to 55% by mass) and preferably 30 to 50% by mass (such as 35 to 45% by mass); or can be mixed (blended) with an ether (such as a cyclic ether such as THF) to prepare a mixture (blend) having at a concentration of the compound of, for example, 5 to 15% by mass.

Preferred examples of the organic solvent for the compound of the embodiment (D) [the compound having an unsaturated hydrocarbon group, particularly the compound (1D)] may include a hydrocarbon, a halogenated hydrocarbon (such as a chlorinated hydrocarbon such as methylene chloride and chloroform), a glycol ether acetate, and an ester. Further preferred examples may include a hydrocarbon (such as an aromatic hydrocarbon such as toluene), a glycol ether acetate (such as a (poly)C₂₋₄alkylene glycol monoC₁₋₄alkyl ether acetate such as PGMEA), and an ester (such as an acetate ester such as ethyl acetate).

The compound of the embodiment (D) [the compound having an unsaturated hydrocarbon group, particularly the compound (1D)] can be mixed (blended) with a hydrocarbon (such as an aromatic hydrocarbon such as toluene), an ester (such as an acetate ester such as ethyl acetate), or a glycol ether acetate (such as a (poly)C₂₋₄alkylene glycol monoC₁₋₄alkyl ether acetate such as PGMEA) to prepare a mixture (blend) having at a concentration of the compound of, for example, not less than 80% by mass (such as 85 to 99% by mass) and preferably not less than 90% by mass (such as 90 to 95% by mass).

Preferred examples of the organic solvent for the compound of the embodiment (E) [the compound having (meth)acryloyl group, particularly the compound (1E)] may include a hydrocarbon, a halogenated hydrocarbon (such as a chlorinated hydrocarbon such as methylene chloride and chloroform), an alcohol, an ether, a glycol ether acetate, an ester, an amide, and a urea (such as a cyclic urea such as DMI). Further preferred examples may include a hydrocarbon, an alcohol such as methanol, an ether, a glycol ether acetate, an ester, and an amide; and particularly a hydrocarbon, an ether, a glycol ether acetate, an ester, and an amide. Among them, more preferred organic solvents are a glycol ether acetate (such as a (poly)C₂₋₄alkylene glycol monoC₁₋₄alkyl ether acetate such as PGMEA), an ester (such as an acetate ester such as ethyl acetate), a hydrocarbon (such as an aromatic hydrocarbon such as toluene), an amide (such as a chain amide such as DMF), and an ether (such as a cyclic ether such as THF) in order of preference (the later ones are more preferred).

When the compound of the embodiment (E) [the compound having (meth)acryloyl group, particularly the compound (1E)] has methacryloyl group, the compound can be mixed (blended) with an alcohol such as methanol to prepare a mixture (blend) having at a concentration of the compound of, for example, 1 to 5% by mass; can be mixed (blended) with a hydrocarbon (such as an aromatic hydrocarbon such as toluene) to prepare a mixture (blend) having at a concentration of the compound of, for example, 40 to 60% by mass and preferably 45 to 55% by mass; can be mixed (blended) with an ether (such as a cyclic ether such as THF) to prepare a mixture (blend) having at a concentration of the compound of, for example, 60 to 80% by mass and preferably 65 to 75% by mass; can be mixed (blended) with an amide (such as a chain amide such as DMF) to prepare a mixture (blend) having at a concentration of the compound of, for example, 50 to 70% by mass and preferably 55 to 65% by mass; can be mixed (blended) with an ester (such as an acetate ester such as ethyl acetate) to prepare a mixture (blend) having at a concentration of the compound of, for example, 35 to 55% by mass and preferably 40 to 50% by mass; or can be mixed (blended) with a glycol ether acetate (such as a (poly)C₂₋₄alkylene glycol monoC₁₋₄alkyl ether acetate such as PGMEA) to prepare a mixture (blend) having at a concentration of the compound of, for example, 20 to 40% by mass and preferably 25 to 35% by mass. When the compound has acryloyl group, the compound can be mixed (blended) with a hydrocarbon (such as an aromatic hydrocarbon such as toluene) to prepare a mixture (blend) having at a concentration of the compound of, for example, 50 to 70% by mass and preferably 55 to 65% by mass; can be mixed (blended) with an ester (such as an acetate ester such as ethyl acetate) to prepare a mixture (blend) having at a concentration of the compound of, for example, 45 to 65% by mass and preferably 50 to 60% by mass; or can be mixed (blended) with a glycol ether acetate (such as a (poly)C₂₋₄alkylene glycol monoC₁₋₄alkyl ether acetate such as PGMEA) to prepare a mixture (blend) having at a concentration of the compound of, for example, 35 to 55% by mass and preferably 40 to 50% by mass.

Preferred examples of the organic solvent for the compound of the embodiment (F) [the compound having an epoxy ring or a thiirane ring, particularly the compound (1F)] may include a hydrocarbon, a halogenated hydrocarbon (such as a chlorinated hydrocarbon such as chloroform), an ether (such as an epihalohydrin such as epichlorohydrin), a glycol ether acetate, and an ester; further preferred examples may include a hydrocarbon (such as an aromatic hydrocarbon such as toluene), a glycol ether acetate (such as a (poly)C₂₋₄alkylene glycol monoC₁₋₄alkyl ether acetate such as PGMEA), and an ester (such as an acetate ester such as ethyl acetate).

The compound of the embodiment (F) [the compound having an epoxy ring or a thiirane ring, particularly the compound (1F)] can be mixed (blended) with a hydrocarbon (such as an aromatic hydrocarbon such as toluene), an ester (such as an acetate ester such as ethyl acetate), or a glycol ether acetate (such as a (poly)C₂₋₄alkylene glycol monoC₁₋₄alkyl ether acetate such as PGMEA) to prepare a mixture (blend) having at a concentration of the compound of, for example, not less than 80% by mass (such as 85 to 99% by mass) and preferably not less than 90% by mass (such as 90 to 95% by mass).

Preferred examples of the organic solvent for the compound of the embodiment (G) [the compound having a carboxyl group or a group derived from a carboxyl group, particularly the compound (1G)] may include an ether, an amide, an ester (such as an acetate ester such as ethyl acetate), and a glycol ether acetate (such as a (poly)C₂₋₄alkylene glycol monoC₁₋₄alkyl ether acetate such as PGMEA); further preferred examples may include an ether (such as a cyclic ether such as THF) and an amide; particularly preferred examples may include an amide (such as a chain amide such as DMF).

The compound of the embodiment (G) [the compound having a carboxyl group or a group derived from a carboxyl group, particularly the compound (1G)] can be mixed (blended) with an ether (such as a cyclic ether such as THF) to prepare a mixture (blend) having at a concentration of the compound of, for example, 10 to 50% by mass (such as 15 to 45% by mass) and preferably 20 to 40% by mass (such as 25 to 35% by mass); can be mixed (blended) with an amide (such as a chain amide such as DMF) to prepare a mixture (blend) having at a concentration of the compound of, for example, 40 to 80% by mass (such as 45 to 75% by mass) and preferably 50 to 70% by mass (such as 55 to 65% by mass); or can be mixed (blended) with a glycol ether acetate (such as a (poly)C₂₋₄alkylene glycol monoC₁₋₄alkyl ether acetate such as PGMEA) or an ester (such as an acetate ester such as ethyl acetate) to prepare a mixture (blend) having at a concentration of the compound of, for example, 0.5 to 3% by mass.

The mixture (blend) according to the present invention can be prepared as a homogeneous composition even in a case where the mixture (blend) contains the compound (1) at a high concentration. Thus, the mixture (blend) can, for example, be used as a coating agent or coat material for forming a film or membrane (such as a coating film and a coating) of the compound (1). Such a coating agent or coat material is adjustable at a wide concentration range, and thus is easy to adjust physical properties of the resulting film, such as film thickness.

The mixture (blend) may further contain other components if necessary. For example, the mixture (blend) may contain the after-mentioned conventional additive or conventional resin [such as a binder component or matrix component to the compound (1) as a resin additive]. For example, a mixture containing the compound (1), an organic solvent, and a conventional resin may be prepared, the after-mentioned resin composition [a composition containing the compound (1) and a resin] may be prepared by removing the organic solvent from the mixture.

The mixture (blend) may be used as a reaction mixture (or a reaction solution) containing a reaction component or a reagent [such as a reaction component with the compound (1)] as other components. For example, the mixture (blend) prepared may be a reaction mixture (or a reaction solution) for preparing a resin containing a constituent unit represented by the after-mentioned formula (1P), that is, a reaction mixture (or a reaction solution) containing the compound (1) as a raw material monomer, a polymerizable component (such as the after-mentioned polymerizable component) polymerizable with the compound (1), an organic solvent, and optionally a catalyst. [Incidentally, in a case where the compound (1) or the polymerizable component is liquid and can also function as a solvent, the reaction mixture (the reaction solution) may not necessarily contain an organic solvent. Moreover, in a case where the compound (1) is polymerizable alone, the reaction mixture (the reaction solution) may not necessarily contain a polymerizable component polymerizable with the compound (1).] The compound (1) may be dissolvable or compatible homogeneously or at a high concentration in the reaction mixture (or the reaction solution), and the reaction easily or efficiently progresses.

The mixture (blend) can easily be prepared by mixing the compound (1), the organic solvent, and optionally the above-mentioned other components.

### [Curable composition containing naphthalene compound represented by formula (1)]

The compound (1) has not only a high dissolubility in an organic solvent but also an excellent compatibility with an organic compound [such as an organic compound that is liquid at a room temperature (about 25°C)] . The present invention includes a curable composition at least containing the compound (1). For such a curable composition, the compound (1) is easily or efficiently compatible even at a high concentration with another organic compound (such as a polymerizable compound) to easily obtain a homogeneous (or uniform) curable composition or a cured product thereof. For example, a curable composition containing the compound (1) having a polymerizable group and another polymerizable compound can easily obtain a homogeneous (or uniform) curable composition or a cured product thereof even in a case where the ratio of the compound (1) to another polymerizable compound (copolymerization ratio) is adjusted within a wide range.

The curable composition is not particularly limited to a specific one and may be any composition which can obtain a cured product (a resin or a polymer, preferably a polymer having a crosslinked structure or a three-dimensional network structure) by such as heat and/or light energy; the curable composition contains at least a polymerizable compound.

The polymerizable compound is not particularly limited to a specific one and may be a compound having at least one (preferably two or more) polymerizable group(s) [such as an ethylenically unsaturated bond-containing group such as an alkenyl group (such as vinyl group and allyl group) and (meth)acryloyl group, an epoxy-containing group such as glycidyl group, and a thiirane-containing group such as 2,3-epithiopropyl group].

As the polymerizable compound, the curable composition may contain the compound (1) having a polymerizable group or may contain another polymerizable compound different from the compound (1). Such a curable composition may be used alone or in combination of two or more.

The curable composition may not necessarily contain the compound (1) having a polymerizable group. For example, in a case where the polymerizable compound contains another polymerizable compound alone, the compound (1) [the compound (1) having no polymerizable group] in the curable composition may function as a resin additive. For example, the curable composition may contain the compound (1) [such as the compound (1A), (1B), or (1G), particularly the compound (1A)] as a resin additive (such as an epoxy curing agent) and an epoxy resin. Also, in a case where a thiophenol resin as a cured product is prepared from the compound (1A) and an aldehyde (such as a formaldehyde such as formalin and paraformaldehyde), the compound (1A) may have no polymerizable group.

### (Compound (1) having polymerizable group)

Representative examples of the compound (1) having a polymerizable group may include a naphthalene compound represented by the formula (1) in which at least one (preferably both) of R¹ and R² represents the above-mentioned group (2D), group (2E), or group (2F); preferably a naphthalene compound in which at least one (preferably both) of R¹ and R² represents an alkenyl group, (meth)acryloyl group, a (meth)acryloyloxyalkyl group, a glycidyl group, a β-methylglycidyl group, a glycidyloxyalkyl group, or a β-methylglycidyloxyalkyl group; and further preferably a naphthalene compound in which at least one (preferably both) of R¹ and R² represents an alkenyl group (such as vinyl group and allyl group) or (meth)acryloyl group.

Preferred examples of the compound (1) having a polymerizable group may include the compound of the embodiment (D) [the compound having an unsaturated hydrocarbon group, particularly the compound (1D)], the compound of the embodiment (E) [the compound having (meth)acryloyl group, particularly the compound (1E)], and the compound of the embodiment (F) [the compound having an epoxy ring or a thiirane ring, particularly the compound (1F)].

### (Another polymerizable compound)

Another polymerizable compound different from the compound (1) may, for example, be a compound having at least one (preferably two or more) polymerizable group(s) described above, and may, for example, be a positional isomer (such as 1,4-, 1,5-, 2,6-, or 2,7-isomer) corresponding to any of the embodiments (D) to (F) [particularly the compounds (1D) to (1F)] that are 1,6-isomers. Examples of another polymerizable compound may include an ethylenically unsaturated compound and an epoxy compound (or an epoxy resin). Such a polymerizable compound may be used alone or in combination of two or more. Among them, an ethylenically unsaturated compound and an epoxy compound (or an epoxy resin) are preferred.

### (Ethylenically unsaturated compound)

The ethylenically unsaturated compound has, as at least one polymerizable group, an ethylenically unsaturated bond-containing group such as an alkenyl group (such as vinyl group and allyl group), an alkenyloxy group (such as vinyl ether group and allyl ether group), and (meth)acryloyl group, and preferably has a group such as an alkenyl group (such as vinyl group) and (meth)acryloyl group. The ethylenically unsaturated compound may be monofunctional or polyfunctional.

Representative examples of the monofunctional ethylenically unsaturated compound may include the following:
an α-olefin (such as an α-C₂₋₁₂olefin such as ethylene, propylene, and 1-butene);
a vinyl ester (such as vinyl acetate and vinyl propionate);
an aromatic vinyl (such as styrene, α-methylstyrene,
and vinyltoluene);
a vinylpyrrolidone (such as N-vinylpyrrolidone);
an unsaturated dicarboxylic acid or a derivative thereof (such as maleic anhydride and itaconic acid); and
(meth)acrylic acid or a derivative thereof {such as (meth)acrylic acid; a (meth)acrylate [such as an aliphatic (meth)acrylate such as methyl (meth)acrylate, butyl (meth)acrylate, and 2-ethylhexyl (meth)acrylate; an alicyclic (meth)acrylate such as cyclohexyl (meth)acrylate, dicyclopentanyl (meth)acrylate, and isobornyl (meth)acrylate; an aromatic (meth)acrylate such as phenyl (meth)acrylate and benzyl (meth)acrylate; a hydroxyalkyl (meth)acrylate (such as hydroxyethyl (meth)acrylate and hydroxypropyl (meth)acrylate); a glycidyl (meth)acrylate; a mono- or dialkylaminoalkyl (meth)acrylate (such as dimethylaminoethyl (meth)acrylate); and a thio(meth)acrylate (such as phenyl thio(meth)acrylate)]; a (meth)acrylamide; a substituted (meth)acrylamide (such as a mono- or dialkyl (meth)acrylamide such as N-isopropyl (meth)acrylamide, and N-methylol (meth)acrylamide); and a (meth)acrylonitrile}.

Representative examples of the polyfunctional ethylenically unsaturated compound may include a diene (such as butadiene and isoprene); an aromatic polyvinyl (such as divinylbenzene); a polyfunctional (meth)acrylate [such as a poly(meth)acrylate of a polyol or an alkylene oxide adduct thereof, specifically a (poly)alkylene glycol di(meth)acrylate such as ethylene glycol di(meth)acrylate and diethylene glycol di(meth)acrylate; a di(meth)acrylate of a bisphenol or an alkylene oxide adduct thereof, such as a di(meth)acrylate of an ethylene oxide adduct of bisphenol A; glycerin di(meth)acrylate, trimethylolpropane tri(meth)acrylate, pentaerythritol tri(meth)acrylate, pentaerythritol tetra(meth)acrylate, dipentaerythritol penta(meth)acrylate, dipentaerythritol hexa(meth)acrylate, and a poly(meth)acrylate of an ethylene oxide adduct of sorbitol]; a urethane (meth)acrylate; an epoxy(meth)acrylate; and a polyester (meth)acrylate.

### (Epoxy compound)

The epoxy compound (or the epoxy resin) has an epoxy-containing group, such as epoxy group and glycidyl group, as at least one polymerizable group. The epoxy compound (or the epoxy resin) may be monofunctional or polyfunctional.

Representative examples of the monofunctional epoxy compound (or reactive diluent) may include an alkyl glycidyl ether such as methyl glycidyl ether and butyl glycidyl ether, an aryl glycidyl ether such as phenyl glycidyl ether, and styrene oxide.

Representative examples of the polyfunctional epoxy compound (or epoxy resin) may include a conventional epoxy resin (or epoxy compound), including a glycidyl ether-type epoxy resin (such as a bisphenol-type epoxy resin such as bisphenol A-type, F-type, and AD-type; a novolac-type epoxy resin such as phenol novolac-type and cresol novolac-type; and tetrakis(glycidyloxyphenyl)ethane); a bromine-containing epoxy resin (such as a tetrabromobisphenol A-type epoxy resin); a glycidyl ester-type epoxy resin (such as a phthalic epoxy resin such as diglycidyl phthalate, diglycidyl tetrahydrophthalate, diglycidyl hexahydrophthalate, and dimethylglycidyl phthalate); a glycidylamine-type epoxy resin (such as diglycidylaniline, diglycidyltoluidine, triglycidylaminophenol, and tetraglycidyldiaminodiphenylmethane); an epoxy resin having a group epoxidized by oxidation of an unsaturated double bond (such as a cyclic aliphatic epoxy resin having cyclohexene oxide group or other groups); and a heterocyclic epoxy resin (such as triglycidyl isocyanurate; and a hydantoin-type epoxy resin such as diglycidyl hydantoin).

Such another polymerizable compound may be used alone or in combination of two or more. Among these another polymerizable compounds, preferred examples of the ethylenically unsaturated compound may include a monofunctional ethylenically unsaturated compound. The ethylenically unsaturated compound may further preferably be an aromatic vinyl, (meth)acrylic acid or a derivative thereof [such as (meth)acrylic acid, and a (meth)acrylate], particularly a styrene (such as styrene, α-methylstyrene, and vinyltoluene), a (meth)acrylate such as an aliphatic (meth)acrylate [such as C₁₋₆alkyl (meth)acrylate such as methyl (meth)acrylate], and especially a styrene such as styrene. Preferred examples of the epoxy compound (the epoxy resin) may include a polyfunctional epoxy resin. The epoxy compound (the epoxy resin) may further preferably be a glycidyl ether-type epoxy resin, and particularly a bisphenol-type epoxy resin such as a bisphenol A-type epoxy resin.

According to the present invention, the compound (1) is compatible at a high concentration with such another polymerizable compound(s) even under a room temperature (such as about 25°C), and it is thus preferred that the curable composition contain the compound (1) [particularly the compound(s) (1D)-(1F)] and another polymerizable compound (particularly the ethylenically unsaturated compound and/or the epoxy compound) in combination. For example, an ethylenically unsaturated bond-series curable composition containing at least the ethylenically unsaturated compound may be prepared. Specifically the ethylenically unsaturated bond-series curable composition (such as a radical-polymerizable curable composition) may be prepared which contains the compound(s) (1D)-(1E) in combination with an ethylenically unsaturated compound different from the compound(s) (1D)-(1E); the ethylenically unsaturated bond-series curable composition may be prepared which contains the ethylenically unsaturated compound [the compound(s) (1D)-(1E), and/or another ethylenically unsaturated compound] in combination with the compound (1) [such as the compound (1B) and the compound (1C), particularly the compound (1C)] as a resin additive (such as a refractive index increasing agent).

The curable composition may be prepared as an epoxy-series curable composition containing at least the epoxy compound (the epoxy resin). For example, the epoxy-series curable composition may be prepared which at least contains the compound (1F) as the epoxy compound (the epoxy resin); the epoxy-series curable composition may be prepared which contains the compound (1F) as the epoxy compound (the epoxy resin) in combination with an epoxy compound (or an epoxy resin) different from the compound (1F); and the epoxy-series curable composition may preferably be prepared which contains the epoxy compound (the epoxy resin) [the compound (1F) and/or another epoxy compound] in combination with the compound (1) [such as the compound (1A), the compound (1B), the compound (1C), and the compound (1G), preferably the compound (1A), (1B), or (1G) which can function as epoxy curing agent, and particularly the compound (1A)] as a resin additive (such as an epoxy curing agent and/or a refractive index increasing agent).

In such ethylenically unsaturated bond-series curable composition and epoxy-series curable composition, each component is homogeneously compatible at a high concentration. The resulting cured product (or a copolymer of the compound (1) and another polymerizable compound) is thus effectively prevented from separation or precipitation and has a tendency to be easily formed as a uniform cured product, even in a case where the copolymerization ratio or blending ratio is adjusted within a wide range (in particular, even in a case where the compound (1) is contained in large quantities). Thus, such a curable composition is hard to generate phase separation and can easily or efficiently prepare a cured product having an excellent transparency. In particular, the curable composition can effectively be used even in an application having an intended high transparency, such as an optical member.

In the curable composition containing the compound (1) and another polymerizable compound, the proportion of the compound (1) relative to (or the concentration of the compound (1) in) the total amount of the compound (1) and another polymerizable compound (particularly the ethylenically unsaturated compound) may, for example, be not less than 10% by mass (such as not less than 20% by mass) or may be preferably about not less than 30% by mass (such as about 40 to 99% by mass), further preferably about not less than 50% by mass (such as about 60 to 99% by mass), especially about not less than 70% by mass (such as about 80 to 97% by mass), and particularly not less than about 90% by mass (such as about 90 to 95% by mass), or may, for example, be about 20 to 80% by mass (such as about 30 to 70% by mass) and preferably about 40 to 60% by mass (about 45 to 55% by mass). The proportion (or the concentration of the compound (1)) may be a proportion (or a concentration) at a temperature of 25°C.

In the curable composition containing the compound (1) as the polymerizable compound, the proportion of the compound (1) [such as the compound(s) of the embodiment(s) (D)-(F), preferably the compound (1) having a polymerizable group, such as the compound (1D), (1E), or (1F)] as the polymerizable compound relative to the whole polymerizable compound [or relative to the total amount of the compound (1) as the polymerizable compound and another polymerizable compound] may, for example, be about not less than 10% by mass (such as about 30 to 100% by mass), preferably about not less than 50% by mass (such as about 70 to 90% by mass), further preferably about not less than 90% by mass, and substantially about 100% by mass.

In the ethylenically unsaturated bond-series curable composition containing the compound of the embodiment (D) and/or (E) as the polymerizable compound, the total amount of the compounds of the embodiments (D) and (E) [in particular, the total amount of the compounds (1D) and (1E)] relative to the whole polymerizable compound [in particular, relative to the whole ethylenically unsaturated compound] may, for example, be about not less than 10% by mass (such as about 30 to 100% by mass), preferably about not less than 50% by mass (such as about 70 to 90% by mass), further preferably about not less than 90% by mass, and substantially about 100% by mass.

In the epoxy-series curable composition containing the compound of the embodiment (F) as the polymerizable compound, the proportion of the compound of the embodiment (F) [particularly the compound (1F)] relative to the whole polymerizable compound [in particular, relative to the whole epoxy compound] may, for example, be about not less than 10% by mass (such as about 30 to 100% by mass), preferably about not less than 50% by mass (such as about 70 to 90% by mass), further preferably about not less than 90% by mass, and substantially about 100% by mass.

The concentration of the compound (1) relative to the whole curable composition may, for example, be not less than 10% by mass (such as not less than 20% by mass, and not less than 25% by mass), may be preferably about not less than 30% by mass (such as about 40 to 99% by mass), further preferably about not less than 50% by mass (such as about 60 to 99% by mass), especially about not less than 70% by mass (such as about 80 to 97% by mass), and particularly about not less than 90% by mass (such as about 90 to 95% by mass), or may be substantially about 100% by mass. The concentration may be a concentration at a temperature of 25°C.

In a case where the proportion of the polymerizable compound represented by the formula (1) is excessively low, the refractive index may fail to be increased or improved effectively. According to the present invention, the compound (1) that is a polymerizable compound has an excellent dissolubility or compatibility, and thus the compound (1) is hard to separate or precipitate in the curable composition [or the reaction system (the reaction solution or the reaction mixture)] and allows easy or efficient progress of the reaction. Thus, the compound (1) [or the constituent unit represented by the formula (1P)] is easily or efficiently introduced at a high proportion in the cured product, and the refractive index can effectively be increased or improved.

In the same manner as the section of the mixture (blend), the proportion or concentration (the maximum dissolvable concentration) can be measured in accordance with the method described in the section "Dissolubility or Compatibility" of Examples below.

The compound of the embodiment (B) [the compound having a hydroxyl group or a mercapto group, particularly the compound (1B)] may, for example, be compatible at a concentration of not less than 80% by mass (such as 85 to 99% by mass) and preferably not less than 90% by mass (such as 90 to 95% by mass), relative to the total amount of the compound of the embodiment (B), and (meth)acrylic acid or the derivative thereof [such as preferably a (meth)acrylate; further preferably an aliphatic (meth)acrylate; and particularly a C₁₋₆alkyl (meth)acrylate such as methyl (meth)acrylate]; or may, for example, be compatible at a concentration of about 1 to 3% by mass, relative to the total amount of the compound of the embodiment (B) and the aromatic vinyl [such as particularly a styrene (such as styrene, α-methylstyrene, and vinyltoluene)].

The concentration [the concentration of the compound of the embodiment (B), particularly the concentration of the compound (1B)] may be a concentration at a temperature of 25°C. The same applies to the compounds [particularly the compounds (1C) to (1G)] of the following embodiments (C) to (G) .

The compound of the embodiment (C) [the compound having a saturated hydrocarbon group which may have a halogen atom, particularly the compound (1C)] may, for example, be compatible at a concentration of about 40 to 80% by mass (such as about 45 to 75% by mass) and preferably about 50 to 70% by mass (such as about 55 to 65% by mass), relative to the total amount of the compound of the embodiment (C), and (meth)acrylic acid or the derivative thereof [such as preferably a (meth)acrylate; further preferably an aliphatic (meth)acrylate; and particularly a C₁₋₆alkyl (meth)acrylate such as methyl (meth)acrylate]; or may, for example, be compatible at a concentration of about 50 to 90% by mass (such as about 55 to 85% by mass) and preferably about 60 to 80% by mass (such as about 65 to 75% by mass), relative to the total amount of the compound of the embodiment (C) and the aromatic vinyl [such as particularly a styrene (such as styrene, α-methylstyrene, and vinyltoluene)].

The compound of the embodiment (D) [the compound having an unsaturated hydrocarbon group, particularly the compound (1D)] may, for example, be compatible at a concentration of not less than 80% by mass (such as 85 to 99% by mass) and preferably not less than 90% by mass (such as 90 to 95% by mass), relative to the total amount of the compound of the embodiment (D), and (meth)acrylic acid or the derivative thereof [such as preferably a (meth)acrylate; further preferably an aliphatic (meth)acrylate; and particularly a C₁₋₆alkyl (meth)acrylate such as methyl (meth)acrylate]; or may, for example, be compatible at a concentration of not less than 80% by mass (such as 85 to 99% by mass) and preferably not less than 90% by mass (such as 90 to 95% by mass), relative to the total amount of the compound of the embodiment (D) and the aromatic vinyl [such as particularly a styrene (such as styrene, α-methylstyrene, and vinyltoluene)].

When the compound of the embodiment (E) [the compound having (meth)acryloyl group, particularly the compound (1E)] has methacryloyl group, the compound may, for example, be compatible at a concentration of about 25 to 65% by mass (such as about 30 to 60% by mass) and preferably about 35 to 55% by mass (such as about 40 to 50% by mass), relative to the total amount of the compound of the embodiment (E), and (meth)acrylic acid or the derivative thereof [such as preferably a (meth)acrylate; further preferably an aliphatic (meth)acrylate; and particularly a C₁₋₆alkyl (meth)acrylate such as methyl (meth)acrylate]; or may, for example, be compatible at a concentration of about 30 to 70% by mass (such as about 35 to 65% by mass) and preferably about 40 to 60% by mass (such as about 45 to 55% by mass), relative to the total amount of the compound of the embodiment (E) and the aromatic vinyl [such as particularly a styrene (such as styrene, α-methylstyrene, and vinyltoluene)]. When the compound of the embodiment (E) [the compound having (meth)acryloyl group, particularly the compound (1E)] has acryloyl group, the compound may, for example, be compatible at a concentration of about 35 to 75% by mass (such as about 40 to 70% by mass) and preferably about 45 to 65% by mass (such as about 50 to 60% by mass), relative to the total amount of the compound of the embodiment (E), and (meth)acrylic acid or the derivative thereof [such as preferably a (meth)acrylate; further preferably an aliphatic (meth)acrylate; and particularly a C₁₋₆alkyl (meth)acrylate such as methyl (meth)acrylate]; or may, for example, be compatible at a concentration of about 40 to 80% by mass (such as about 45 to 75% by mass) and preferably about 50 to 70% by mass (such as about 55 to 65% by mass), relative to the total amount of the compound of the embodiment (E) and the aromatic vinyl [such as particularly a styrene (such as styrene, α-methylstyrene, and vinyltoluene)].

The compound of the embodiment (F) [the compound having an epoxy ring or a thiirane ring, particularly the compound (1F)] may, for example, be compatible at a concentration of not less than 80% by mass (such as 85 to 99% by mass) and preferably not less than 90% by mass (such as 90 to 95% by mass), relative to the total amount of the compound of the embodiment (F), and (meth)acrylic acid or the derivative thereof [such as preferably a (meth)acrylate; further preferably an aliphatic (meth)acrylate; and particularly a C₁₋₆alkyl (meth)acrylate such as methyl (meth)acrylate]; or may, for example, be compatible at a concentration of not less than 80% by mass (such as 85 to 99% by mass) and preferably not less than 90% by mass (such as 90 to 95% by mass), relative to the total amount of the compound of the embodiment (F) and the aromatic vinyl [such as particularly a styrene (such as styrene, α-methylstyrene, and vinyltoluene)].

The compound of the embodiment (G) [the compound having a carboxyl group or a group derived from a carboxyl group, particularly the compound (1G)] may, for example, be compatible at a concentration of 0.05 to 0.5% by mass, relative to the total amount of the compound of the embodiment (G), and (meth)acrylic acid or the derivative thereof [such as preferably a (meth)acrylate; further preferably an aliphatic (meth)acrylate; and particularly a C₁₋₆alkyl (meth)acrylate such as methyl (meth)acrylate].

The curable composition according to the present invention may further contain another component, if necessary, as far as the effects of the present invention are not damaged; examples of such a component includes a polymerization initiator, a curing agent or a curing accelerator, a solvent, and a conventional additive.

### (Polymerization initiator)

In a case where the curable composition contains a compound having an ethylenically unsaturated bond, for example, the compound of the embodiment (D) [the compound having an unsaturated hydrocarbon group, particularly the compound (1D)], the compound of the embodiment (E) [the compound having (meth)acryloyl group, particularly the compound (1E)], the ethylenically unsaturated compound (in particular, an aromatic vinyl, and (meth)acrylic acid or a derivative thereof), and others, the curable composition (the ethylenically unsaturated bond-series curable composition) may contain a conventional polymerization initiator [a thermal polymerization initiator, or a photopolymerization initiator or a photoinitiator (a thermal radical generator or a photo-radical generator)] .

Examples of the thermal polymerization initiator may include an organic peroxide (such as a dialkyl peroxide such as di-tert-butyl peroxide, a diacyl peroxide such as benzoyl peroxide, and a peroxy acid or a peroxy acid ester), and an azo compound [such as an azonitrile compound such as 2,2'-azobis(isobutyronitrile)] . Such a thermal polymerization initiator may be used alone or in combination of two or more.

Examples of the photopolymerization initiator may include a benzoin (such as benzoin, and a benzoin alkyl ether), an acetophenone (such as acetophenone), an aminoacetophenone, a benzophenone (such as benzophenone), an anthraquinone (such as anthraquinone), a xanthone, a thioxanthone (such as 2,4-diethylthioxanthone), and a ketal (such as acetophenone dimethyl ketal). Such a photopolymerization initiator may be used alone or in combination of two or more.

The ratio of the polymerization initiator (the thermal polymerization initiator or the photopolymerization initiator) may, for example, be about 0.1 to 15 parts by mass and preferably about 0.5 to 10 parts by mass, relative to 100 parts by mass of the total amount of the compound having an ethylenically unsaturated bond.

The photopolymerization initiator may be used in combination with a conventional photosensitizer. Examples of the photosensitizer may include a tertiary amine (such as a trialkylamine, a trialkanolamine, an alkyl ester of a dialkylaminobenzoate, a bis(dialkylamino)benzophenone, and a dialkylaminobenzophenone). Such a photosensitizer may be used alone or in combination of two or more. The ratio of the photosensitizer may, for example, be about 1 to 200 parts by mass and preferably about 5 to 150 parts by mass, relative to 100 parts by mass of the photopolymerization initiator.

### (Curing agent or curing accelerator)

In a case where the curable composition contains a compound having an epoxy-containing group, for example, the compound of the embodiment (F) [the compound having an epoxy ring or a thiirane ring, particularly the compound (1F)], the above-mentioned epoxy compound (or the epoxy resin), and others, the curable composition (the epoxy-series curable composition) may contain the compound (1) [such as the compound of the embodiment (A), (B), or (G), preferably the compound (1A), (1B), or (1G), and particularly the compound (1A)] as a curing agent capable of curing the epoxy resin, or may contain a conventional curing agent or curing accelerator for an epoxy resin [including a cationic polymerization initiator (such as a photocationic polymerization initiator)]. Such a curing agent or curing accelerator may be used alone or in combination of two or more.

The present invention also includes a curing agent (an epoxy curing agent) represented by the formula (1). Such a curing agent is not particularly limited to a specific one, and may be any curing agent that is represented by the formula (1) and is capable of curing an epoxy resin. Example of the curing agent may include a compound in which R¹ and R² in the formula (1) independently represent a group defined by the formula (2B) or the formula (2G). Representative examples of the compound (1) that can function as a curing agent may include the compound of the embodiment (A), (B), or (G), preferably the compound (1A), (1B), or (1G), and particularly preferably the compound (1A). The compound (1) as the curing agent may also function as a refractive index increasing agent (or may increase or improve the refractive index of the curable composition or a cured product thereof).

The curing agent or curing accelerator is not particularly limited to a specific one and may be any compound that is curable by allowing to react with an oxirane ring (or an epoxy-containing group). Examples of the conventional curing agent or curing accelerator may include an amine-series compound [such as an aliphatic amine such as an alkylamine, an alkanolamine, a (poly)alkylenepolyamine or an alkylsubstituted form thereof, and a (poly)etherpolyamine; an alicyclic amine such as mensendiamine, isophoronediamine, dialkylcycloalkylamine, a spirocyclic or bridged cyclic amine, and a hydrogenated product of an aromatic amine; an aromatic amine such as a (poly)aminomethyl-benzene, a (poly)dialkylaminomethyl-phenol, a poly(aminophenyl)alkane, a poly(aminophenyl)sulfone, a phthalocyanineamine, and a benzylalkylamine; a heterocyclic amine such as a pyridine, a morpholine, an imidazole, triethylenediamine (DABCO), and a cyclic amidine (such as DBU and DBN); a modified amine such as an amine adduct, a polyamine obtained by Michael addition, a polyamine obtained by Mannich addition, a polyamine obtained by thiourea addition, a polyamine obtained by ketone capping (ketimine), dicyandiamide, guanidine, an organic acid hydrazide, diaminomaleonitrile, and an amineimide; or a salt of each amine mentioned above (a salt with an organic acid or an inorganic acid)]; an amide-series compound such as a polyaminoamide that is a condensed product of a dimer acid and a polyamine; an ester-series compound such as an active carbonyl compound; a phenolic compound such as a phenol resin (an aralkyl-type, a novolac-type, and a modified form of each type mentioned above (a modified phenol resin)); an alcoholic compound such as a polyol; an ether-series compound; a thiol-series compound such as a polymercaptan; a thioether-series compound such as a polysulfide; a urea-series compound such as butylated urea and butylated melamine; a thiourea-series compound such as butylated thiourea; a Lewis acid-series compound such as boron trifluoride or a complex thereof; a phosphorus-containing compound such as an organic phosphine compound; an anhydride-series compound such as an aliphatic polycarboxylic anhydride, an alicyclic polycarboxylic anhydride, an aromatic polycarboxylic anhydride, and a halogenated acid anhydride; an onium salt-series compound (or a cationic polymerization initiator) such as an aryldiazonium salt, a diaryliodonium salt, an arylsulfonium salt, an arylmethylsulfonium salt, a diarylsulfonium salt, and a triarylsulfonium salt; and an active silicon compound-aluminum complex such as a triphenylsilanol-aluminum complex. Such a curing agent or curing accelerator may be used alone or in combination of two or more.

Among these conventional curing agents or curing accelerators, an imidazole is preferred. The imidazole is any compound having an imidazole ring and may include, for example, an alkylimidazole such as imidazole and 2-methylimidazole, a dialkylimidazole such as 2-ethyl-4-methylimidazole, and a cyanoalkyldialkylimidazole such as 1-cyanoethyl-2-ethyl-4-methylimidazole. Such an imidazole may be used alone or in combination of two or more. Among them, the dialkylimidazole such as 2-ethyl-4-methylimidazole is preferred.

The ratio of the curing agent or curing accelerator is suitably adjusted based on an epoxy equivalent of the compound having an epoxy-containing group in the curable composition. For example, in a case where the curing agent is used, the ratio of the active hydrogen of the curing agent [such as the compound of the embodiment (A), (B), or (G), preferably the compound (1A), (1B), or (1G), and particularly the compound (1A)] relative to 1 mol of the epoxy group of the epoxy resin in the curable composition may, for example, be adjusted to 0.8 to 1.2 mol and preferably 0.9 to 1.1 mol (particularly 1 mol). The proportion of the curing agent represented by the formula (1) [such as the compound of the embodiment (A), (B), or (G), preferably the compound (1A), (1B), or (1G), and particularly the compound (1A)] relative to the whole curing agent may, for example, be about not less than 10% by mass (such as about 30 to 100% by mass), preferably about not less than 50% by mass (such as about 70 to 90% by mass), further preferably about not less than 90% by mass, and substantially about 100% by mass. In a case where the proportion of the curing agent represented by the formula (1) is excessively low, the refractive index may fail to be increased or improved effectively. According to the present invention, the compound (1) that is a curing agent has an excellent dissolubility or compatibility, and thus the compound (1) is hard to separate or precipitate in the curable composition [or the reaction system (the reaction solution or the reaction mixture)] and allows easy or efficient progress of the reaction. Thus, the compound (1) [or the constituent unit represented by the formula (1P)] is easily or efficiently introduced at a high proportion in the cured product, and the refractive index can effectively be increased or improved.

In a case where the curing accelerator is used, the ratio of the curing accelerator (such as the above-mentioned imidazole) relative to 1 mol of the epoxy group of the epoxy resin in the curable composition may, for example, be about 0.01 to 0.5 mol and preferably about 0.05 to 0.3 mol (such as about 0.1 to 0.15 mol).

### (Solvent)

The curable composition may contain a solvent if necessary. The solvent is not particularly limited to a specific one and, for example, may include the same as the solvent exemplified in the section of the mixture (blend), including preferred embodiments. The amount of the solvent is suitably adjusted depending on the intended use and others, for example, may be adjusted in consideration of coatability (or applicability), film thickness, and others in coating of the curable composition. The solvent may have the same as the concentration exemplified in the section of the mixture (blend), including preferred embodiments.

### (Other additives)

Further, the curable composition may contain a conventional additive, including a filler, a reinforcing agent, a coloring agent (such as a dye and a pigment), a stabilizer (such as a heat stabilizer, an antioxidant, and an ultraviolet absorbent), a flame retardant, an antistatic agent, a mold-releasing agent, a surfactant, a defoaming agent, a thickener, a reactive diluent, a plasticizer, a reducing agent, a polymerization inhibitor, and a binder (or any other resin, including a transparent resin such as a polycarbonate resin, a methacrylic resin, and a styrene-series resin). Such an additive may be used alone or in combination of two or more. The proportion of the total amount of these additives is not particularly limited to a specific one, and may, for example, be about 0 to 80% by mass (such as about 0.1 to 50% by mass) and preferably about 1 to 30% by mass (such as about 1 to 10% by mass) relative to the whole curable composition.

The curable composition can easily be prepared by mixing the compound (1), and optionally the above-mentioned another polymerizable compound and/or another component.

### (Cured product)

The curable composition may form a homogeneous cured product by such as heat and/or light energy. In curing the curable composition by heating, the heating temperature may, for example, be about 50 to 200°C and preferably about 100 to 150°C. In curing the curable composition by light irradiation (such as UV), the curable composition may, for example, be irradiated at about 100 to 10000 mJ/cm², and preferably about 1000 to 5000 mJ/cm²; the curable composition may be irradiated at more preferably about 10000 to 500000 mJ/cm² (such as about 30000 to 400000 mJ/cm²), and further preferably about 60000 to 300000 mJ/cm². In curing the curable composition by light irradiation (such as UV), the curable composition may, for example, be irradiated at about 10 to 5000 mW/cm², preferably about 100 to 1000 mW/cm², and further preferably about 400 to 600 mW/cm². In curing the curable composition by light irradiation (such as UV), the curable composition may be, for example, irradiated with a light having a wavelength of about 200 to 400 nm, preferably about 300 to 380 nm, and further preferably about 350 to 380 nm. Such a heating temperature or an irradiation energy may suitably be adjusted depending on the components (such as the species of the polymerizable compound, the polymerization initiator, and the curing agent or curing accelerator) and others in the curable composition.

The refractive index of the cured product may suitably be adjusted depending on the species of the polymerizable compound or the additive contained in the curable composition, or the proportion thereof. The refractive index of the cured product at a temperature of 25°C and a wavelength of 589 nm may, for example, be not less than 1.6 (such as about 1.64 to 1.73), preferably about 1.65 to 1.72 (such as about 1.66 to 1.71), and further preferably about 1.67 to 1.7(such as about 1.68 to 1.69).

In this description and claims, the refractive index can be measured in accordance with the method described in Examples below.

The cured product may be formed in a desired shape by a conventional forming method, for example, coating on a substrate and casting in a die (a mold), depending on the intended use and others. The shape is not particularly limited to a specific one. The cured product may, for example, have a one-dimensional shape including a linear shape, a rod shape, and a tube shape, a two-dimensional shape including a membrane shape, a film shape, and a sheet shape, or a three-dimensional shape (a complex shape).

### [Resin containing constituent unit represented by formula (1P)]

The compound (1) can be used as a raw material (a monomer component or a polymerizable component) to prepare a resin (a polymer), and thus the present invention includes a polymer of a polymerizable component at least containing the compound (1), particularly a resin at least containing a constituent unit represented by the formula (1P) [hereinafter, the constituent unit may be referred to as a constituent unit (1P)]: 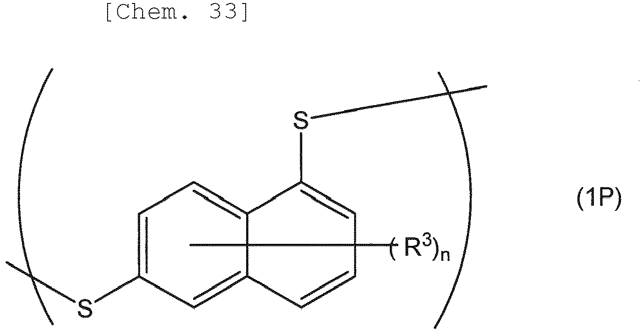 wherein R³ and n, including preferred embodiments, each have the same meanings as defined in the formula (1).

The compound (1) has a high refractive index and an excellent dissolubility or compatibility, and thus the resulting resin (the resulting polymer) may also have a high refractive index and an excellent dissolubility or compatibility. Moreover, probably because the constituent unit (1P) makes the resin difficult to crystalize, the resin often has an excellent transparency (or is amorphous) even having a large amount of the naphthalene skeleton. In particular, the resin can effectively be used even in an application having an intended high transparency, such as an optical member.

The proportion of the constituent unit (1P) in the resin may, for example, be about not less than 10 mol% (such as about 30 to 100 mol%) and preferably about not less than 40 mol% (such as about 50 to 80 mol%) relative to the whole constituent unit (the whole constituent unit derived from the polymerizable component) in the resin. Depending on the species of the resin, and others, the proportion may, for example, be about 30 to 70 mol% (such as about 40 to 60 mol%) and preferably about 45 to 55 mol% (such as about 45 to 50 mol%). In a case where the proportion of the constituent unit (1P) is excessively low, it may be difficult to effectively increase or improve a refractive index. According to the present invention, the compound (1) that is a raw material (a monomer component or a polymerizable component) has an excellent dissolubility or compatibility, and thus the compound (1) is hard to separate or precipitate in the reaction system (the reaction solution or the reaction mixture) and allows easy or efficient progress of the reaction. Thus, the constituent unit (1P) is easily or efficiently introduced at a high proportion in the resin (the polymer), and the refractive index can effectively be increased or improved.

The species or kind of the resin is not particularly limited to a specific one, and the resin may be any resin that contains at least the constituent unit (1P). The resin may be a thermosetting or photo-curable resin [such as a resin that can form the cured product of the curable composition, such as a polyfunctional (meth)acrylic resin, an epoxy resin, and a thiophenol resin]. Since the constituent unit (1P) is usually a divalent group, the resin may be a thermoplastic resin having a substantially linear structure (a non-three-dimensional network structure) [in particular, a thermoplastic resin containing at least the constituent unit (1P) (or containing the constituent unit (1P) as a repeating unit) in a main chain structure thereof].

The species of the thermoplastic resin is not particularly limited to a specific one. Examples of the thermoplastic resin may include a polyether-series resin, a polysulfide-series resin (such as a thiol-ene resin obtained by a thiol-ene reaction), a poly(thio)ester-series resin (such as a polyester resin, a polythioester resin, a polycarbonate resin, and a polythiocarbonate resin), a poly(thio)urethane-series resin (such as a thermoplastic polyurethane resin and a thermoplastic polythiourethane resin), and a (thio)phenoxy resin.

Representative examples of the resin [the polymer of the polymerizable component at least containing the compound (1)] may include a polymer at least containing, as a polymerizable component, a compound in which R¹ and R² in the formula (1) independently represent the group (2B), the group (2D), the group (2E), the group (2F), or the group (2G) [in particular, the compound (1A), the compound (1B), the compound (1D), the compound (1E), the compound (1F), and/or the compound (1G)]. Preferred examples may include a polymer of any of the following polymerizable components (a) to (c) [in particular, a polymer of a polymerizable component (a)].
(a) A polymerizable component containing a dithiol component and a component having two ethylenically unsaturated bonds; wherein
   the dithiol component contains the compound (1) in which each of R¹ and R² represents the group (2B) wherein X¹ represents a sulfur atom, and/or
   the component having two ethylenically unsaturated bonds contains the compound (1) in which each of R¹ and R² represents the group (2D) or (2E)
(b) A polymerizable component containing a diol component and/or a dithiol component, and at least one component selected from the group consisting of a dicarboxylic acid component, a diisocyanate component, and a carbonyl component; wherein
   the diol component and/or the dithiol component (the di(thi)ol component) contains the compound (1B), that is, a compound in which each of R¹ and R² in the compound (1) represents the group (2B), and/or
   the dicarboxylic acid component contains the compound (1G), that is, a compound in which each of R¹ and R² in the compound (1) represents a group defined by the formula (2G)
(c) A polymerizable component for forming a (thio)phenoxy resin; wherein the polymerizable component contains
   a diol component and/or a dithiol component, and an epihalohydrin component, wherein the diol component and/or the dithiol component contains the compound (1) in which each of R¹ and R² represents the group (2B), or
   an epoxy component and a bisphenol component, wherein the epoxy component contains the compound (1) in which each of R¹ and R² represents the group (2F)

### (Polymer of polymerizable component (a) and process for producing the same)

The polymer of the polymerizable component (a) may be a thiol-ene-based resin which is obtained by a thiol-ene reaction between a dithiol component and a component having two ethylenically unsaturated bonds. In the polymerizable component (a), at least one component selected from the group consisting of the dithiol component and the component having two ethylenically unsaturated bonds (preferably the dithiol component) contains at least the compound (1) .

In a case where the dithiol component contains the compound (1), the dithiol component may at least contain the compound (1) in which R¹ and R² independently represent the group (2B) in which X¹ represents a sulfur atom; examples of the group (2B) may include a hydrogen atom and a mercaptoC₂₋₄alkyl group such as mercaptoethyl group. Preferred examples of the compound (1) may include the compound (1A) and/or the compound (1B) in which X¹ represents a sulfur atom, specifically the compound described in any of the embodiments (A) to (B). Such a compound may be used alone or in combination of two or more.

The dithiol component may contain another dithiol component (a conventional dithiol component) different from the compound (1). Examples of another dithiol component may include the following:
an aliphatic dithiol {such as a (poly)alkanedithiol [such as 1,2-ethanedithiol and bis(2-mercaptoethyl) sulfide], a dimercapto(poly)oxaalkane [such as a dimercaptomono- to hexaoxaalkane], and a diester of an aliphatic polyol and a mercapto fatty acid [such as a diester of a (poly)alkanediol as an aliphatic polyol and a mercaptoalkanoic acid]};
an alicyclic dithiol {such as a cycloalkanedithiol (such as 1,4-dimercaptocyclohexane), and a dimercaptoalkylthiane [such as a bis(mercaptoalkyl)thiane and a bis(mercaptoalkyl)dithiane)]}; and
an aromatic dithiol {such as a dimercaptobenzene, a bis(mercaptoalkyl)benzene, a dimercaptobiphenyl, a bis(mercaptoaryl)alkane [such as 2,2-bis(4-mercaptophenyl)propane], a bis(mercaptoalkylaryl)alkane [such as 2,2-bis[4-(mercaptomethyl)phenyl]propane], a bis(mercaptoaryl) ether [such as bis(4-mercaptophenyl) ether], a bis(mercaptoalkylaryl) ether [such as bis[4-(mercaptomethyl)phenyl] ether], a bis(mercaptoaryl) sulfide [such as bis(4-mercaptophenyl) sulfide], a bis(mercaptoalkylaryl) sulfide [such as bis[4-(mercaptomethyl)phenyl] sulfide], and a heterocyclic dithiol [such as thiophenedithiol]}.
Such another dithiol component may be used alone or in combination of two or more.

The proportion of the compound (1) [the proportion of the compound in which R¹ and R² independently represent the group (2B) in which X¹ represents a sulfur atom, preferably the proportion of the total amount of the compound (1A) and/or the compound (1B) in which X¹ represents a sulfur atom] relative to the whole dithiol component may be selected from 0 to 100 mol%, and may, for example, be about not less than 10 mol% (such as about 30 to 100 mol%), preferably about not less than 50 mol% (such as about 70 to 99 mol%), further preferably not less than 80 mol% (such as about 90 to 100 mol%), and particularly substantially about 100 mol%. In a case where the proportion of the compound (1) is excessively low, it may be difficult to effectively increase or improve a refractive index. According to the present invention, the compound (1) that is a raw material (a monomer component or a polymerizable component) has an excellent dissolubility or compatibility, and thus the compound (1) is hard to separate or precipitate in the reaction system (the reaction solution or the reaction mixture) and allows easy or efficient progress of the reaction. Thus, the constituent unit (1P) is easily or efficiently introduced at a high proportion in the resin (the polymer), and the refractive index can effectively be increased or improved.

In the component having two ethylenically unsaturated bonds (ethylenically unsaturated bond-containing groups), examples of the ethylenically unsaturated bond-containing group may include the group exemplified in the section of the curable composition. Examples of the component having two ethylenically unsaturated bonds may include a divinyl component, a diallyl component, a bis(meth)acrylate component, and a diene component.

In a case where the component having two ethylenically unsaturated bonds contains the compound (1), the component may at least contain the compound (1) in which each of R¹ and R² represents the group (2D) or (2E). Preferred examples of the compound (1) may include the compound (1D) and/or the compound (1E), specifically the compound described in any of the embodiments (D) to (E). Such a compound may be used alone or in combination of two or more.

The component having two ethylenically unsaturated bonds may contain a conventional component [such as a divinyl component, a diallyl component, a bis(meth)acrylate component, and a diene component] different from the compound (1). The conventional component may, for example, be a difunctional compound among the compounds exemplified as another polymerizable compound (ethylenically unsaturated compound) in the section of the curable composition. Examples of the difunctional compound may include a divinyl (such as an aromatic divinyl such as divinylbenzene), a diallyl ether [such as diallyl ether, a (poly)alkylene glycol diallyl ether, a diallyl ether of a polyol, a diallyl ether of a biphenol (or an alkylene oxide adduct thereof) or a bisphenol (or an alkylene oxide adduct thereof), a heterocyclic diallyl ether (such as isosorbide diallyl ether); a diallyl sulfide (such as diallyl sulfide); a diallyl ester [such as a diallyl saturated aliphatic dicarboxylate, a diallyl saturated mono- to tricycloalkanedicarboxylate, a diallyl arenedicarboxylate, a diallyl ester of a bis(carboxyaryl) ether, a diallyl ester of a bis(carboxyaryl) ketone, and a diallyl ester of a bis(carboxyaryl) sulfone]; a diallyl carbonate [such as a (poly)alkylene glycol bis(allyl carbonate)]; a bisallylphenol [such as a bi(allyl-hydroxyaryl) and a bis(allyl-hydroxyaryl)alkane]; a diallylamine [such as diallylamine and a diallylalkylamine]; a diallylurea [such as 1,3-diallylurea]; a diallyl isocyanurate [such as diallyl isocyanurate, diallyl-alkyl isocyanurate, and diallyl glycidyl isocyanurate]; a diallylsilane [such as a diallyldialkylsilane and a diallyldiarylsilane]; and a diene [such as butadiene, isoprene, and 1,5-hexadiene]]. Such a component may be used alone or in combination of two or more. Among them, the aromatic divinyl such as divinylbenzene is preferred.

The proportion of the compound (1) [the proportion of the compound (1) in which each of R¹ and R² represents the group (2D) or (2E), preferably the proportion of the total amount of the compound (1D) and the compound (1E)] relative to the whole component having two ethylenically unsaturated bonds may be selected from 0 to 100 mol%, and may, for example, be about not less than 10 mol% (such as about 30 to 100 mol%), preferably about not less than 50 mol% (such as about 70 to 99 mol%), further preferably about not less than 80 mol% (such as about 90 to 100 mol%), and particularly substantially about 100 molt. In a case where the proportion of the compound (1) is excessively low, it may be difficult to effectively increase or improve a refractive index. According to the present invention, the compound (1) that is a raw material (a monomer component or a polymerizable component) has an excellent dissolubility or compatibility, and thus the compound (1) is hard to separate or precipitate in the reaction system (the reaction solution or the reaction mixture) and allows easy or efficient progress of the reaction. Thus, the constituent unit (1P) is easily or efficiently introduced at a high proportion in the resin (the polymer), and the refractive index can effectively be increased or improved.

The polymer of the polymerizable component (a) can be prepared by a thiol-ene reaction between the above components according to a conventional method. The ratio of the dithiol component relative to the component having two ethylenically unsaturated bonds may, for example, be about 1/0.8 to 1/1.2, preferably about 1/0.9 to 1/1.1, and particularly equimolar ratio in terms of the former/the latter (molar ratio).

The thiol-ene reaction may be carried out in the presence or absence of a basic catalyst or a nucleophilic catalyst, if necessary. Examples of the basic catalyst may include an alkylamine (such as a mono- to trialkylamine) and a heterocyclic amine (such as a heterocyclic tertiary amine such as DBN and DBU). Examples of the nucleophilic catalyst may include a tertiary phosphine (such as a trialkylphosphine and a triarylphosphine), and a cyclic tertiary amine (such as quinuclidine and triethylenediamine). Such a catalyst may be used alone or in combination of two or more. The ratio of the catalyst may be about 0.1 to 50 mol and preferably about 0.5 to 20 mol, relative to 100 mol of the total amount of the polymerizable component.

The thiol-ene reaction may be carried out using a radical initiator instead of the catalyst, if necessary. The radical initiator may be the polymerization initiator exemplified in the section of the curable composition. Such a polymerization initiator may be used alone or in combination of two or more. The ratio of the polymerization initiator may, for example, be about 0.1 to 15 parts by mass and preferably about 0.5 to 10 parts by mass, relative to 100 parts by mass of the total amount of the polymerizable component. In the same manner as the section of the curable composition, the photopolymerization initiator may be used in combination with the photosensitizer. The ratio of the photosensitizer may, for example, be about 1 to 200 parts by mass and preferably about 5 to 150 parts by mass, relative to 100 parts by mass of the polymerization initiator.

The thiol-ene reaction may be carried out in the presence or absence of a solvent. The solvent is an inert or inactive solvent to the reaction, for example, an inert or inactive solvent to the reaction among the organic solvents exemplified in the section of the mixture (blend). Examples of the solvent may include a halogenated hydrocarbon. Such a solvent may be used alone or in combination of two or more. The proportion of the solvent to be used is not particularly limited to a specific one.

The thiol-ene reaction may be carried out under an inert gas atmosphere, for example, under an atmosphere of nitrogen gas or a rare or noble gas such as helium and argon. The reaction temperature may, for example, be about 0 to 50°C and preferably about 10 to 40°C. The reaction time is not particularly limited to a specific one and may, for example, be about 0.5 to 3 hours.

After the completion of the reaction, the reaction product may optionally be separated with purification by a conventional separation-purification means, for example, washing, extraction, filtration, dehydration, concentration, drying, decantation, reprecipitation, chromatography, and activated carbon treatment, and a combination of these means.

### (Polymer of polymerizable component (b) and process for producing the same)

The polymer of the polymerizable component (b) may be a poly(thio)ester-series resin or a poly(thio)urethane-series resin each of which is obtained by a reaction (a condensation reaction) between a diol component and/or a dithiol component (a di(thi)ol component) and at least one component selected from the group consisting of a diisocyanate component and a carbonyl component, wherein either polymerizable component at least contains the compound (1). For example, the di(thi)ol component may contain the compound (1B), that is, a compound in which each of R¹ and R² in the compound (1) represents the group (2B), or the dicarboxylic acid component may contain the compound (1G), that is, a compound in which each of R¹ and R² in the compound (1) represents a group defined by the formula (2G).

The di(thi)ol component may at least contain a compound in which R¹ and R² in the compound (1) independently represent the group (2B) (such as hydrogen atom; a hydroxyC₂₋₄alkyl group such as hydroxyethyl group; and a mercaptoC₂₋₄alkyl group such as mercaptoethyl group). Preferred examples of the di(thi)ol component may include the compound (1A) and/or the compound (1B), specifically the compound described in any of the embodiments (A) to (B). Such a di(thi)ol component may be used alone or in combination of two or more.

The di(thi)ol component may contain another (conventional) dithiol component and/or diol component different from the compound (1). Examples of another dithiol component may include another dithiol exemplified in the section of the polymerizable component (a). Examples of another diol component may include an aliphatic diol [such as a (poly)alkanediol such as ethylene glycol, propylene glycol, and diethylene glycol]; an alicyclic diol [such as a cycloalkanediol, a bis(hydroxyalkyl)cycloalkane, and a heterocyclic diol (such as isosorbide), a bi- to tricycloalkanediol, and a spirocyclic diol]; and an aromatic diol [such as a bis(hydroxyalkyl)arene, and a bi- or bisphenol or an alkylene oxide adduct thereof]. Such another component may be used alone or in combination of two or more.

Examples of the dicarboxylic acid component may include an aliphatic dicarboxylic acid (such as an alkanedicarboxylic acid such as succinic acid, adipic acid, sebacic acid, and dodecanedioic acid, and an unsaturated aliphatic dicarboxylic acid); an alicyclic dicarboxylic acid [such as a cycloalkanedicarboxylic acid, a cycloalkenedicarboxylic acid, a bi- to tricycloalkanedicarboxylic acid, and a bi- to tricycloalkenedicarboxylic acid]; an aromatic dicarboxylic acid [such as an arenedicarboxylic acid (such as terephthalic acid, isophthalic acid, a naphthalenedicarboxylic acid, and an alkylisophthalic acid), a heterocyclic dicarboxylic acid (such as a thiophenedicarboxylic acid and a furandicarboxylic acid), a bis(carboxyaryl)alkane, and a bis(carboxyaryl)ketone]; and an esterifiable derivative of each dicarboxylic acid mentioned above (such as an alkyl ester, an acid halide, and an anhydride). Such a dicarboxylic acid component may be used alone or in combination of two or more.

Examples of the diisocyanate component may include an aliphatic diisocyanate [such as an alkane-diisocyanate such as hexamethylene diisocyanate (HDI), trimethylhexamethylene diisocyanate, and lysine diisocyanate]; an alicyclic diisocyanate [such as cyclohexane diisocyanate, isophorone diisocyanate (IPDI), dicyclohexylmethane diisocyanate (hydrogenated MDI), bis(isocyanatomethyl)cyclohexane (hydrogenated XDI), and norbornane diisocyanate]; an aromatic diisocyanate [such as phenylene diisocyanate, tolylene diisocyanate (TDI), diphenyl diisocyanate, naphthalene diisocyanate (NDI), diphenylmethane diisocyanate (MDI), tolidine diisocyanate (TODI), diphenyl ether diisocyanate, diphenylsulfone diisocyanate, xylylene diisocyanate (XDI), and tetramethylxylylene diisocyanate (TMXDI)]; a urethane prepolymer (such as a urethane diisocyanate obtained by a reaction between a polyol and a polyisocyanate); and a modified form of each component mentioned above [such as a polymer (a dimer and a trimer), a carbodiimide form, a biuret form, an allophanate form, and a uretdione form]. Such a diisocyanate component may be used alone or in combination of two or more.

Examples of the carbonyl component may include a phosgene (such as phosgene and triphosgene) and a carbonate (such as a carbonate diester such as diphenyl carbonate).

The polymer of the polymerizable component (b) can be prepared by a reaction (condensation reaction) between the above components according to a conventional method. The method is not particularly limited to a specific one, and may, for example, be solution polymerization, melt polymerization or direct polymerization, and interfacial polymerization. Since the compound (1) has an excellent dissolubility or compatibility, the method may be solution polymerization, melt polymerization, or direct polymerization.

In the reaction, the ratio of the di(thi)ol component relative to the total amount of the dicarboxylic acid component, the diisocyanate component, and the carbonyl component may usually be about equimolar ratio, or may suitably be adjusted depending on the polymerization method and others.

The reaction may be carried out in the presence of a corresponding catalyst according to the type of the reaction. For example, a catalyst for esterification (or carbonate esterification) may, for example, be a metal catalyst [such as a metal compound having a metal such as an alkali metal, an alkaline earth metal, a transition metal, and a metal in any of groups 13 to 15 of the Periodic Table (such as a metal alkoxide, an organic acid salt or an inorganic acid salt, and a metal oxide)]; and a nitrogen-containing compound [such as a quaternary ammonium hydroxide (such as a tetraalkylammonium hydroxide and a trialkyl-aralkylammonium hydroxide), a tertiary amine (such as a trialkylamine, a dimethyl-aralkylamine, and a triarylamine), an amide (such as DMF), and a pyridine (such as 4-(N,N-dimethylamino)pyridine (DMAP))]. Examples of a catalyst for urethanization may include an organic metal catalyst (such as an organotin compound and a metal naphthenate) and a tertiary amine (such as a chain amine such as a trialkylamine and an aralkyldialkylamine, and a cyclic amine such as pyridine, N,N-dimethylpiperazine, and triethylenediamine). Such a catalyst may be used alone or in combination of two or more. The amount of the catalyst is suitably adjusted depending on the kind of the polymerizable component and/or that of the reaction, and others.

The reaction may be carried out in the presence or absence of a solvent. The solvent may be an inert or inactive organic solvent to the reaction, for example, an inert or inactive solvent to the reaction, among the organic solvents described in the section of the mixture (blend), depending on the type of the reaction, and others. Examples of the solvent may include a hydrocarbon, a halogenated hydrocarbon, an ether, a ketone, an ester, and a nitrile. Such a solvent may be used alone or in combination of two or more. The proportion of the solvent to be used is not particularly limited to a specific one.

The reaction may be carried out in the presence of an additive such as a stabilizer (such as an antioxidant and a heat stabilizer) if necessary.

The reaction may be carried out under an atmosphere of an inert gas (such as nitrogen gas, and a rare or noble gas such as helium gas and argon gas). The reaction may be carried out under a normal or ordinary pressure, under an applied pressure, or under a reduced pressure. The reaction temperature is suitably adjusted depending on the kind of the polymerizable component and/or that of the reaction, and others. The reaction temperature may, for example, be about 30 to 300°C and preferably about 50 to 200°C.

After the completion of the reaction, the reaction product may optionally be separated with purification by a conventional separation-purification means, for example, washing, extraction, filtration, dehydration, concentration, drying, decantation, reprecipitation, chromatography, and activated carbon treatment, and a combination of these means.

### (Polymer of polymerizable component (c) and process for producing the same)

The polymer of the polymerizable component (c) is a (thio)phenoxy resin and contains at least the compound (1) [in particular, at least one member selected from the group consisting of the compound (1A), (1B), and (1F)] as the polymerizable component. For example, the compound (1) may be either of the following polymerizable component (c-1) or (c-2) depending on the polymerization method.

(c-1) A polymerizable component containing a diol component and/or a dithiol component (a di(thi)ol component), and an epihalohydrin component, wherein the di(thi)ol component contains a compound in which each of R¹ and R² in the compound (1) represents the group (2B)

(c-2) A polymerizable component containing an epoxy component and a bis(thio)phenol component, wherein the epoxy component contains a compound in which each of R¹ and R² in the compound (1) represents the group (2F)

In the polymerizable component (c-1), the di(thi)ol component may at least contain a compound in which R¹ and R² in the compound (1) independently represent the group (2B) (such as hydrogen atom; a hydroxyC₂₋₄alkyl group such as hydroxyethyl group; and a mercaptoC₂₋₄alkyl group such as mercaptoethyl group). Preferred examples of the di(thi)ol component may include the compound (1A) and/or the compound (1B), specifically the compound described in any of the embodiments (A) to (B). Such a di(thi)ol component may be used alone or in combination of two or more.

The di(thi)ol component may contain another (conventional) dithiol component and/or diol component different from the compound (1). Examples of another dithiol component and another diol component may include another dithiol component and another diol component exemplified in the sections of the polymerizable components (a) and (b). Such a di(thi)ol component may be used alone or in combination of two or more.

In the polymerizable component (c-1), examples of the epihalohydrin component may include the epihalohydrin (such as epichlorohydrin) exemplified in the section of the embodiment (F). Such an epihalohydrin component may be used alone or in combination of two or more.

In the polymerizable component (c-2), the epoxy component may at least contain a compound in which R¹ and R² in the compound (1) independently represent the group (2F) (such as glycidyl group and β-methylglycidyl group). Preferred examples of the epoxy component may include the compound (1F), specifically the compound described in the embodiment (F). Such an epoxy component may be used alone or in combination of two or more. The epoxy component may contain another (conventional) epoxy component different from the compound (1). Examples of another epoxy component may include the compound exemplified as another polymerizable compound (epoxy compound) in the section of the curable composition. Such an epoxy component may be used alone or in combination of two or more.

In the polymerizable component (c-2), examples of the bis(thio)phenol component may include a conventional bisphenol, specifically bisphenol A, bisphenol F, bisphenol AD, and bisphenol S. Such a bis(thio)phenol component may be used alone or in combination of two or more.

The polymerizable component (c-2) may contain the compound (1A) as the bis(thio)phenol component. Such a bis(thio)phenol component may be used alone or in combination of two or more. In the polymerizable component (c-2), the compound (1) is contained as the epoxy component and/or the bis(thio)phenol component.

The polymer of the polymerizable component (c) can be prepared by a reaction depending on the above components according to a conventional method. The method is not particularly limited to a specific one.

In the polymerizable component (c-1), the ratio of the di(thi)ol component relative to the epihalohydrin component may usually be about equimolar ratio; in the polymerizable component (c-2), the ratio of the epoxy component relative to the bis(thio)phenol component may usually be about equimolar ratio.

The reaction may be carried out in the presence of a corresponding catalyst according to the type of the reaction. The catalyst may, for example, be the phase transfer catalyst exemplified in the section of the embodiment (F), and may also include an alkali metal hydroxide (such as sodium hydroxide), a nitrogen-containing compound [such as a tertiary amine (such as a trialkylamine, a dimethyl-aralkylamine, a triarylamine, and DBU), an imidazole (such as 2-methylimidazole and 2-ethyl-4-methylimidazole), a quaternary ammonium (such as tetramethylammonium bromide and benzyltrimethylammonium bromide)], a phosphorus-containing compound [such as a phosphine (such as a trialkylphosphine and a triarylphosphine), and a phosphonium (such as an alkyltriarylphosphonium bromide and phosphonium borate)] . Such a catalyst may be used alone or in combination of two or more. The amount of the catalyst is suitably adjusted depending on the kind of the polymerizable component and/or that of the reaction, and others.

The reaction may be carried out in the presence or absence of a solvent. The solvent may be water, or an inert or inactive solvent, among the organic solvents described in the section of the mixture (blend), depending on the type of the reaction, and others. Examples of the solvent may include a hydrocarbon (such as an aromatic hydrocarbon), water, an alcohol, an ether, a glycol ether, a glycol ether acetate, a ketone, and an ester. Such a solvent may be used alone or in combination of two or more. The proportion of the solvent to be used is not particularly limited to a specific one.

The reaction may be carried out under an atmosphere of an air or an inert gas (such as nitrogen gas, and a rare or noble gas such as helium gas and argon gas). The reaction may be carried out under a normal or ordinary pressure, under an applied pressure, or under a reduced pressure. The reaction temperature is suitably adjusted depending on the kind of the polymerizable component and/or that of the reaction, and others. The reaction temperature may, for example, be about 50 to 300°C and preferably about 100 to 250°C.

After the completion of the reaction, the reaction product may optionally be separated with purification by a conventional separation-purification means, for example, washing, extraction, filtration, dehydration, concentration, drying, decantation, reprecipitation, chromatography, and activated carbon treatment, and a combination of these means.

The resin at least containing the constituent unit (1P) may, for example, have a refractive index at a temperature of 25°C and a wavelength of 589 nm of about not less than 1.6 (such as about 1.65 to 1.74), preferably about 1.66 to 1.73 (such as about 1.67 to 1.72), and further preferably about 1.68 to 1.71 (such as about 1.69 to 1.7).

In this description and claims, the refractive index can be measured in accordance with the method described in Examples below.

The resin at least containing the constituent unit (1P) may be in the form of a resin composition containing another component if necessary. Examples of another component may include a conventional additive and another resin [a resin free from the constituent unit (1P)].

The conventional additive may contain, for example, the conventional additive (such as a filler and a reinforcing agent) exemplified in the section "Other additives" of the curable composition. Such an additive may be used alone or in combination of two or more.

In a case where the resin composition contains another resin, the resin composition may be in the form of a polymer alloy or a polymer blend. Another resin is not particularly limited to a specific one, and examples of another resin may include the following:
a curable resin [such as a phenol resin (such as a resol-type or novolac-type phenol resin), an amino resin (such as a urea resin and a melamine resin), a furan resin, an unsaturated polyester resin, a diallyl phthalate resin, a polyfunctional (meth)acrylic resin, an epoxy resin, a vinyl ester resin, a curable polyurethane-series resin, a curable polyimide-series resin, and a curable silicone-series resin]; and
a thermoplastic resin [such as a polyolefin resin (such as a chain olefinic resin and a cyclic olefinic resin); a styrene-series resin (such as a general purpose polystyrene (GPPS), a syndiotactic polystyrene (SPS), a styrene-methyl methacrylate copolymer (MS resin), a styrene-acrylonitrile copolymer (AS resin), a high impact polystyrene (HIPS), an acrylonitrile-butadiene-styrene copolymer (ABS resin), an acrylonitrile-acrylic rubber-styrene copolymer (AAS resin), an acrylonitrile-chlorinated polyethylene-styrene copolymer (ACS resin), an acrylonitrile-(ethylene-propylene-diene rubber)-styrene copolymer (AES resin), and a methyl methacrylate-butadiene-styrene copolymer (MBS resin)); a monofunctional (meth)acrylic resin (such as a polymethyl methacrylate (PMMA) and a (meth)acrylic acid-(meth)acrylic acid ester copolymer); a vinyl acetate-series resin (such as a polyvinyl acetate (PVAc), a polyvinyl alcohol (PVA), and a polyvinyl acetal); a vinyl chloride-series resin (such as a vinyl chloride homopolymer or copolymer, and a vinylidene chloride copolymer); a fluororesin (such as a polytetrafluoroethylene (PTFE), a polyvinylidene fluoride (PVDF), a polychlorotrifluoroethylene (PCTFE), a polyvinyl fluoride (PVF), a tetrafluoroethylene-hexafluoropropylene copolymer (FEP), a tetrafluoroethylene-perfluoroalkylvinyl ether copolymer (PFA), an ethylene-tetrafluoroethylene copolymer (ETFE), and an ethylene-chlorotrifluoroethylene copolymer (ECTFE)); a poly(thio)ester-series resin (such as a polyalkylene arylate-series resin (such as a polyethylene terephthalate (PET), a polybutylene terephthalate (PBT), and a polyethylene naphthalate (PEN)), a polyarylate-series resin, and a polycarbonate (PC) resin (such as a bisphenol A-type polycarbonate-series resin)); a thermoplastic poly(thio)urethane-series resin; a polyamide (PA)-series resin (such as an aliphatic polyamide resin (such as a polyamide 66), a semi-aromatic polyamide resin, and a aramid resin); a thermoplastic polyimide resin (such as a polyetherimide (PEI) and a polyamideimide); a polyacetal-series resin; a polyphenylene ether (PPE)-series resin; a polyetherketone-series resin (such as a polyetherketone (PEK) resin, a polyetheretherketone (PEEK) resin, and a polyetherketoneetherketoneketone (PEKEKK) resin); a (thio)phenoxy resin; a polyketone resin; a polyphenylene sulfide-series resin (PPS); a polysulfone-series resin (such as a polysulfone (PSF) resin, and a polyethersulfone (PES) resin); a cellulose derivative (such as a cellulose ester and a cellulose ether); a polyether nitrile resin; and a thermoplastic elastomer (TPE)].

The proportion of the total amount of these other components is not particularly limited to a specific one, and may, for example, be about 0 to 80% by mass (such as about 0.1 to 50% by mass) and preferably about 1 to 30% by mass (such as about 1 to 10% by mass), relative to the whole.

The resin at least containing the constituent unit (1P) may be formed into a desired form by a conventional molding method (such as an injection molding and an extrusion molding) depending on the intended use and/or others, to obtain a molded product. The shape of the molded product is not particularly limited to a specific one and may include, for example, the same as the shape exemplified in the section of the cured product.

### [Resin composition]

The present invention includes a resin composition at least containing the compound (1) and a resin. The resin composition has an increased or improved refractive index due to the compound (1). Specifically, the compound (1) functions as a refractive index increasing agent in the resin composition. In particular, probably because the compound (1) has an excellent dissolubility in or compatibility with the resin, even the resin composition containing a large amount of the compound (1) can be prepared homogeneously while being effectively prevented from precipitation or aggregation of the compound (1). Thus, the resin composition significantly increases a refractive index while having a transparency and is suitably used for an optical member and others.

The compound (1) is not particularly limited to a specific one and may preferably be the compound of the embodiment (C) [particularly the compound (1C)] from the point of view of dispersibility or non-reactivity (stability) and others.

The resin in the resin composition is not particularly limited to a specific one, may be a thermoplastic resin or may be thermosetting or photo-curable resin. The resin may preferably be a thermoplastic resin. Examples of the resin may include the resin containing the constituent unit (1P), and another resin exemplified in the above-mentioned section "[Resin containing constituent unit represented by formula (1P)]" [the resin free from the constituent unit (1P), preferably the thermoplastic resin]. Such a resin may be used alone or in combination of two or more. The resin may preferably be a (meth)acrylic resin such as a PMMA.

In the resin composition, the ratio of the compound (1) may, for example, be about 1 to 300 parts by mass (such as about 30 to 200 parts by mass), preferably about 50 to 150 parts by mass (such as about 70 to 130 parts by mass), and further preferably about 80 to 120 parts by mass (such as about 90 to 110 parts by mass), relative to 100 parts by mass of the resin. In a case where the ratio of the compound (1) is excessively low, it may be difficult to effectively increase or improve a refractive index. In a case where the ratio of the compound (1) is excessively high, the resin composition may have a low transparency due to the separation or precipitation of the compound (1) or may have low mechanical characteristics. However, according to the present invention, the compound (1) also has an excellent dissolubility in or compatibility with the resin, and even the resin composition containing the compound (1) at a relatively high concentration allows a significant increase or improvement in refractive index while being effectively prevented from precipitation or aggregation of the compound (1).

The resin composition may contain a conventional additive. Examples of the conventional additive may include the conventional additive (such as a filler and a reinforcing agent) exemplified in the section "(Other additives)" of the curable composition. Such an additive may be used alone or in combination of two or more. The total amount of the additive is not particularly limited to a specific one, and may, for example, be about 0 to 80% by mass (such as about 0.1 to 50% by mass) and preferably about 1 to 30% by mass (such as about 1 to 10% by mass), relative to the whole resin composition.

The resin composition may be formed into a desired form by a conventional molding method (such as an injection molding and an extrusion molding) depending on the intended use and others, to give a molded product. The shape of the molded product is not particularly limited to a specific one and may include, for example, the same as the shape exemplified in the section of the cured product.

The refractive index of the resin composition may suitably be adjusted depending on the species of the resin or the additive contained in the resin composition, or the proportion thereof. The refractive index of the resin composition at a temperature of 25°C and a wavelength of 589 nm may, for example, be not less than 1.6 (such as about 1.6 to 1.7) and preferably about 1.61 to 1.65 (such as about 1.62 to 1.63).

In this description and claims, the refractive index can be measured in accordance with the method described in Examples below.

The process for producing the resin composition is not particularly limited to a specific one, and includes mixing the compound (1) and the resin. The mixing may be carried out while heating, and the heating temperature may, for example, be about 40 to 100°C and preferably about 50 to 70°C. If necessary, the compound (1) and the resin may be mixed with an organic solvent [such as the same solvent as the organic solvent exemplified in the section of the mixture (blend), including preferred embodiments, preferably an ether such as THF], and the organic solvent may be removed from the resulting mixture (the mixture containing the compound (1), the resin, and the organic solvent) by means such as heating and/or reduced pressure, thus obtaining the resin composition.

### [Method for improving dissolubility or compatibility]

The present invention includes a method for improving a dissolubility or compatibility of a naphthalene compound having sulfur atoms directly bonded to a naphthalene ring thereof at least at two positions (or substitution positions), wherein the improvement comprises including or positioning the sulfur atoms at least at the 1,6-positions (or preparing the positions of the sulfur atoms so as to be at least the 1,6-positions, or using a naphthalene compound having sulfur atoms at least at the 1,6-positions); the method enables the naphthalene compound to have an improved dissolubility or compatibility compared with corresponding other positional isomers (such as 1,5-, 2,6-, or 2,7-isomer). The method may be a method for improving the dissolubility or compatibility of the naphthalene compound, which comprise introducing the constituent unit (1P) in the chemical structure of the compound.

The sulfur atom directly bonded to each of the 1,6-positions of the naphthalene ring may be a sulfur atom of sulfinyl group [-S(=O)-] or sulfonyl group [-S(=O)₂-], and may preferably be a sulfur atom of sulfide bond [-S-] or mercapto group. The sulfur atom directly bonded to each of the 1,6-positions of the naphthalene ring may be a sulfur atom of a polythio group [-Sₙ-] such as dithio group [-S-S-], or a mercaptopolythio group [-Sₙ-H] (in each formula, n denotes an integer of not less than 2).

The naphthalene compound having sulfur atoms directly bonded to a naphthalene ring thereof at least at two positions may be a low molecular weight compound or may be a high molecular weight compound (a resin or a polymer).

This method is useful in that simply positioning of sulfur atoms at the 1,6-positions allows a significant improved dissolubility or compatibility without considerable decrease of a refractive index of the compound (or while maintaining or improving a refractive index).

A subject in which the naphthalene compound has a high dissolubility or compatibility (or a compound in which the naphthalene compound is to be dissolved, or a compound with which the naphthalene compound is to be compatible) may preferably include an organic compound. Examples of the organic compound may include the above-mentioned organic solvent, polymerizable compound or polymerizable component, and resin [such as the resin containing the constituent unit represented by the formula (1P), another resin described in the section "[Resin containing constituent unit represented by formula (1P)]" (such as a (meth)acrylic resin such as a PMMA)]. Further preferred examples may include the liquid organic compound (such as an organic compound that is liquid at a room temperature (about 25°C)), especially the organic solvent exemplified in the section of the mixture (or blend) mentioned above, and another polymerizable compound (such as the ethylenically unsaturated compound) exemplified in the section of the curable composition mentioned above. The preferred embodiments of these compounds may be the same as the above-exemplified embodiments.

The particularly preferred organic solvent may include a hydrocarbon (such as an aromatic hydrocarbon such as toluene), a halogenated hydrocarbon (such as a chlorinated hydrocarbon such as methylene chloride and chloroform), an alcohol such as methanol, an ether (such as a cyclic ether such as THF, and an epihalohydrin such as epichlorohydrin), a glycol ether acetate (such as a (poly)C₂₋₄alkylene glycol monoC₁₋₄alkyl ether acetate such as PGMEA), an ester (such as an acetate ester such as ethyl acetate), an amide (such as a chain amide such as DMF), and a urea (such as a cyclic urea such as DMI).

The particularly preferred polymerizable compound may include an ethylenically unsaturated compound (such as a monofunctional ethylenically unsaturated compound); more preferably an aromatic vinyl, (meth)acrylic acid or a derivative thereof [such as (meth)acrylic acid and a (meth)acrylate]; and further preferably a styrene (such as styrene, α-methylstyrene, and vinyltoluene), a (meth)acrylate such as an aliphatic (meth)acrylate [such as a C₁₋₆alkyl (meth)acrylate such as methyl (meth)acrylate].

### EXAMPLES

The following examples are intended to describe this invention in further detail and should by no means be interpreted as defining the scope of the invention. The evaluation methods were shown below.

### (¹H-NMR)

The ¹H-NMR was measured using "JNM-ECZ400 (400 MHz)" manufactured by JEOL Ltd. and "JNM-ECA600 (600 MHz)" manufactured by JEOL Ltd., and using chloroform-d (CDCl₃) as a deuterated solvent and tetramethylsilane (TMS) as a standard substance.

### (Purity)

The HPLC purity was determined using HPLC (high-performance (or high-speed) liquid chromatography, "SPD-20A" manufactured by SHIMADZU CORPORATION), by calculation based on an area normalization (or area percentage) method using a reverse-phase column.

### (Melting point)

The melting point was measured using a melting point measuring apparatus ("535" manufactured by BUCHI) in accordance with Japanese Industrial Standards (JIS) K 4101 (1993) [5.1 Method according to Visual Observation].

### (Refractive index)

The refractive index of each sample was measured using an abbe refractometer ("NAR-1T" manufactured by ATAGO CO., LTD.) at a temperature of 25°C and a wavelength of 589 nm. Depending on the species of each sample, the refractive index was measured as follows.

For the after-mentioned 16NDSH, 15NDSH, 26NDSH and 27NDSH; 15DGNDSH; 15DHENDSH; 16DMNDSH and 15DMNDSH; and 16DCNDSH and 15DCNDSH, a sample was dissolved in 1,3-dimethyl-2-imidazolidinone (DMI) to prepare multiple solutions with different concentrations, and the refractive index of each solution was measured at a temperature of 25°C and a wavelength of 589 nm. From the results of the refractive index at each concentration, the refractive index value at 100% by mass of the concentration was determined by extrapolation.

For the after-mentioned 16NDSHMA, 15NDSHMA, 26NDSHMA and 27NDSHMA; 16NDSHA and 15NDSHA; 15DANDSH; and 15DVNDSH, polymerization may occur during the mixing, and thus a sample was dissolved in a methoquinone/DMI solution (a solution containing 4000 ppm of methoquinone relative to the mass of DMI) to prepare multiple solutions with different concentrations. The refractive index of each solution was measured at a temperature of 25°C and a wavelength of 589 nm. From the results of the refractive index at each concentration, the refractive index value at 100% by mass of the concentration was determined by extrapolation.

For the after-mentioned 16DANDSH; 16DVNDSH; 16DGNDSH; and 16DHENDSH, a sample was liquid for a while after preparation, and thus the refractive index was measured in the state of an undiluted solution. Further, thiol-ene-based resins (thiol-ene-based resins obtained from 16NDSH or 15NDSH and divinylbenzene) were measured in the same manner.

For the after-mentioned cured product of the curable composition containing the bis(methacryloylthio)naphthalene compound, the after-mentioned cured product of the curable composition containing the epoxy resin curing agent, and the after-mentioned resin composition containing the refractive index increasing agent, the solid sample (the cured product or the resin composition) was directly measured using methylene iodide as an intermediate liquid.

### (Dissolubility or compatibility)

In each of predetermined compounds (solvents or polymerizable components) described in the Tables below, a sample was mixed at a temperature of 25°C, and the dissolubility (or compatibility) of the sample was evaluated. In more detail, the sample was added to a predetermined amount of a solvent or a polymerizable component until the sample was no longer dissolved, and the sample content of the liquid layer (the solvent layer) of the resulting mixture was analyzed using an HPLC ("SPD-20A" manufactured by SHIMADZU CORPORATION), and thus the concentration (the maximum dissolved concentration) of the sample in the liquid layer (the solvent layer) was determined as a solubility.

### <Naphthalenedithiol compound>

### [Example 1] Synthesis of 16NDSH

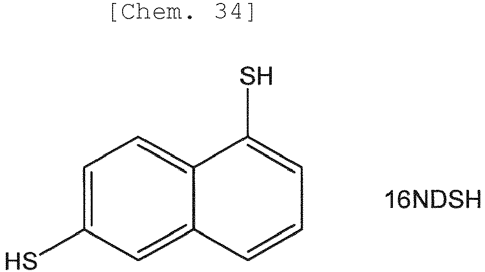

In a 1000 mL flask, 100 g (300.9 mmol) of disodium 1,6-naphthalenedisulfonate, 451 g of toluene, 8.8 g (0.4 molar ratio) of N,N-dimethylformamide, and 89.6 g (2.5 molar ratio) of thionyl chloride were charged under a nitrogen atmosphere, and the mixture was heated to 100°C. After the heating, the temperature fluctuated from 97 to 102°C, and the reaction was carried out for 8 hours while maintaining the temperature in a thermostatic bath. After the completion of the reaction, the reaction mixture was cooled to 30°C, and 451 g of water was added to the cooled mixture at 20 to 30°C, and the resulting mixture was stirred for 10 minutes or more. The resulting lower aqueous (or water) layer was removed by liquid-liquid separation (or liquid-liquid phase separation). To the obtained organic layer was added 451 g of water in the same manner, the resulting mixture was stirred for 10 minutes or more and was subjected to liquid-liquid separation to obtain, as an organic layer, 622.5 g of a toluene solution containing 1,6-naphthalenedisulfonyl chloride (16NDSC) (purity by HPLC: 98%, theoretical yield: 97 mol%).

In a 5000 mL flask, 1800 g of toluene and 1439.8 g (48.8 molar ratio) of 36% by mass hydrochloric acid were charged under a nitrogen atmosphere, and the mixture was heated to 60°C. After the heating, 622.5 g (291.0 mmol) of the above-mentioned 16NDSC toluene solution and 258.8 g (13.6 molar ratio) of zinc powder were dividedly added to the heated mixture over 3 hours and 30 minutes. The temperature during the addition was 60 to 70°C, and the reaction was carried out for one hour while maintaining the temperature in a thermostatic bath. After the completion of the reaction, the reaction mixture was cooled to 30°C and was allowed to stand at 0 to 30°C. Then, the resulting lower aqueous layer was removed by liquid-liquid separation. Thereafter, the obtained organic layer was subjected to filtration, and the residue was washed with 33 g of toluene. At not higher than 45°C under a reduced pressure, the obtained filtrate was subjected to desolvation and drying, and 50.5 g of a solid matter containing 1,6-naphthalenedithiol (16NDSH) was obtained (purity of HPLC: 99%, theoretical yield: 90 mol%) .

The resulting 16NDSH has a melting point of 35 to 36°C and a refractive index of 1.722, and shows the results of ¹H-NMR as follows.

¹H-NMR (CDCl₃): δ (ppm) 3.57 (s, 1H), 3.61 (s, 1H), 7.31 (dd, J 8.4 7.4, 1H), 7.40 (dd, J 9.0 1.8, 1H), 7.48 (d, J 7.2, 1H), 7.55 (d, J 8.4, 1H), 7.72 (d, J 1.8, 1H), 8.03 (d, J 9.0, 1H)

### [Comparative Example 1-1] Synthesis of 15NDSH

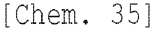

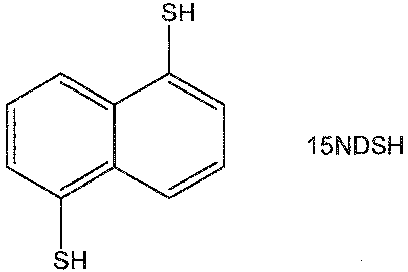

In a 5 L flask, 100 g (300.9 mmol) of disodium 1,5-naphthalenedisulfonate, 2003 g of toluene, 8.8 g (0.4 molar ratio) of N,N-dimethylformamide, and 85.9 g (2.4 molar ratio) of thionyl chloride were charged under a nitrogen atmosphere, and the mixture was heated to 100°C. After the heating, the temperature fluctuated from 97 to 102°C, and the reaction was carried out for 5 hours while maintaining the temperature in a thermostatic bath. After the completion of the reaction, the reaction mixture was cooled to 30°C, and 451 g of water was added thereto at 20 to 30°C, and the resulting mixture was stirred for 10 minutes or more. The resulting lower aqueous layer was removed by liquid-liquid separation. In the same manner, 150 g of water was added to the obtained organic layer. The resulting mixture was stirred for 10 minutes or more, and an organic layer was obtained by liquid-liquid separation. The organic layer was subjected to filtration, and the residue was washed with 30 g of toluene to obtain 2141.6 g of a toluene solution containing 1,5-naphthalenedisulfonyl chloride (15NDSC) (purity by HPLC: 96%, theoretical yield: 96 mol%).

In a 10 L flask, 580 g of toluene and 1422.1 g (48.4 molar ratio) of 36% by mass hydrochloric acid were charged under a nitrogen atmosphere, and the mixture was heated to 60°C. After the heating, 2141.6 g (289.8 mmol) of the above-mentioned 15NDSC toluene solution and 257.7g (13.6 molar ratio) of zinc powder were dividedly added to the heated mixture over one hour and 30 minutes. The temperature during the addition was 60 to 70°C, and the reaction was carried out for one hour while maintaining the temperature in a thermostatic bath. After the completion of the reaction, the reaction mixture was cooled to 30°C and was allowed to stand at 0 to 30°C, and the resulting lower aqueous layer was removed by liquid-liquid separation. Thereafter, the obtained organic layer was subjected to filtration, and the residue was washed with 188 g of toluene. At not higher than 40°C under a reduced pressure, the resulting filtrate was subjected to desolvation and drying, and 58.5 g of a solid matter containing 1,5-naphthalenedithiol (15NDSH) was obtained (purity by HPLC: 99%, theoretical yield: 99 mol%).

The resulting 15NDSH has a melting point of 120 to 121°C and a refractive index of 1.711, and shows the results of ¹H-NMR as follows.
¹H-NMR (CDCl₃): δ (ppm) 3.62 (s, 2H), 7.40 (dd, J 8.4 7.2, 2H), 7.59 (d, J 7.2, 2H), 8.04 (d, J 8.4, 2H)

### [Comparative Example 1-2] Synthesis of 26NDSH

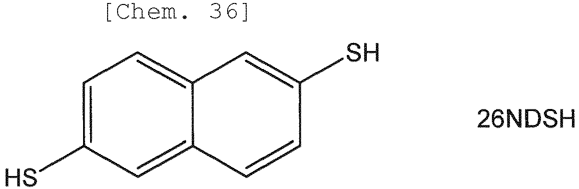

In a 1 L flask, 100 g (300.9 mmol) of disodium 2,6-naphthalenedisulfonate, 451 g of toluene, 8.8 g (0.4 molar ratio) of N,N-dimethylformamide, and 100.3 g (2.8 molar ratio) of thionyl chloride were charged under a nitrogen atmosphere, and the mixture was heated to 100°C. After the heating, the temperature fluctuated from 97 to 102°C, and the reaction was carried out for 8 hours while maintaining the temperature in a thermostatic bath. After the completion of the reaction, the reaction mixture was cooled to 30°C, and 226 g of water was added thereto at 20 to 30°C, and the resulting mixture was stirred for 2 hours or more and was then subjected to filtration. The resulting wet crystal was washed with 60 g of toluene, and the washed wet crystal was dried to obtain 92.6 g of 2,6-naphthalenedisulfonyl chloride (26NDSC) as a solid matter (purity by HPLC: 98%, theoretical yield: 93 mol%).

In a 10 L flask, 4478 g of toluene and 249.0 g (13.6 molar ratio) of zinc powder were charged under a nitrogen atmosphere, and the mixture was heated to 60°C. After the heating, 92.6 g (279.9 mmol) of 26NDSC and 692.4 g (24.4 molar ratio) of 36% by mass hydrochloric acid were dividedly added to the heated mixture over one hour. The temperature during the addition was 60 to 70°C, and the reaction was carried out for 2 hours while maintaining the temperature in a thermostatic bath. After the completion of the reaction, the reaction mixture was subjected to filtration, and the resulting residue was washed with 55 g of toluene that was pre-heated to 60°C. The resulting filtrate was subjected to liquid-liquid separation at 55 to 60°C, then removing a lower aqueous layer. Thereafter, 221 g of water and 21.0 g (0.74 molar ratio) of 36% by mass hydrochloric acid were added to the obtained organic layer, and the mixture was heated to 50°C. After the heating, the temperature fluctuated from 50 to 60°C, and the mixture was stirred for 30 minutes while maintained at the above-mentioned temperature in a thermostatic bath. Then the resulting lower aqueous layer was removed by liquid-liquid separation. The obtained organic layer was subjected to desolvation to 398 g at not higher than 60°C under a reduced pressure, and the desolvated mixture was cooled to 5°C. After the cooling, the temperature fluctuated from 0 to 5°C, and the desolvated mixture was stirred for one hour while maintained at the above-mentioned temperature in a thermostatic bath, and then was subjected to filtration. The resulting wet crystal was washed with 55 g of water, and the washed wet crystal was dried to obtain 40.2 g of a solid matter containing 2,6-naphthalenedithiol (26NDSH) (purity by HPLC: 99%, theoretical yield: 75 mol%).

The resulting 26NDSH has a melting point of 196 to 197°C and a refractive index of 1.718, and shows the results of ¹H-NMR as follows.
¹H-NMR (CDCl₃): δ (ppm) 3.58 (s, 2H), 7.32 (dd, J 8.0 1.6, 2H), 7.57 (d, J 8.0, 2H), 7.68 (d, J 1.6, 2H)

### [Comparative Example 1-3] Synthesis of 27NDSH

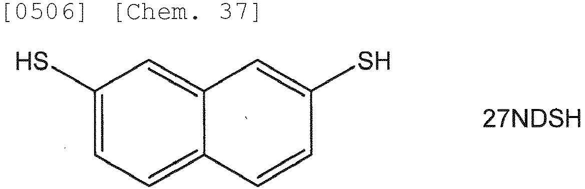

In a 1 L flask, 100 g (300.9 mmol) of disodium 2,7-naphthalenedisulfonate, 632 g of toluene, 8.8 g (0.4 molar ratio) of N,N-dimethylformamide, and 89.5 g (2.5 molar ratio) of thionyl chloride were charged under a nitrogen atmosphere, and the mixture was heated to 100°C. After the heating, the temperature fluctuated from 97 to 102°C, and the reaction was carried out for 3 hours while maintaining the temperature in a thermostatic bath. After the completion of the reaction, the reaction mixture was cooled to 30°C, and 451 g of water was added thereto at 20 to 30°C, and the resulting mixture was stirred for 10 minutes or more. The resulting lower aqueous layer was removed by liquid-liquid separation. In the same manner, 451 g of water was added to the obtained organic layer, and the resulting mixture was stirred for 10 minutes or more and was subjected to liquid-liquid separation to obtain, as an organic layer, 738.6 g of a toluene solution containing 2,7-naphthalenedisulfonyl chloride (27NDSC) (purity by HPLC: 99%, theoretical yield: 99 mol%).

In a 10 L flask, 6507 g of toluene and 1264.3 g (41.9 molar ratio) of 36% by mass hydrochloric acid were charged under a nitrogen atmosphere, and the mixture was heated to 60°C. After the heating, 738.6 g (297.6 mmol) of the 27NDSC toluene solution and 264.7 g (13.6 molar ratio) of zinc powder were dividedly added to the heated mixture over 3 hours and 30 minutes. The temperature during the addition was 60 to 70°C, and the reaction was carried out for one hour while maintaining the temperature in a thermostatic bath. After the completion of the reaction, the reaction mixture was cooled to 30°C and was allowed to stand at 0 to 30°C, and the resulting lower aqueous layer was removed by liquid-liquid separation. Thereafter, the obtained organic layer was subjected to filtration, and the residue was washed with 75 g of toluene. The resulting filtrate was subjected to desolvation to 203 g at not higher than 55°C under a reduced pressure, and the desolvated mixture was subjected to filtration. The resulting wet crystal was washed with 59 g of toluene, and the washed wet crystal was dried to obtain 43.1 g of a solid matter containing 2,7-naphthalenedithiol (27NDSH) (purity by HPLC: 99%, theoretical yield: 75 mol%) .

The resulting 27NDSH has a melting point of 186 to 188°C and a refractive index of 1.721, and shows the results of ¹H-NMR as follows.
¹H-NMR (CDCl₃): δ (ppm) 3.60 (s, 2H), 7.27 (dd, J 8.4 1.2, 2H), 7.58 (d, J 1.2, 2H), 7.64 (d, J 8.4, 2H)

The following Table shows the evaluation results of the naphthalenedithiols obtained in Example 1 and Comparative Examples 1-1 to 1-3. In the Table, THF means tetrahydrofuran, DMF means N,N-dimethylformamide, and PGMEA means propylene glycol monomethyl ether acetate (the same applies hereinafter).

### [Table 1]

**Table 1**

| Naphthalenedithiol compound | | Refractive index | Dissolubility [% by mass] | | | | | |
|---|---|---|---|---|---|---|---|---|
| | | | Methanol | Toluene | THF | DMF | Ethyl acetate | PGMEA |
| Example 1 | 16NDSH | 1.722 | 19.3 | Freely blended (>90) | | | | 85.3 |
| Comparative Example 1-1 | 15NDSH | 1.711 | 1.08 | 7.26 | 29.0 | 34.4 | 7.05 | 7.44 |
| Comparative Example 1-2 | 26NDSH | 1.718 | 0.11 | 0.64 | 4.03 | 3.54 | 0.65 | 0.73 |
| Comparative Example 1-3 | 27NDSH | 1.721 | 0.16 | 0.76 | 5.24 | 7.75 | 0.90 | 1.05 |

As apparent from the results shown in Table 1, among Comparative Examples, Comparative 1-1 (15NDSH), which had the lowest refractive index, had the highest dissolubility in all solvents. On the other hand, Example 1 (16NDSH) had both high refractive index and high dissolubility compared with Comparative Example 1-1 (15NDSH), in particular, had a significantly high dissolubility. Specifically, Example 1 (16NDSH) was dissolvable even at a concentration of not less than 90% by mass in each one of toluene, THF, DMF, and ethyl acetate, and even at a concentration of not less than 85% by mass in PGMEA.

### <Bis(methacryloylthio)naphthalene compound>

### [Example 2] Synthesis of 16NDSHMA

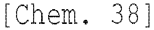

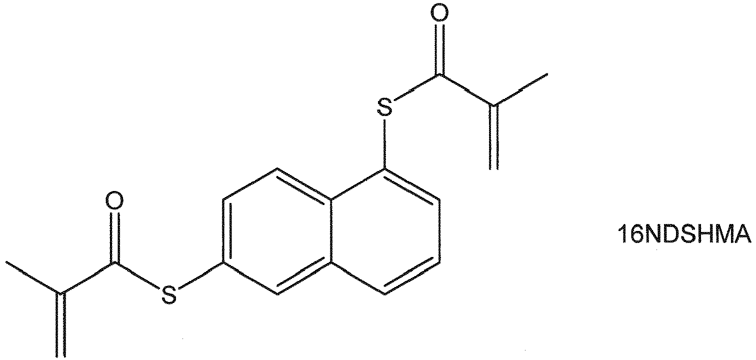

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 16NDSH, and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C, and 2 g of a filter auxiliary agent was added thereto. The resulting mixture was stirred for 5 minutes or more and was subjected to filtration. The residue was washed with 5 g of water to obtain a 16NDSH·Na liquid (an aqueous solution containing a sodium salt of 16NDSH).

To a 200 mL flask, 100 g of methylene chloride, 0.05 g of methoquinone, and 13.1 g (2.4 molar ratio) of methacryloyl chloride were added under a nitrogen atmosphere and under light shielding, and the mixture was cooled to 5°C. After the cooling, the temperature fluctuated from 0 to 5°C, and the above-mentioned temperature was maintained in a thermostatic bath. Thereafter, the above-mentioned 16NDSH·Na liquid was dropwise added thereto over 30 minutes, and the reaction was carried out at the above-mentioned temperature for one hour. After the completion of the reaction, the reaction mixture was allowed to stand at 0 to 30°C, and then the resulting upper aqueous layer was removed by liquid-liquid separation. The obtained organic layer was subjected to desolvation at not higher than 30°C under a reduced pressure to obtain 24 g of a concentrated liquid. The concentrated liquid was cooled to 15°C. After the cooling, the temperature fluctuated from 10 to 15°C. While the concentrated liquid was maintained at the above-mentioned temperature in a thermostatic bath, 21 g of methanol was dropwise added thereto over 30 minutes, and then the resulting mixture was cooled to 5°C. After the cooling, the temperature fluctuated from 0 to 5°C, and the mixture was stirred for one hour or more while maintained at the above-mentioned temperature in a thermostatic bath. Thereafter, the mixture was subjected to filtration. The resulting wet crystal was washed with 23 g of methanol that was pre-cooled to 0 to 5°C, and the washed wet crystal was dried to obtain 13.7 g of a solid matter containing 1,6-bis(methacryloyl sulfide)naphthalene (or 1,6-bis(methacryloylthio)naphthalene, 16NDSHMA) (purity by HPLC: 100%, theoretical yield: 79 mol%).

The resulting 16NDSHMA has a melting point of 68 to 69°C and a refractive index of 1.647, and shows the results of ¹H-NMR as follows.
¹H-NMR (CDCl₃): δ (ppm) 2.03 (s, 6H), 5.74 (d, J 2.4, 1H), 5.79 (d, J 1.8, 1H), 6.25 (s, 1H), 6.36 (s, 1H), 7.52-7.56 (m, 2H), 7.76 (m, 1H), 7.94 (d, J 8.4, 1H), 8.02 (d, J 1.2, 1H), 8.21 (d, J 8.4, 1H)

### [Comparative Example 2-1] Synthesis of 15NDSHMA

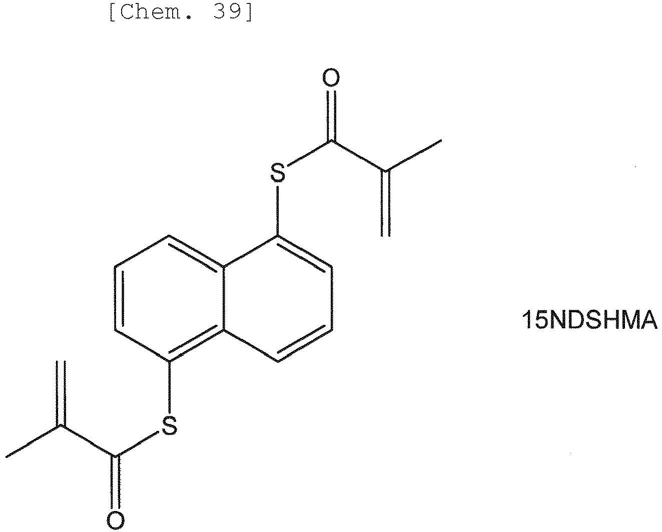

In a 300 mL flask, 241 g of monochlorobenzene was charged under a nitrogen atmosphere and was heated to 60°C. To this liquid was added 10 g (52.0 mmol) of 1,5-naphthalenedithiol (15NDSH), and the mixture was stirred until complete dissolution. After the heating, the temperature fluctuated from 55 to 65°C, and the above-mentioned temperature was maintained in a thermostatic bath. To this solution was dropwise added a mixture of 18.2 g (2.8 molar ratio) of a 32% by mass sodium hydroxide aqueous solution, 40 g of water, and 0.1 g (0.05 molar ratio) of sodium borohydride, and the reaction was carried out at the above-mentioned temperature for 30 minutes. After the completion of the reaction, the resulting upper organic layer was removed by liquid-liquid separation, and the obtained aqueous layer was cooled to 30°C to obtain a 15NDSH·Na liquid (an aqueous solution containing a sodium salt of 15NDSH).

To a 300 mL flask, 156 g of toluene, 0.05 g of hydroquinone, and 12.0 g (2.2 molar ratio) of methacryloyl chloride were added under a nitrogen atmosphere and under light shielding, and the mixture was cooled to 5°C. After the cooling, the temperature fluctuated from 0 to 5°C, and the above-mentioned temperature was maintained in a thermostatic bath. Thereafter, the above-mentioned 15NDSH·Na liquid was poured into the flask, and the reaction was carried out at the above-mentioned temperature for 30 minutes. After the completion of the reaction, the reaction mixture was allowed to stand for 0 to 30°C, and then the resulting lower aqueous layer was removed by liquid-liquid separation. To the obtained organic layer was added 52 g of water, and the resulting mixture was stirred for 20 minutes or more. The resulting lower aqueous layer was removed by liquid-liquid separation. The obtained organic layer was subjected to desolvation at not higher than 30°C under a reduced pressure to obtain 39 g of a concentrated liquid. The concentrated liquid was heated to 40°C. After the heating, the temperature fluctuated from 37 to 42°C. While the concentrated liquid was maintained at the above-mentioned temperature in a thermostatic bath, 25 g of methanol was dropwise added thereto over 15 minutes, and then the resulting mixture was stirred for 25 minutes. Thereafter, the mixture was cooled to 5°C. After the cooling, the temperature fluctuated from 0 to 5°C, and the mixture was stirred for 30 minutes or more while maintained at the above-mentioned temperature in a thermostatic bath. Thereafter, the mixture was subjected to filtration. The resulting wet crystal was washed with 20 g of methanol, and the washed wet crystal was dried to obtain 12.0 g of a solid matter containing 1,5-bis(methacryloyl sulfide)naphthalene (or 1,5-bis(methacryloylthio)naphthalene, 15NDSHMA) (purity by HPLC: 99%, theoretical yield: 70 mol%).

The resulting 15NDSHMA has a melting point of 129 to 130°C and a refractive index of 1.638, and shows the results of ¹H-NMR as follows.
¹H-NMR (CDCl₃): δ (ppm) 2.04 (s, 6H), 5.78 (d, J 1.0, 2H), 6.35 (d, J 1.0, 2H), 7.57 (dd, J 8.0 7.2, 2H), 7.76 (d, J 7.2 0.8, 2H), 8.34 (d, J 8.0 0.8, 2H)

### [Comparative Example 2-2] Synthesis of 26NDSHMA

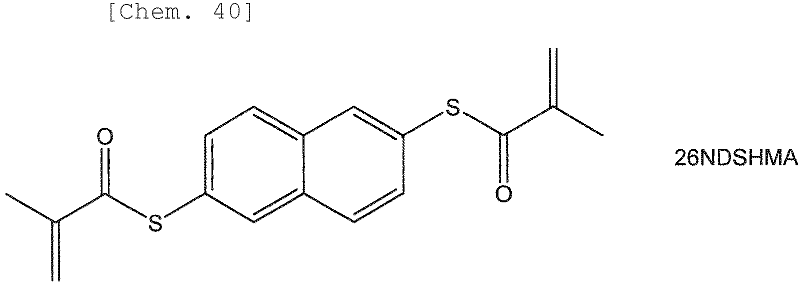

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 26NDSH, and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C, and 2 g of a filter auxiliary agent was added thereto. The resulting mixture was stirred for 5 minutes or more and was subjected to filtration. The residue was washed with 10 g of water to obtain a 26NDSH·Na liquid (an aqueous solution containing a sodium salt of 26NDSH).

To a 500 mL flask, 268 g of methylene chloride, 0.05 g of hydroquinone, and 12.0 g (2.2 molar ratio) of methacryloyl chloride were added under a nitrogen atmosphere and under light shielding, and the mixture was cooled to 5°C. After the cooling, the temperature fluctuated from 0 to 5°C, and the above-mentioned temperature was maintained in a thermostatic bath. Thereafter, the above-mentioned 26NDSH·Na liquid was poured into the flask, and the reaction was carried out at the above-mentioned temperature for one hour. After the completion of the reaction, the reaction mixture was allowed to stand at 0 to 30°C, and then the resulting upper aqueous layer was removed by liquid-liquid separation. The obtained organic layer was subjected to desolvation at not higher than 30°C under a reduced pressure to obtain 67 g of a concentrated liquid. To the concentrated liquid was dropwise added 118 g of isopropyl alcohol at 0 to 30°C over one hour, and then the resulting mixture was stirred for one hour or more. Thereafter, the mixture was subjected to filtration. The resulting wet crystal was washed with 16 g of isopropyl alcohol, and the washed wet crystal was dried to obtain 15.0 g of a solid matter containing 2,6-bis(methacryloyl sulfide)naphthalene (or 2,6-bis(methacryloylthio)naphthalene, 26NDSHMA) (purity by HPLC: 100%, theoretical yield: 87 mol%).

The resulting 26NDSHMA has a melting point of 147 to 148°C and a refractive index of 1.651, and shows the results of ¹H-NMR as follows.
¹H-NMR (CDCl₃): δ (ppm) 2.04 (dd, J 1.6 0.8, 6H), 5.74 (q, J 1.6, 2H), 6.26 (d, J 0.8, 2H), 7.51 (dd, J 8.4 2.0, 2H), 7.86 (d, J 8.4, 2H), 8.00 (d, J 2.0, 2H)

### [Comparative Example 2-3] Synthesis of 27NDSHMA

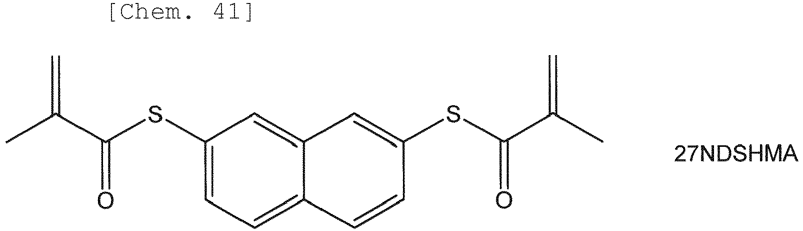

In a 300 mL flask, 241 g of monochlorobenzene was charged under a nitrogen atmosphere and was heated to 60°C. To this liquid was added 10 g (52.0 mmol) of 2,7-naphthalenedithiol (27NDSH), and the mixture was stirred until complete dissolution. After the heating, the temperature fluctuated from 55 to 65°C, and the above-mentioned temperature was maintained in a thermostatic bath. To this solution was dropwise added a mixture of 18.2 g (2.8 molar ratio) of a 32% by mass sodium hydroxide aqueous solution, 40 g of water, and 0.1 g (0.05 molar ratio) of sodium borohydride, and the reaction was carried out at the above-mentioned temperature for one hour. After the completion of the reaction, the resulting upper organic layer was removed by liquid-liquid separation, and the obtained aqueous layer was cooled to 30°C to obtain a 27NDSH·Na liquid (an aqueous solution containing a sodium salt of 27NDSH).

To a 300 mL flask, 156 g of toluene, 0.05 g of hydroquinone, and 12.0 g (2.2 molar ratio) of methacryloyl chloride were added under a nitrogen atmosphere and under light shielding, and the mixture was cooled to 5°C. After the cooling, the temperature fluctuated from 0 to 5°C, and the above-mentioned temperature was maintained in a thermostatic bath. Thereafter, the above-mentioned 27NDSH·Na liquid was poured into the flask, and the reaction was carried out at the above-mentioned temperature for 30 minutes. After the completion of the reaction, the reaction mixture was allowed to stand for 0 to 30°C, and then the resulting lower aqueous layer was removed by liquid-liquid separation. To the obtained organic layer was added 52 g of water, and the resulting mixture was stirred for 20 minutes or more. The resulting lower aqueous layer was removed by liquid-liquid separation. The obtained organic layer was subjected to desolvation at not higher than 30°C under a reduced pressure to obtain 38 g of a concentrated liquid. The concentrated liquid was heated to 40°C. After the heating, the temperature fluctuated from 37 to 42°C. While the concentrated liquid was maintained at the above-mentioned temperature in a thermostatic bath, 23 g of methanol was dropwise added thereto over 15 minutes, and then the resulting mixture was stirred for one hour. Thereafter, the mixture was cooled to 5°C. After the cooling, the temperature fluctuated from 0 to 5°C, and the mixture was stirred for one hour or more while maintained at the above-mentioned temperature in a thermostatic bath. Thereafter, the mixture was subjected to filtration. The resulting wet crystal was washed with 16 g of methanol, and the washed wet crystal was dried to obtain a crude product. To a 1 L flask, 478 g of n-heptane was charged and was heated to 60°C. To this solution was added the resulting crude product, and the resulting mixture was stirred for 10 minutes or more. After the heating, the temperature fluctuated from 60 to 70°C, and the mixture was maintained at the above-mentioned temperature in a thermostatic bath to obtain a n-heptane liquid containing the crude product. In another 1 L flask, 44 g of n-heptane was charged and was cooled to 3°C. After the cooling, the temperature fluctuated from 0 to 10°C, and the above-mentioned temperature was maintained in a thermostatic bath. To the cooled n-heptane was dropwise added the resulting n-heptane liquid containing the crude product over one hour and 30 minutes, and the resulting mixture was stirred for one hour or more and was then subjected to filtration. The resulting wet crystal was dried to obtain 12.2 g of a solid matter containing 2,7-bis(methacryloyl sulfide)naphthalene (or 2,7-bis(methacryloylthio)naphthalene, 27NDSHMA) (purity by HPLC: 99%, theoretical yield: 71 mol%).

The resulting 27NDSHMA has a melting point of 107 to 108°C and a refractive index of 1.649, and shows the results of ¹H-NMR as follows.
¹H-NMR (CDCl₃): δ (ppm) 2.03 (dd, J 1.6 0.8, 6H), 5.74 (q, J 1.6, 2H), 6.25 (d, J 0.8, 2H), 7.52 (dd, J 8.8 1.6, 2H), 7.90 (d, J 8.8, 2H), 7.96 (d, J 1.6, 2H)

The following Table shows the evaluation results of the bis(methacryloylthio)naphthalene compounds obtained in Example 2 and Comparative Examples 2-1 to 2-3. In the Table, MMA means methyl methacrylate (the same applies hereinafter).

### [Table 2]

**Table 2**

| Bis(methacryloylthio) naphthalene compound | | Refractive index | Dissolubility [% by mass] | | | | | | | |
|---|---|---|---|---|---|---|---|---|---|---|
| | | | Methanol | Toluene | THF | DMF | Ethyl acetate | PGMEA | MMA | Styrene |
| Example 2 | 16NDSHMA | 1.647 | 2.49 | 50.7 | 67.6 | 59.5 | 44.6 | 31.1 | 44.1 | 51.1 |
| Comparative Example 2-1 | 15NDSHMA | 1.638 | 0.36 | 6.73 | 18.7 | 13.2 | 4.36 | 3.34 | 5.22 | 10.4 |
| Comparative Example 2-2 | 26NDSHMA | 1.651 | 0.13 | 3.73 | 9.59 | 4.59 | 1.08 | 1.46 | 2.42 | 5.00 |
| Comparative Example 2-3 | 27NDSHMA | 1.649 | 0.20 | 6.84 | 13.1 | 17.5 | 3.20 | 2.68 | 4.36 | 7.92 |

As apparent from the results shown in Table 2, in the same as the results of the naphthalenedithiol compounds, among Comparative Examples, Comparative Example 2-1 (15NDSHMA), which is a 1,5-form, had the lowest refractive index and a high dissolubility in many solvents or copolymerizable components. On the other hand, Example 2 (16NDSHMA) had both high refractive index and high dissolubility compared with Comparative Example 2-1 (15NDSHMA), in particular, had a significantly high dissolubility.

### <Bis(acryloylthio)naphthalene compound>

### [Example 3] Synthesis of 16NDSHA

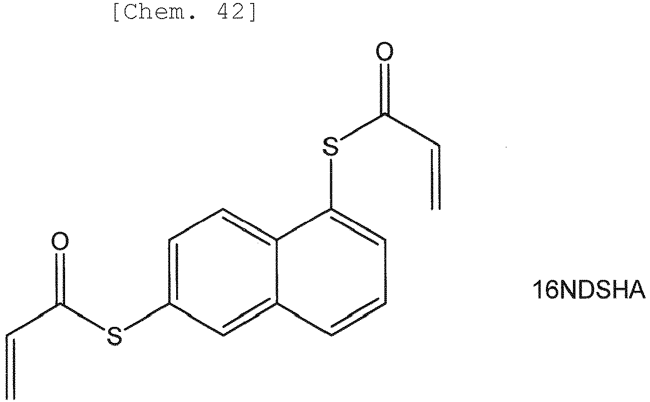

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,6-naphthalenedithiol (16NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C, and 2 g of a filter auxiliary agent was added thereto. The resulting mixture was stirred for 5 minutes or more and was subjected to filtration. The residue was washed with 5 g of water to obtain a 16NDSH·Na liquid (an aqueous solution containing a sodium salt of 16NDSH).

To a 500 mL flask, 268 g of methylene chloride, 0.05 g of methoquinone, 0.05 g of hydroquinone, and 10.4 g (2.2 molar ratio) of acryloyl chloride were added under a nitrogen atmosphere and under light shielding, and the mixture was cooled to 10°C. After the cooling, the temperature fluctuated from 0 to 10°C, and the above-mentioned temperature was maintained in a thermostatic bath. Thereafter, the above-mentioned 16NDSH·Na liquid was dropwise added thereto over 30 minutes, and the reaction was carried out at the above-mentioned temperature for one hour. After the completion of the reaction, the reaction mixture was allowed to stand at 0 to 30°C, and then the resulting upper aqueous layer was removed by liquid-liquid separation. To the obtained organic layer were added 0.05 g of methoquinone and 0.05 g of hydroquinone, and the resulting mixture was subjected to desolvation at not higher than 30°C under a reduced pressure to obtain 34 g of a concentrated liquid. To the resulting concentrated liquid was added and mixed 9 g of n-hexane, and the mixture was subjected to separation-purification by silica gel column chromatography (methylene chloride/n-hexane (volume ratio) = 1/1) to obtain 5.5 g of a solid matter containing 1,6-bis(acryloyl sulfide)naphthalene (or 1,6-bis(acryloylthio)naphthalene, 16NDSHA) (purity by HPLC: 99%, theoretical yield: 35 mol%).

The resulting 16NDSHA has a melting point of 64 to 66°C and a refractive index of 1.667, and shows the results of ¹H-NMR as follows.
¹H-NMR (CDCl₃): δ (ppm) 5.78-5.83 (m, 2H), 6.39-6.56 (m, 4H), 7.52-7.56 (m, 2H), 7.77 (dd, J 10.8 1.2, 1H), 7.94 (d, J 12.6 1H), 8.03 (d, J 3.0, 1H), 8.21 (d, J 12.6, 1H)

### [Comparative Example 3] Synthesis of 15NDSHA

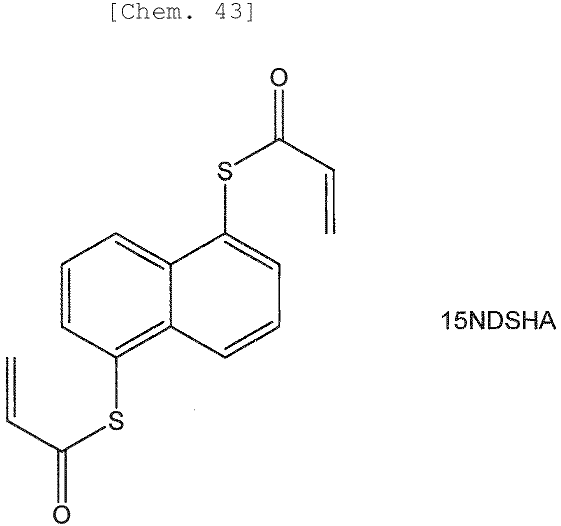

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,5-naphthalenedithiol (15NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C to obtain a 15NDSH·Na liquid (an aqueous solution containing a sodium salt of 15NDSH).

To a 500 mL flask, 100 g of methylene chloride, 0.05 g of methoquinone, and 10.4 g (2.2 molar ratio) of acryloyl chloride were added under a nitrogen atmosphere and under light shielding, and the mixture was cooled to 10°C. After the cooling, the temperature fluctuated from 0 to 10°C, and the above-mentioned temperature was maintained in a thermostatic bath. Thereafter, the above-mentioned 15NDSH·Na liquid was dropwise added thereto over 30 minutes, and the reaction was carried out at the above-mentioned temperature for one hour. After the completion of the reaction, the reaction mixture was allowed to stand at 0 to 30°C, and then the resulting upper aqueous layer was removed by liquid-liquid separation. To the obtained organic layer was added 0.05 g of methoquinone, and the resulting mixture was subjected to desolvation at not higher than 30°C under a reduced pressure to obtain 25 g of a concentrated liquid. The resulting concentrated liquid was subjected to separation-purification by silica gel column chromatography (methylene chloride/n-heptane (volume ratio) = 2/3) to obtain 2.8 g of a solid matter containing 1,5-bis(acryloyl sulfide)naphthalene (or 1,5-bis(acryloylthio)naphthalene, 15NDSHA) (purity by HPLC: 99%, theoretical yield: 18 mol%).

The resulting 15NDSHA has a melting point of 156 to 158°C and a refractive index of 1.646, and shows the results of ¹H-NMR as follows.

¹H-NMR (CDCl₃): δ (ppm) 5.83 (d, J 9.6 1.2, 2H), 6.43-6.58 (m, 4H), 7.59 (dd, J 8.8 7.6, 2H), 7.79 (d, J 7.6, 2H), 8.35 (d, J 8.8, 2H)

The following Table shows the evaluation results of the bis(acryloylthio)naphthalene compounds obtained in Example 3 and Comparative Example 3.

### [Table 3]

**Table 3**

| Bis(acryloylthio) naphthalene compound | | Refractive index | Dissolubility [% by mass] | | | | |
|---|---|---|---|---|---|---|---|
| | | | Toluene | Ethyl acetate | PGMEA | MMA | Styrene |
| Example 3 | 16NDSHA | 1.667 | 58.4 | 56.3 | 44.3 | 55.7 | 59.4 |
| Comparative Example 3 | 15NDSHA | 1.646 | 2.61 | 1.57 | 1.50 | 1.93 | 4.24 |

As apparent from the results shown in Table 3, the bis(acryloylthio)naphthalene compounds also showed the same results as the aforementioned examples, specifically Example 3 (16NDSHA) had high refractive index and high dissolubility compared with Comparative Example 3 (15NDSHA), in particular, had a significantly high dissolubility.

### <Bis(allylthio)naphthalene compound>

### [Example 4] Synthesis of 16DANDSH

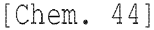

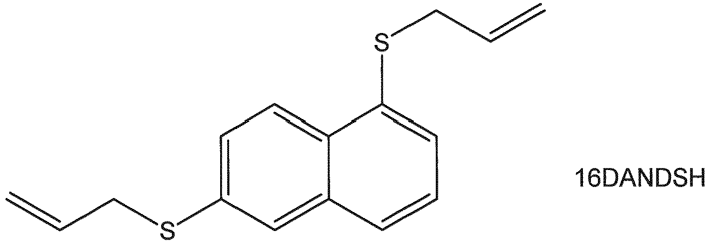

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,6-naphthalenedithiol (16NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C to obtain a 16NDSH·Na liquid (an aqueous solution containing a sodium salt of 16NDSH).

To a 200 mL flask, 99 g of methylene chloride, 0.05 g of methoquinone, and 8.8 g (2.2 molar ratio) of allyl chloride were added under a nitrogen atmosphere, and the mixture was cooled to 5°C. After the cooling, the temperature fluctuated from 0 to 5°C, and the above-temperature was maintained in a thermostatic bath. Thereafter, the above-mentioned 16NDSH·Na liquid was poured into the flask, and the reaction was carried out at the above-mentioned temperature for 18 hours. After the completion of the reaction, the reaction mixture was allowed to stand at 0 to 30°C, and then the resulting upper aqueous layer was removed by liquid-liquid separation. Thereafter, to the obtained organic layer was added 52 g of water, and the mixture was stirred for 10 minutes or more. The resulting upper aqueous layer was removed by liquid-liquid separation. The obtained organic layer was subjected to desolvation at not higher than 40°C under a reduced pressure to obtain 12 g of a concentrated liquid. The resulting concentrated liquid was subjected to separation-purification by silica gel column chromatography (methylene chloride/n-heptane (volume ratio) = 1/9) to obtain 10.2 g of a liquid matter containing 1,6-bis(allyl sulfide)naphthalene (or 1,6-bis(allylthio)naphthalene, 16DANDSH) (purity by HPLC: 100%, theoretical yield: 72 mol%).

The resulting 16DANDSH has a refractive index of 1.667 and shows the results of ¹H-NMR as follows.
¹H-NMR (CDCl₃): δ (ppm) 3.59 (d, J 7.2, 2H), 3.66 (d, J 7.2, 2H), 5.01-5.22 (m, 4H), 5.84-5.98 (m, 2H), 7.38 (t, J 8.4, 1H), 7.47-7.52 (m, 2H), 7.62 (d, J 8.4, 1H), 7.74 (d, J 2.0, 1H), 8.32 (d, J 9.2, 1H)

### [Comparative Example 4] Synthesis of 15DANDSH

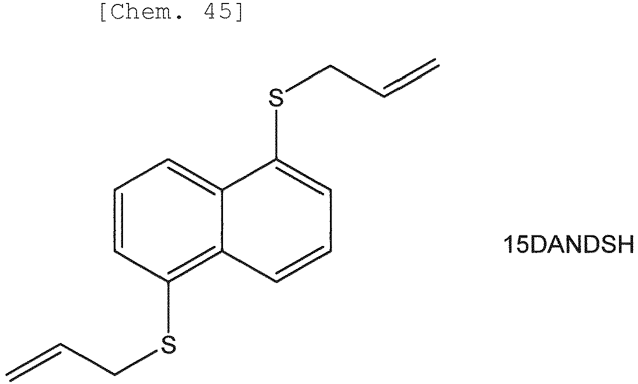

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,5-naphthalenedithiol (15NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C to obtain a 15NDSH·Na liquid (an aqueous solution containing a sodium salt of 15NDSH).

To a 300 mL flask, 268 g of methylene chloride, 0.05 g of methoquinone, and 10.3 g (2.6 molar ratio) of allyl chloride were added under a nitrogen atmosphere, and the above-mentioned 15NDSH·Na liquid was poured into the flask at 0 to 30°C. Thereafter, the mixture was heated to 40°C. After the heating, the temperature fluctuated from 35 to 40°C, and the reaction was carried out for 3 hours while maintaining the temperature in a thermostatic bath. After the completion of the reaction, the reaction mixture was cooled to 30°C and was allowed to stand at 10 to 30°C, and then the resulting upper aqueous layer was removed by liquid-liquid separation. Thereafter, to the obtained organic layer was added 76 g of water, and the mixture was stirred for 10 minutes or more. The resulting upper aqueous layer was removed by liquid-liquid separation. The obtained organic layer was subjected to desolvation at not higher than 30°C under a reduced pressure to obtain 11 g of a concentrated liquid. The resulting concentrated liquid was subjected to separation-purification by silica gel column chromatography (methylene chloride/n-hexane (volume ratio) = 1/8) to obtain 7.4 g of a solid matter containing 1,5-bis(allyl sulfide)naphthalene (or 1,5-bis(allylthio)naphthalene, 15DANDSH) (purity by HPLC: 100%, theoretical yield: 52 mol%).

The resulting 15DANDSH has a melting point of 78 to 79°C and a refractive index of 1.653, and shows the results of ¹H-NMR as follows.
¹H-NMR (CDCl₃): δ (ppm) 3.59-3.61 (m, 4H), 5.01-5.07 (m, 4H), 5.84-5.94 (m, 2H), 7.47 (dd, J 8.8 7.2, 2H), 7.60 (d, J 7.2, 2H), 8.37 (d, J 8.8, 2H)

The following Table shows the evaluation results of the bis(allylthio)naphthalene compounds obtained in Example 4 and Comparative Example 4.

### [Table 4]

**Table 4**

| Bis(allylthio) naphthalene compound | | Refractive index | Dissolubilty [% by mass] | | | | |
|---|---|---|---|---|---|---|---|
| | | | Toluene | Ethyl acetate | PGMEA | MMA | Styrene |
| Example 4 | 16DANDSH | 1.667 | | Freely blended (>90) | | | |
| Comparative Example 4 | 15DANDSH | 1.653 | 26.1 | 16.4 | 13.7 | 26.6 | 10.6 |

As apparent from the results shown in Table 4, the bis(allylthio)naphthalene compounds also showed the same results as the aforementioned examples, specifically Example 4 (16DANDSH) had high refractive index and high dissolubility compared with Comparative Example 4 (15DANDSH), in particular, had a significantly high dissolubility.

### <Bis(vinylthio)naphthalene compound>

### [Example 5] Synthesis of 16DVNDSH

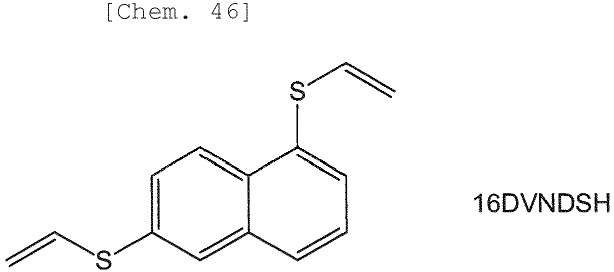

In accordance with Example 7-2 mentioned below, a concentrated liquid containing 1,6-bis(2-hydroxyethylthio)naphthalene (16DHENDSH) was prepared [specifically, the concentrated liquid was obtained as follows: allowing a 16NDSH·Na liquid to react with 2-chloroethanol, adding tetrahydrofuran (THF) and 36% by mass hydrochloric acid to the reaction mixture, removing the resulting aqueous layer by liquid-liquid separation, and desolvating the obtained organic layer (a THF solution of 16DHENDSH) under a reduced pressure to obtain the concentrated liquid].

To a 100 mL flask, the above-mentioned concentrated liquid containing 10.0 g (35.7 mmol) of 16DHENDSH, and 41.5 g of toluene were added under a nitrogen atmosphere, and the mixture was heated to 70°C. After the heating, 9.8 g (2.3 molar ratio) of thionyl chloride was dropwise added thereto at 70°C over 30 minutes. After the completion of the dropwise addition, the reaction was carried out at 70°C for 17 hours. After the completion of the reaction, the reaction mixture was cooled to not higher than 30°C, and 21.4 g (1.5 molar ratio) of a 10% by mass sodium hydroxide aqueous solution was added thereto. The resulting mixture was subjected to liquid-liquid separation. To the obtained organic layer was added another 13 g of water, and the resulting mixture was stirred for 10 minutes or more, and the resulting lower aqueous layer was removed by liquid-liquid separation, thus obtaining 52.3 g of an organic layer containing 1,6-bis(2-chloroethylthio)naphthalene (35.3 mmol).

Under a nitrogen atmosphere, 52.3 g of the organic layer containing 1,6-bis(2-chloroethylthio)naphthalene (35.3 mmol) obtained in the preceding paragraph, 0.6 g (0.05 molar ratio) of tetrabutylammonium bromide, and 8.2 g (2.8 molar ratio) of a 48% by mass sodium hydroxide aqueous solution were charged, and the mixture was heated to 75°C. After the heating, the temperature fluctuated from 75 to 85°C, and the reaction was carried out for 15 hours while maintaining the above-temperature in a thermostatic bath. After the completion of the reaction, the reaction mixture was cooled to 30°C, and 29 g of water and 2.9 g (0.8 molar ratio) of 36% by mass hydrochloric acid were dropwise added thereto, and then the resulting mixture was subjected to liquid-liquid separation to obtain a separated upper organic layer. The obtained organic layer was further washed with 15 g of water, and the washed organic layer was desolvated to obtain 8.0 g of a liquid matter containing 1,6-bis(vinylthio)naphthalene (16DVNDSH) (purity by HPLC: 100%, theoretical yield: 89 mol%).

The resulting 16DVNDSH has a refractive index of 1.699 and shows the results of ¹H-NMR as follows.
¹H-NMR (CDCl₃): δ (ppm) 5.13 (d, J 16.4, 1H), 5.31 (d, J 9.6, 1H), 5.43-5.48 (m, 2H), 6.51 (dd, J 16.8 9.6, 1H), 6.64 (dd, J 16.8 9.6, 1H), 7.45 (dd, J 8.4 7.2, 1H), 7.52 (dd, J 9.2 2.0, 1H), 7.65 (dd, J 7.2 2.0, 1H), 7.74 (d, J 8.4, 1H), 7.83 (d, J 2.0, 1H), 8.27 (d, J 9.2, 1H)

### [Comparative Example 5] Synthesis of 15DVNDSH

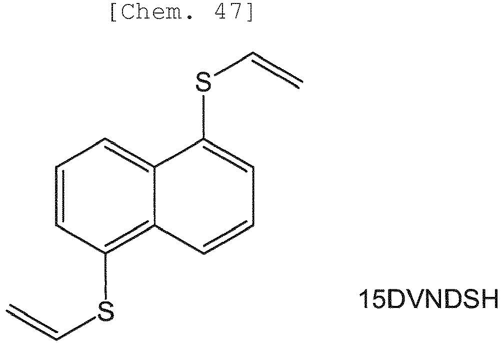

To a 100 mL flask, 10.0 g (35.7 mmol) of 1,5-bis(2-hydroxyethylthio)naphthalene (15DHENDSH obtained in Comparative Example 7 mentioned below) and 50 g of toluene were added under a nitrogen atmosphere, and the mixture was heated to 70°C. After the heating, 8.9 g (2.1 molar ratio) of thionyl chloride was dropwise added thereto at 70°C over 2 hours. After the completion of the dropwise addition, the reaction was carried out at 70°C for 2 hours. After the completion of the reaction, 21.4 g (1.5 molar ratio) of a 10% by mass sodium hydroxide aqueous solution was added thereto, and the resulting mixture was subjected to liquid-liquid separation at 70°C. The organic layer obtained by liquid-liquid separation was cooled to 10°C and was subjected to filtration. The resulting wet crystal was washed with 20 g of n-heptane, and the washed wet crystal was dried to obtain 10.5 g (0.0332 mol) of a solid matter containing 1,5-bis(2-chloroethylthio)naphthalene.

In a 100 mL flask, 10.5 g (33.2 mmol) of 1,5-bis(2-chloroethylthio)naphthalene, 25 g of n-heptane, 0.5 g (0.05 molar ratio) of tetrabutylammonium bromide, and 7.7 g (2.8 molar ratio) of a 48% by mass sodium hydroxide aqueous solution were charged under a nitrogen atmosphere, and the mixture was heated to 75°C. After the heating, the temperature fluctuated from 75 to 85°C, and the reaction was carried out for 5.5 hours while maintaining the above-mentioned temperature in a thermostatic bath. After the completion of the reaction, the reaction mixture was cooled to 60°C, and 15 g of water was added thereto, and then the resulting mixture was subjected to liquid-liquid separation at 60°C to obtain a separated upper organic layer. The obtained organic layer was further washed with 10 g of water twice, and the washed organic layer was desolvated to obtain 7.9 g of a solid matter containing 1,5-bis(vinylthio)naphthalene (15DVNDSH) (purity by HPLC: 98%, theoretical yield: 91 mol%).

The resulting 15DVNDSH has a melting point of 50 to 52°C and a refractive index of 1.685, and shows the results of ¹H-NMR as follows.
¹H-NMR (CDCl₃): δ (ppm) 5.15 (d, J 16.4, 2H), 5.33 (d, J 9.6, 2H), 6.52 (dd, J 16.4 9.6, 2H), 7.52 (dd, J 8.8 6.8, 2H), 7.72 (d, J 6.8, 2H), 8.36 (d, J 8.8, 2H)

The following Table shows the evaluation results of the bis(vinylthio)naphthalene compounds obtained in Example 5 and Comparative Example 5.

### [Table 5]

**Table 5**

| Bis(vinylthio) naphthalene compound | | Refractive index | Dissolubility [% hv mass] | | | | |
|---|---|---|---|---|---|---|---|
| | | | Toluene | Ethyl acetate | PGMEA | MMA | Styrene |
| Example 5 | 16DVNDSH | 1.699 | Freely blended (>90) | | | | |
| Comparative Example 5 | 15DVNDSH | 1.685 | 59.6 | 39.0 | 26.3 | 43.6 | 45.8 |

As apparent from the results shown in Table 5, the bis(vinylthio)naphthalene compounds also showed the same results as the aforementioned examples, specifically Example 5 (16DVNDSH) had high refractive index and high dissolubility compared with Comparative Example 5 (15DVNDSH), in particular, had a significantly high dissolubility.

### <Bis(glycidylthio)naphthalene compound>

### [Example 6] Synthesis of 16DGNDSH

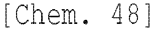

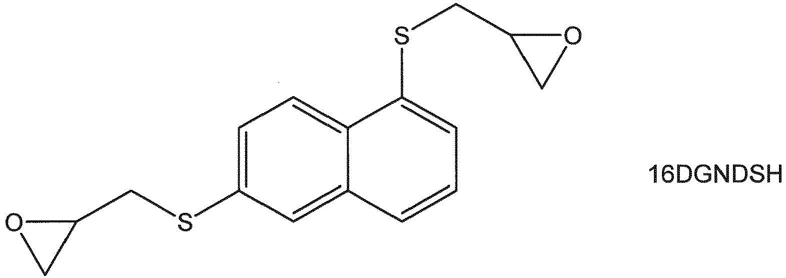

In a 300 mL flask, 10 g (52.0 mmol) of 1,6-naphthalenedithiol (16NDSH), 168.4 g (35 molar ratio) of epichlorohydrin, and 15.9 g (0.5 molar ratio) of a 51% by mass benzyltributylammonium chloride aqueous solution were charged under a nitrogen atmosphere, and the mixture was heated to 75°C. After the heating, the temperature fluctuated from 75 to 85°C, and the reaction was carried out for 2 hours while maintaining the above-mentioned temperature in a thermostatic bath. After the completion of the reaction, the reaction mixture was cooled to 40°C, and 26 g of water and 19.9 g (3.06 molar ratio) of a 32% by mass sodium hydroxide aqueous solution were added thereto. The resulting mixture was stirred for 30 minutes or more, and the resulting lower aqueous layer was separated by liquid-liquid separation. The obtained organic layer was desolvated at not higher than 30°C under a reduced pressure to obtain a concentrated liquid. To the concentrated liquid were added 84 g of ethyl acetate and 42 g of water, and the resulting mixture was stirred for 10 minutes or more, and the resulting lower aqueous layer was removed by liquid-liquid separation. To the obtained organic layer was added 42 g of water in the same manner, the resulting mixture was stirred for 10 minutes or more, and the resulting lower layer was separated by liquid-liquid separation. The obtained organic layer was desolvated at not higher than 30°C under a reduced pressure to obtain 16.0 g of a liquid matter containing 1,6-bis(glycidylthio)naphthalene (16DGNDSH) (purity by HPLC: 94%, theoretical yield: 86 mol%).

The resulting 16DGNDSH has a refractive index of 1.665 and shows the results of ¹H-NMR as follows.
¹H-NMR (CDCl₃): δ (ppm) 2.40 (q, J 2.4, 1H), 2.59 (q, J 2.4, 1H), 2.70 (t, J 4.2, 1H), 2.80 (t, J 4.2, 1H), 2.97 (dd, J 13.8 6.0, 1H), 3.09 (dd, J 13.8 6.0, 1H), 3.10-3.30 (m, 4H), 7.42 (dd, J 7.8 7.2, 1H), 7.57 (dd, J 9.0 1.8, 1H), 7.68 (d, J 7.2, 1H), 7.70 (d, J 7.8, 1H), 7.86 (d, J 1.8, 1H), 8.39 (d, J 9.0, 1H)

### [Comparative Example 6] Synthesis of 15DGNDSH

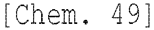

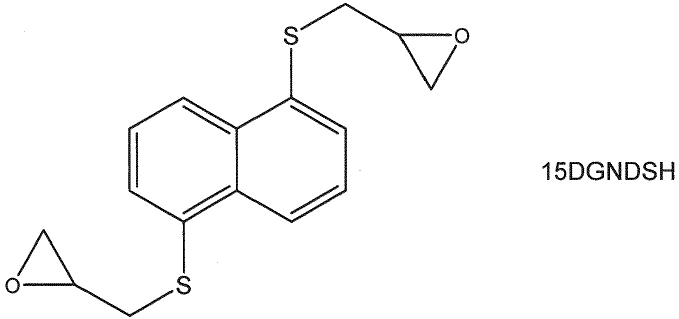

In a 300 mL flask, 10 g (52.0 mmol) of 1,5-naphthalenedithiol (15NDSH), 168.4 g (35 molar ratio) of epichlorohydrin, and 15.9 g (0.5 molar ratio) of a 51% by mass benzyltributylammonium chloride aqueous solution were charged under a nitrogen atmosphere, and the mixture was heated to 75°C. After the heating, the temperature fluctuated from 75 to 85°C, and the reaction was carried out for one hour while maintaining the above-mentioned temperature in a thermostatic bath. After the completion of the reaction, the reaction mixture was cooled to 30°C, and 26 g of water and 19.9 g (3.06 molar ratio) of a 32% by mass sodium hydroxide aqueous solution were added thereto. The resulting mixture was stirred for 30 minutes or more, and the resulting lower aqueous layer was separated by liquid-liquid separation. The obtained organic layer was desolvated at not higher than 30°C under a reduced pressure to obtain a concentrated liquid. To the concentrated liquid were added 84 g of ethyl acetate and 42 g of water, and the resulting mixture was stirred for 10 minutes or more, and the resulting lower aqueous layer was removed by liquid-liquid separation. To the obtained organic layer was added 42 g of water in the same manner, the resulting mixture was stirred for 10 minutes or more, and the resulting lower layer was separated by liquid-liquid separation. The obtained organic layer was desolvated at not higher than 30°C under a reduced pressure, and then 69 g of n-heptane was added thereto. The resulting mixture was stirred for 10 minutes or more, and then was subjected to filtration. The resulting wet crystal was washed with 24 g of n-heptane, and the washed wet crystal was dried to obtain 13.1 g of a solid matter containing 1,5-bis(glycidylthio)naphthalene (15DGNDSH) (purity by HPLC: 97%, theoretical yield: 81 mol%).

The resulting 15DGNDSH has a melting point of 50 to 53°C and a refractive index of 1.621, and shows the results of ¹H-NMR as follows.
¹H-NMR (CDCl₃): δ (ppm) 2.41 (dd, J 4.8 2.4, 2H), 2.71 (t, J 4.8, 2H), 2.93-3.03 (m, 2H), 3.14-3.22 (m, 4H), 7.51 (dd, J 8.8 7.2, 2H), 7.77 (dd, J 7.2, 2H), 8.47 (d, J 8.8, 2H)

The following Table shows the evaluation results of the bis(glycidylthio)naphthalene compounds obtained in Example 6 and Comparative Example 6.

### [Table 6]

**Table 6**

| Bis(glycidylthio) naphthalene compound | | Refractive index | Dissolubility [% by mass] | | | | |
|---|---|---|---|---|---|---|---|
| | | | Toluene | Ethyl acetate | PGMEA | MMA | Styrene |
| Example 6 | 16DGNDSH | 1.665 | Freely blended (>90) | | | | |
| Comparative Example 6 | 15DGNDSH | 1.621 | 56.8 | 51.9 | 62.3 | 60.9 | 43.6 |

As apparent from the results shown in Table 6, for the bis(glycidylthio)naphthalene compounds, Example 6 (16DGNDSH) had significantly high refractive index and dissolubility compared with Comparative Example 6 (15DGNDSH).

### <Bis(2-hydroxyethylthio)naphthalene compound>

### [Example 7] Synthesis of 16DHENDSH

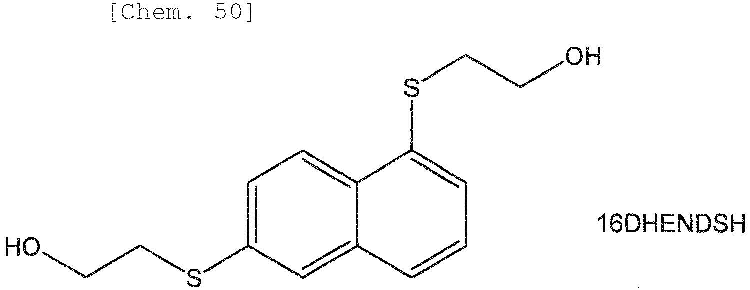

### (Example 7-1)

In a 500 mL flask, 10 g (52.0 mmol) of 1,6-naphthalenedithiol (16NDSH), 80 g of N,N-dimethylformamide, and 11.9 g (2.6 molar ratio) of ethylene carbonate were charged under a nitrogen atmosphere, and the mixture was heated to 100°C. After the heating, the temperature fluctuated from 97 to 100°C, and the reaction was carried out for one hour while maintaining the above-mentioned temperature in a thermostatic bath. After the completion of the reaction, the reaction mixture was desolvated at not higher than 95°C under a reduced pressure to obtain a concentrated liquid. To the obtained concentrated liquid were added 66 g of ethyl acetate and 61.2 g (1.0 molar ratio) of a 3.4% by mass sodium hydroxide aqueous solution, and the resulting mixture was stirred for 10 minutes or more. The resulting lower aqueous layer was removed by liquid-liquid separation. To the obtained organic layer was added 7.5 g of water in the same manner, the resulting mixture was stirred for 10 minutes or more, and the resulting lower layer was removed by liquid-liquid separation. To the obtained organic layer was added 7.5 g of water in the same manner, the resulting mixture was stirred for 10 minutes or more, the resulting lower layer was removed by liquid-liquid separation, thus obtaining an organic layer. At not higher than 70°C under a reduced pressure, the organic layer was subjected to desolvation and drying, and 14.4 g of a liquid matter containing 1,6-bis(2-hydroxyethylthio)naphthalene (16DHENDSH) was obtained (purity by HPLC: 97%, theoretical yield: 95 mol%).

The resulting 16DHENDSH has a melting point of 43 to 44°C and a refractive index of 1.672, and shows the results of ¹H-NMR as follows.
¹H-NMR (CDCl₃): δ (ppm) 2.12 (t, J 6.0, 1H), 2.16 (t, 6.0, 1H), 3.14 (t, J 6.0, 2H), 3.23 (t, J 6.0, 2H), 3.72 (q, J 6.0, 2H), 3.81 (q, J 6.0, 2H), 7.41 (dd, J 8.4 7.2, 1H), 7.54 (dd, J 9.0 1.8, 1H), 7.60 (d, J 7.2, 1H), 7.67 (d, J 8.4, 1H), 7.81 (d, J 1.8, 1H), 8.37 (d, J 9.0, 1H)

### (Example 7-2)

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,6-naphthalenedithiol (16NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C to obtain a 16NDSH·Na liquid (an aqueous solution containing a sodium salt of 16NDSH).

Under a nitrogen atmosphere, to the 16NDSH·Na liquid obtained above was dropwise added 8.8 g (2.1 molar ratio) of 2-chloroethanol at a room temperature (about 25°C) over one hour. The temperature after the dropwise addition was a room temperature (about 25°C), and the resulting mixture was stirred for one hour while maintained at the above-mentioned temperature in a thermostatic bath. After the completion of the reaction, 26 g of tetrahydrofuran (THF) was added thereto, and the resulting mixture was stirred for 10 minutes or more. The resulting lower aqueous layer was removed by liquid-liquid separation. To the obtained organic layer was added 12.5 g of water in the same manner, and to the resulting mixture was dropwise added 0.045 g (0.0085 molar ratio) of a 36% by mass hydrochloric acid. After the dropwise addition, the resulting mixture was stirred for 10 minutes or more, and the resulting lower layer was removed by liquid-liquid separation, thus obtaining an organic layer. At not higher than 100°C under a reduced pressure, the organic layer was subjected to desolvation and drying, and 14.5 g of a concentrated liquid was obtained.

In a 50 mL flask, 16.4 g of toluene was charged under a nitrogen atmosphere and was cooled to not higher than 5°C, and then a solution containing the concentrated liquid obtained in the preceding paragraph and 5.5 g of ethyl acetate was dropwise added thereto over one hour or more. After the dropwise addition, the resulting mixture was stirred for 30 minutes or more and was subjected to filtration. The resulting wet crystal was washed with 10.8 g of chlorotoluene that was pre-cooled to 0 to 5°C, and the washed wet crystal was dried to obtain 12.4 g of a solid matter containing 1,6-bis(2-hydroxyethylthio)naphthalene (16DHENDSH) (purity by HPLC: 100%, theoretical yield: 85 mol%). The results of the melting point, refractive index, ¹H-NMR, and dissolubility of the obtained 16DHENDSH were the same as those of Example 7-1.

### [Comparative Example 7] Synthesis of 15DHENDSH

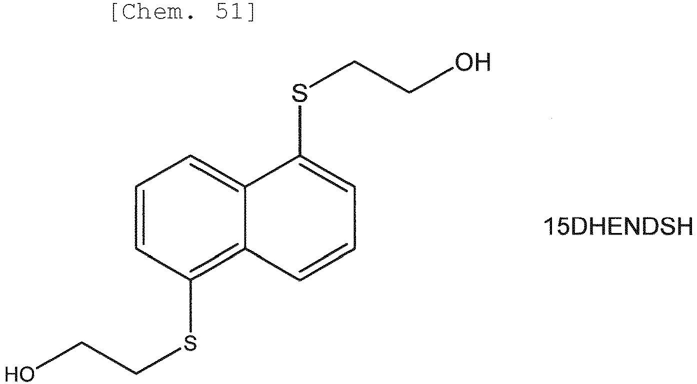

In a 200 mL flask, 10 g (52.0 mmol) of 1,5-naphthalenedithiol (15NDSH), 100 g of N,N-dimethylformamide (DMF), and 13.1 g (2.8 molar ratio) of ethylene carbonate were charged under a nitrogen atmosphere, and the mixture was heated to 100°C. After the heating, the temperature fluctuated from 97 to 100°C, and the reaction was carried out for 17 hours while maintaining the above-mentioned temperature in a thermostatic bath. After the completion of the reaction, the reaction mixture was cooled to 30°C, and 91 g of water added thereto at 0 to 30°C, and the resulting mixture was stirred for 30 minutes or more and was then subjected to filtration. The resulting filtrate was cooled to 10°C. After the cooling, the temperature fluctuated from 0 to 10°C. While the filtrate was maintained at the above-mentioned temperature in a thermostatic bath, 194 g of water was poured into the flask. The resulting mixture was stirred for one hour and 30 minutes or more and was then subjected to filtration. The resulting wet crystal was washed with 62 g of water, and the washed wet crystal was dried to obtain 11.1 g of a solid matter containing 1,5-bis(2-hydroxyethylthio)naphthalene (15DHENDSH) (purity by HPLC: 97%, theoretical yield: 74 mol%).

The resulting 15DHENDSH has a melting point of 84 to 86°C and a refractive index of 1.668, and shows the results of ¹H-NMR as follows.
¹H-NMR (CDCl₃): δ (ppm) 2.03 (t, J 6.0, 2H), 3.16 (t, J 6.0, 4H), 3.73 (q, J 6.0, 4H), 7.50 (dd, J 8.8 7.2, 2H), 7.70 (d, J 7.2, 2H), 8.44 (d, J 8.8, 2H)

The following Table shows the evaluation results of the bis(2-hydroxyethylthio)naphthalene compounds obtained in Example 7 and Comparative Example 7.

### [Table 7]

**Table 7**

| Bis(2-hydroxyethylthio) naphthalene compound | | Refractive index | Dissolubility [% by mass] | | | | |
|---|---|---|---|---|---|---|---|
| | | | Toluene | Ethyl acetate | PGMEA | MMA | Styrene |
| Example 7 | 16DHENDSH | 1.672 | 1.69 | Freely blended (>90) | | | 2.87 |
| Comparative Example 7 | 15DHENDSH | 1.668 | 0.19 | 3.01 | 3.60 | 34.7 | 0.35 |

As apparent from the results shown in Table 7, the bis(2-hydroxyethylthio)naphthalene compounds also showed the same results as the aforementioned examples, specifically, Example 7 (16DHENDSH) had high refractive index and high dissolubility compared with Comparative Example 7 (15DHENDSH), in particular, had a significantly high dissolubility.

### <Bis(methylthio)naphthalene compound>

### [Example 8] Synthesis of 16DMNDSH

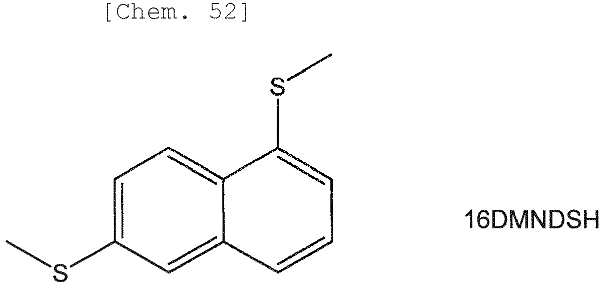

### (Example 8-1)

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,6-naphthalenedithiol (16NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C to obtain a 16NDSH▪Na liquid (an aqueous solution containing a sodium salt of 16NDSH).

To a 200 mL flask, 99 g of methylene chloride, 0.05 g of methoquinone, and 16.2 g (2.2 molar ratio) of methyl iodide were added under a nitrogen atmosphere, and the mixture was cooled to 5°C. Thereafter, the above-mentioned 16NDSH▪Na liquid was poured into the flask, and then 10 g of water was added thereto. The reaction was carried out at the above-mentioned temperature for 20 hours. After the completion of the reaction, the reaction mixture was allowed to stand at 0 to 30°C, and then the resulting upper aqueous layer was removed by liquid-liquid separation. Thereafter, to the obtained organic layer was added 52 g of water, and the mixture was stirred for 10 minutes or more. The resulting upper aqueous layer was removed by liquid-liquid separation. The obtained organic layer was subjected to desolvation at not higher than 60°C under a reduced pressure to obtain 11 g of a concentrated liquid. The resulting concentrated liquid was subjected to separation and purification by silica gel column chromatography (methylene chloride/n-heptane (volume ratio) = 1/8) to obtain 10.5 g of a solid matter containing 1,6-bis(methylthio)naphthalene (16DMNDSH) (purity by HPLC: 100%, theoretical yield: 91 mol%).

The resulting 16DMNDSH has a melting point of 52 to 53°C and a refractive index of 1.690, and shows the results of ¹H-NMR as follows.
¹H-NMR (CDCl₃): δ (ppm) 2.56 (s, 3H), 2.58 (s, 3H), 7.30 (dd, J 7.2 1.2, 1H), 7.37-7.43 (m, 2H), 7.54-7.58 (m, 2H), 8.17 (d, J 8.8, 1H)

### (Example 8-2)

In the same manner as Example 8-1, 11 g of a concentrated liquid (a concentrated liquid before the separation-purification by silica gel column chromatography in Example 8-1) was obtained. The concentrated liquid was distilled at an internal temperature of 175 to 190°C and a pressure of 1.8 to 2.1 hPa to obtain 9.4 g of 16DMNDSH as a distillate fraction distillated at a column top temperature (a top temperature) of 170 to 180°C (purity by HPLC: 100%, theoretical yield: 94 mol%). The results of the melting point, refractive index, ¹H-NMR, and dissolubility of the obtained 16DMNDSH were the same as those of Example 8-1.

### [Comparative Example 8] Synthesis of 15DMNDSH

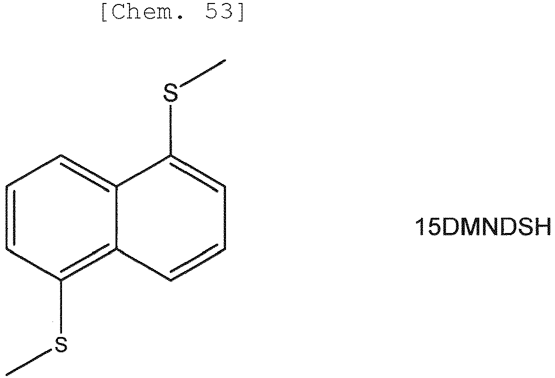

In a 100 mL flask, 18.2 g (2.8 molar ratio) of a 32% by mass sodium hydroxide aqueous solution, 40 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,5-naphthalenedithiol (15NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C to obtain a 15NDSH▪Na liquid (an aqueous solution containing a sodium salt of 15NDSH).

To a 200 mL flask, 99 g of methylene chloride, 0.05 g of methoquinone, and 16.2 g (2.2 molar ratio) of methyl iodide were added under a nitrogen atmosphere, and the mixture was cooled to 5°C. Thereafter, the above-mentioned 15NDSH▪Na liquid was poured into the flask, and then 10 g of water was added thereto. The reaction was carried out at the above-mentioned temperature for 20 hours. After the completion of the reaction, to the reaction mixture was added 83 g of methylene chloride, and the resulting mixture was allowed to stand at 0 to 30°C, and then the resulting upper aqueous layer was removed by liquid-liquid separation. Thereafter, to the obtained organic layer was added 52 g of water, and the mixture was stirred for 10 minutes or more. The resulting upper aqueous layer was removed by liquid-liquid separation. The obtained organic layer was subjected to desolvation at not higher than 40°C under a reduced pressure to obtain 67 g of a concentrated liquid. The concentrated liquid was cooled to 30°C. After the cooling, the temperature fluctuated from 20 to 30°C. The concentrated liquid was stirred for one hour or more while maintained at the above-mentioned temperature in a thermostatic bath, and then the concentrated liquid was subjected to filtration. The resulting wet crystal was washed with 10 g of methylene chloride that was pre-cooled to 0 to 5°C, and the washed wet crystal was dried to obtain 8.6 g of a solid matter containing 1,5-bis(methylthio)naphthalene (15DMNDSH) (purity by HPLC: 100%, theoretical yield: 75 mol%).

The resulting 15DMNDSH has a melting point of 149 to 151°C and a refractive index of 1.690, and shows the results of ¹H-NMR as follows.
¹H-NMR (CDCl₃): δ (ppm) 2.57 (s, 6H), 7.40-7.49 (m, 4H), 8.14 (d, J 8.8, 2H)

The following Table shows the evaluation results of the bis(methylthio)naphthalene compounds obtained in Example 8 and Comparative Example 8.

### [Table 8]

**Table 8**

| Bis(methylthio) naphthalene compound | | Refractive index | Dissolubility [% by mass] | | | | |
|---|---|---|---|---|---|---|---|
| | | | Toluene | Ethyl acetate | PGMEA | MMA | Styrene |
| Example 8 | 16DMNDSH | 1.690 | Freely blended (>90) | | 42.3 | 62.3 | 68.7 |
| Comparative Example 8 | 15DMNDSH | 1.690 | 2.36 | 1.25 | 1.25 | 1.44 | 3.13 |

As apparent from the results shown in Table 8, for the bis(methylthio)naphthalene compounds, Example 8 (16DMNDSH) had significantly high dissolubility compared with Comparative Example 8 (15DMNDSH). Moreover, both compounds had a high refractive index.

### <Bis(carboxymethylthio)naphthalene compound>

### [Example 9] Synthesis of 16DCNDSH

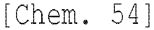

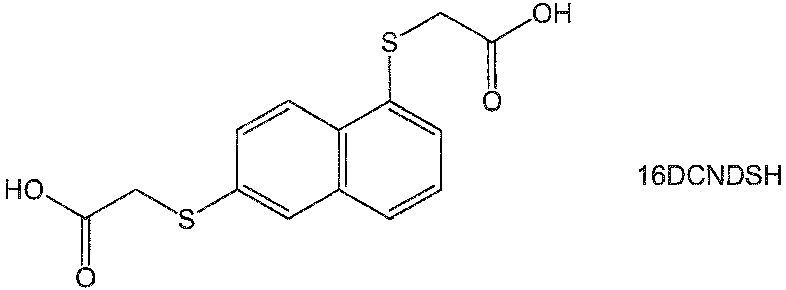

In a 100 mL flask, 39.0 g (6.0 molar ratio) of a 32% by mass sodium hydroxide aqueous solution, 85.8 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,6-naphthalenedithiol (16NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C to obtain a 16NDSH▪Na liquid (an aqueous solution containing a sodium salt of 16NDSH).

To a 200 mL flask, 15.3 g (2.4 molar ratio) of chloroethyl acetate was added under a nitrogen atmosphere, the above-mentioned 16NDSH▪Na liquid was dropwise added thereto over 30 minutes, and the resulting mixture was stirred for one hour and was then heated to 75°C. After the heating, the temperature fluctuated from 75 to 80°C, and the reaction was carried out for one hour while maintaining the temperature in a thermostatic bath. After the completion of the reaction, the reaction mixture was cooled to 30°C, and 20 g of toluene was added thereto. The resulting mixture was stirred for 10 minutes or more. Thereafter, the mixture was allowed to stand at 0 to 30°C, and the resulting upper organic layer was removed by liquid-liquid separation. Then, to the obtained aqueous layer was added 40 g of toluene, and 21.6 g (4.1 molar ratio) of 36% by mass hydrochloric acid was dropwise added thereto. The resulting mixture was stirred for 30 minutes or more and was then subjected to filtration. The resulting wet crystal was washed with 20 g of water to obtain 24.6 g of a crude crystal.

In a 100 mL flask, 24.6 g of the above-mentioned crude crystal and 35 g of tetrahydrofuran were charged under a nitrogen atmosphere, and the mixture was stirred until complete dissolution. To this solution was added 20 g of water, the resulting mixture was stirred for 10 minutes or more and was then allowed to stand at 0 to 30°C. The resulting upper aqueous layer was removed by liquid-liquid separation. The obtained organic layer was desolvated at not higher than 40°C under a reduced pressure to obtain 23.8 g of a concentrated liquid. To the resulting concentrated liquid was added 20 g of toluene, and the resulting mixture was subjected to filtration. The resulting wet crystal was washed with 20 g of toluene, and the washed wet crystal was dried to obtain 11.8 g of a solid matter containing 1,6-bis(carboxymethylthio)naphthalene (16DCNDSH) (purity by HPLC: 100%, theoretical yield: 74 mol%).

The resulting 16DCNDSH has a melting point of 176 to 178°C and a refractive index of 1.663, and shows the results of ¹H-NMR as follows.
¹H-NMR (DMSO-D6): δ (ppm) 3.87 (s, 2H), 3.95 (s, 2H), 7.44-7.56 (m, 3H), 7.71 (d, J 8.0, 1H), 7.85 (d, J 2.0, 1H), 8.14 (d, J 8.8, 1H), 12.81 (s, 2H)

### [Comparative Example 9] Synthesis of 15DCNDSH

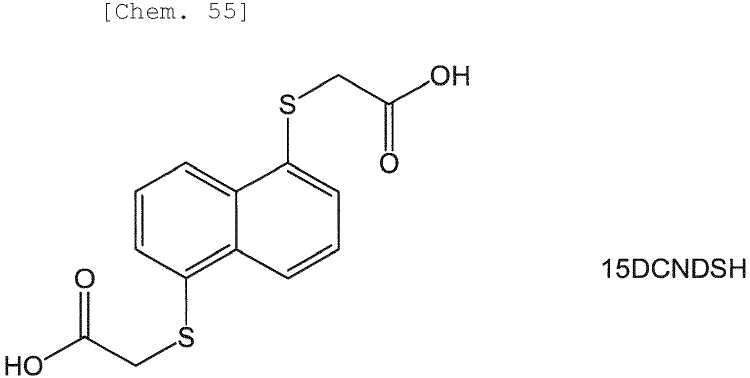

In a 100 mL flask, 39.0 g (6.0 molar ratio) of a 32% by mass sodium hydroxide aqueous solution, 85.8 g of water, and 0.1 g (0.05 molar ratio) of sodium borohydride were charged under a nitrogen atmosphere, and the mixture was stirred until complete dissolution. To the resulting aqueous solution was added 10 g (52.0 mmol) of 1,5-naphthalenedithiol (15NDSH), and the mixture was heated to 60°C. After the heating, the temperature fluctuated from 55 to 65°C, and the reaction was carried out for one hour while maintaining the temperature in a thermostatic bath. After the completion of the reaction, the reaction mixture was cooled to 30°C to obtain a 15NDSH▪Na liquid (an aqueous solution containing a sodium salt of 15NDSH).

To a 200 mL flask, 15.3 g (2.4 molar ratio) of chloroethyl acetate was added under a nitrogen atmosphere, the above-mentioned 15NDSH▪Na liquid was dropwise added thereto over 30 minutes, and the resulting mixture was stirred for 17 hours and was then heated to 75°C. After the heating, the temperature fluctuated from 75 to 80°C, and the reaction was carried out for 5 hours while maintaining the temperature in a thermostatic bath. After the completion of the reaction, the reaction mixture was cooled to 30°C, and 30 g of toluene was added thereto. The resulting mixture was stirred for one hour or more. Thereafter, 19.5 g (3.7 molar ratio) of 36% by mass hydrochloric acid was dropwise added thereto, the resulting mixture was stirred for one hour or more and was then subjected to filtration. The resulting wet crystal was washed with 40 g of water and toluene, and the washed wet crystal was dried to obtain 14.5 g of a solid matter containing 1,5-bis(carboxymethylthio)naphthalene (15DCNDSH) (purity by HPLC: 100%, theoretical yield: 90 mol%).

The resulting 15DCNDSH has a melting point of 256 to 259°C and a refractive index of 1.657, and shows the results of ¹H-NMR as follows.
¹H-NMR (DMSO-D6): δ (ppm) 3.90 (s, 4H), 7.62-7.54 (m, 4H), 8.13 (d, J 8.4, 2H), 12.82 (s, 2H)

The following Table 9 shows the evaluation results of the bis(carboxymethylthio)naphthalene compounds obtained in Example 9 and Comparative Example 9.

### [Table 9]

**Table 9**

| Bis(carboxymethylthio) naphthalene compound | | Refractive index | Dissolubility [% by mass] | | | | |
|---|---|---|---|---|---|---|---|
| | | | THF | DMF | Ethyl acetate | PGMEA | MMA |
| Example 9 | 16DCNDSH | 1.663 | 30.5 | 61.0 | 0.56 | 0.98 | 0.14 |
| Comparative Example 9 | 15DCNDSH | 1.657 | 0.67 | 10.1 | 0.03 | 0.02 | Less than 0.01 |

As apparent from the results shown in Table 9, the bis(carboxymethylthio)naphthalene compounds also showed the same results as the aforementioned examples, specifically, Example 9 (16DCNDSH) had high refractive index and high dissolubility compared with Comparative Example 9 (15DCNDSH), in particular, had a significantly high dissolubility.

### <Thiol-ene-based resin>

### [Example 10] Polymerization of 16NDSH and divinylbenzene

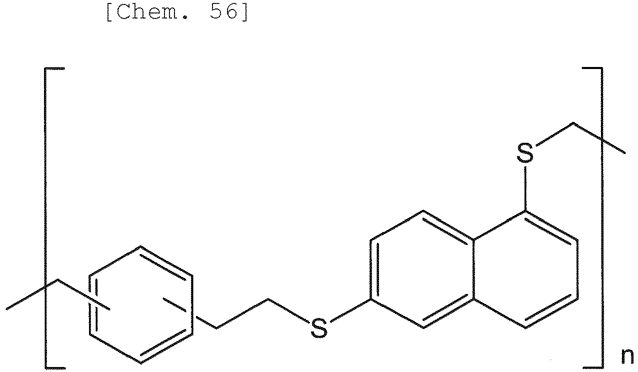

In a 10 mL screw tube, 1.9 g (0.01 mol) of 1,6-naphthalenedithiol (16NDSH) and 2.4 g (0.01 mol as divinylbenzene, refractive index: 1.558) of divinylbenzene ["55% Divinylbenzene (isomer mixture)" manufactured by FUJIFILM Wako Pure Chemical Corporation, model number: 042-22625] were charged under a nitrogen atmosphere, and the mixture was stirred at an ordinary temperature (about 25°C) for one hour. The resulting viscous colorless transparent liquid was poured into a petri dish and was dried at 80°C under a reduced pressure to obtain 4.1 g of a more viscous, colorless transparent resin. The resulting resin had a refractive index of 1.692.

### [Comparative Example 10] Polymerization of 15NDSH and divinylbenzene

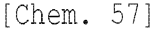

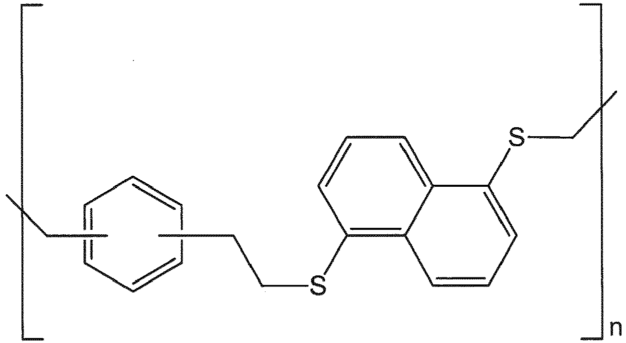

### (Comparative Example 10-1)

In a 10 mL screw tube, 1.9 g (0.01 mol) of 1,5-naphthalenedithiol (15NDSH) and 2.4 g (0.01 mol as divinylbenzene, refractive index: 1.558) of divinylbenzene ["55% Divinylbenzene (isomer mixture)" manufactured by FUJIFILM Wako Pure Chemical Corporation, model number: 042-22625] were charged under a nitrogen atmosphere, and the mixture was stirred at an ordinary temperature (about 25°C) for one hour to obtain 4.3 g of a viscous colorless opaque substance. 15NDSH had a low dissolubility, failing to obtain a transparent resin and to increase the ratio of 15NDSH for the resin to have an increased refractive index.

### (Comparative Example 10-2)

A resin containing 15NDSH at a concentration of about 5% by mass, which was a saturation solubility of 15NDSH, was prepared. Specifically, the resin was obtained in the same manner as the above-mentioned Comparative Example 10-1 except that 0.12 g of 15NDSH and 2.4 g of divinylbenzene ["55% Divinylbenzene (isomer mixture)" manufactured by FUJIFILM Wako Pure Chemical Corporation, model number: 042-22625] were used. The resin had a refractive index of 1.678. However, most of 55% by mass divinylbenzene remained unreacted. After drying at 80°C under a reduced pressure, the resulting resin weighed 0.2 g.

### (Comparative Example 10-3)

The reaction was carried out in the same manner as the above-mentioned Comparative Example 10-1 except that the stirring was carried out under heating of 70°C. A mixture of 15NDSH and divinylbenzene (an incompatible mixed slurry liquid) was once obtained as a completely dissolved colorless solution, and then the solution immediately started to turn cloudy. After 10 minutes, a viscous cloudy resin was obtained. 15NDSH was partially crystallized, probably failing to obtain a transparent resin and to increase the ratio of 15NDSH for the resin to have an increased refractive index.

### <Curable composition containing bis(methacryloylthio)naphthalene compound, and cured product thereof>

### [Example 11] Cured product of 16NDSHMA alone

On a glass formwork, 0.1 g of 1,6-bis(methacryloylthio)naphthalene (16NDSHMA) was placed, was heat-fused at 80°C, and was irradiated with light for 2 minutes using an UV-LED light source apparatus (wavelength = 365 nm, 500 mW/cm², manufactured by HAMAMATSU PHOTONICS K.K.) to give 0.1 g of a transparent curable resin (cured product). The resulting resin had a refractive index of 1.687 to 1.688.

### [Comparative Example 11] Cured product of 15NDSHMA alone

On a glass formwork, 0.1 g of 1,5-bis(methacryloylthio)naphthalene (15NDSHMA) was placed, was heat-fused at 140°C, and was irradiated with light for 2 minutes using an UV-LED light source apparatus (wavelength = 365 nm, 500 mW/cm², manufactured by HAMAMATSU PHOTONICS K.K.) to give 0.1 g of a transparent curable resin (cured product). The resulting resin had a refractive index of 1.687 to 1.688.

### [Example 12] Curable composition containing 16NDSHMA-styrene, and cured product thereof

In a 10 mL brown screw tube, 0.05 g (0.15 mmol) of 1,6-bis(methacryloylthio)naphthalene (16NDSHMA) and 0.05 g of styrene (0.48 mmol, refractive index: 1.544) were charged, and the mixture was stirred at an ordinary temperature (about 25°C) for one minute. The resulting colorless solution (curable composition) was poured into a glass formwork and was irradiated with light for 2 minutes using an UV-LED light source apparatus (wavelength = 365 nm, 500 mW/cm², manufactured by HAMAMATSU PHOTONICS K.K.) to obtain 0.1 g of a transparent curable resin (cured product). The resulting resin had a refractive index of 1.651 to 1.653.

### [Comparative Example 12] Curable composition containing 15NDSHMA-styrene, and cured product thereof

### (Comparative Example 12-1)

In a 10 mL brown screw tube, 0.05 g (0.15 mmol) of 1,5-bis(methacryloylthio)naphthalene (15NDSHMA) and 0.05 g of styrene (0.48 mmol) were charged, and the mixture was stirred at an ordinary temperature (about 25°C) for one minute. The resulting cloudy liquid was poured into a glass formwork and was irradiated with light for 10 minutes using an UV-LED light source apparatus (wavelength = 365 nm, 500 mW/cm², manufactured by HAMAMATSU PHOTONICS K.K.) to obtain 0.1 g of an opaque curable resin. 15NDSHMA had a low dissolubility and was incompatible and phase-separated, probably failing to obtain a transparent resin and to increase the ratio of the 15NDSHMA for the resin to have an increased refractive index.

### (Comparative Example 12-2)

A curable resin containing 15NDSHMA at a concentration of about 10% by mass, which was a saturation solubility of 15NDSHMA, was prepared. Specifically, the curable resin was prepared in the same manner as the above-mentioned Comparative Example 12-1 except that 0.05 g (0.15 mmol) of 15NDSHMA and 0.5 g (4.8 mmol) of styrene were used. Although the curable resin was transparent, the curable resin had a refractive index of as low as 1.621 to 1.623.

### <Epoxy resin curing agent, curable composition containing the same, and cured product thereof>

### [Example 13] Curable composition containing 16NDSH-epoxy resin, and cured product thereof

In a formwork made of an aluminum foil, 1.11 g (6 mmol as epoxy group, refractive index: 1.573) of a bisphenol A-type liquid epoxy resin ("828" manufactured by Mitsubishi Chemical Corporation, epoxy equivalent = about 185) and 0.58 g (6 mmol as mercapto group) of 1,6-naphthalenedithiol (16NDSH) as a curing agent were charged and were mixed at 40°C. The mixture was cooled to an ordinary temperature (about 25°C), and 0.066 g (0.6 mmol, refractive index: 1.499) of 2-ethyl-4-methylimidazole as a curing accelerator was mixed therewith, thus preparing a curable composition. The curable composition was allowed to stand for 30 minutes to obtain 1.75 g of a transparent curable resin (cured product). The resulting resin had a refractive index of 1.659 to 1.670.

### [Comparative Example 13] Curable composition containing 15NDSH-epoxy resin, and cured product thereof

### (Comparative Example 13-1)

In a formwork made of an aluminum foil, 1.11 g (6 mmol as epoxy group, refractive index: 1.573) of a bisphenol A-type liquid epoxy resin ("828" manufactured by Mitsubishi Chemical Corporation, epoxy equivalent = about 185) and 0.58 g (6 mmol as mercapto group) of 1,5-naphthalenedithiol (15NDSH) as a curing agent were charged and were mixed at 40°C. The mixture was cooled to an ordinary temperature (about 25°C), and 0.066 g (0.6 mmol, refractive index: 1.499) of 2-ethyl-4-methylimidazole as a curing accelerator was mixed therewith, thus preparing a curable composition. The curable composition was allowed to stand for 30 minutes to obtain 1.75 g of a translucent curable resin. 15NDSH had a low dissolubility in the bisphenol A-type liquid epoxy resin and thus remained unreacted, probably failing to obtain a transparent resin and to increase the ratio of 15NDSH for the resin to have an increased refractive index.

### (Comparative Example 13-2)

A curable composition containing 15NDSH at a concentration of about 0.1% by mass, which was a saturation solubility of 15NDSH, was prepared. Specifically, the curable composition was prepared in the same manner as the above-mentioned Comparative Example 13-1 except that 1.11 g (6 mmol as epoxy group) of a bisphenol A-type liquid epoxy resin, 0.012 g (0.12 mmol as mercapto group) of 15NDSH, and 0.066 g (0.6 mmol) of 2-ethyl-4-methylimidazole were used. Although the curable composition was transparent, the curable composition remained viscous liquid without curing probably because of an excessively small amount of the curing agent and had a refractive index of as low as 1.560.

### <Refractive index increasing agent, and resin composition containing the same>

### [Example 14] Resin composition containing 16DMNDSH

In a 10 mL screw tube, 0.2 g (refractive index: 1.488 to 1.489) of a polymethyl methacrylate (PMMA), 0.2 g of 1,6-bis(methylthio)naphthalene (16DMNDSH), and 2.0 g of tetrahydrofuran (THF) were charged, and the mixture was stirred at 60°C for 30 minutes. The resulting viscous liquid was poured into a formwork made of an aluminum foil and was dried at 60°C for one hour under a reduced pressure, and was then cooled to a room temperature (about 25°C) to obtain 0.4 g of a transparent resin composition. The resulting resin composition had a refractive index of 1.621 to 1.624.

### [Comparative Example 14] Resin composition containing 15DMNDSH

### (Comparative Example 14-1)

In a 10 mL screw tube, 0.2 g (refractive index: 1.488 to 1.489) of a polymethyl methacrylate (PMMA), 0.2 g of 1,5-bis(methylthio)naphthalene (15DMNDSH), and 2.0 g of tetrahydrofuran (THF) were charged, and the mixture was stirred at 60°C for 30 minutes. The resulting viscous liquid was poured into a formwork made of an aluminum foil and was dried at 60°C for one hour under a reduced pressure, and was then cooled to a room temperature (about 25°C) to obtain 0.4 g of an opaque resin composition. 15DMNDSH has a low dissolubility, precipitating and failing to obtain a transparent resin composition and to increase the ratio of 15DMNDSH for the resin to have an increased refractive index.

### (Comparative Example 14-2)

A resin composition containing 15DMNDSH at a concentration of about 10% by mass, which was a saturation solubility of 15DMNDSH, was prepared. Specifically, the resin composition was prepared in the same manner as the above-mentioned Comparative Example 14-1 except that 0.2 g of PMMA, 0.02 g of 15DMNDSH, and 2.0 g of THF were used. Although the resin composition was transparent, the resin composition had a refractive index of as low as 1.531 to 1.538.

### INDUSTRIAL APPLICABILITY

The 1,6-naphthalenedithiol and the derivative thereof according to the present invention have both high dissolubility and high refractive index and are used for various uses. For example, the 1,6-naphthalenedithiol and the derivative thereof can be used for an optical material [such as a display material such as an anti-reflective film (such as an index matching film), and an additive (or a resin additive) such as a refractive index increasing agent, a fluorescence quencher, a crosslinking agent, or a crosslinking co-agent], an electric and electronic material (such as a resist material such as a resist under-layer, and an organic semiconductor material), and a coating material or a coat material (such as a coating composition for forming a hardcoat layer and/or others), and can also be used as a raw material or a reaction intermediate, for example, a resin raw material or a monomer component (such as a monomer component having a high refractive index). As examples of the resin which can be prepared from the above-mentioned resin raw material or monomer component, there may be mentioned a thermosetting or photo-curable resin such as a (meth)acrylic resin, an epoxy-series resin, and a diallyl-series resin; and a thermoplastic resin such as a polyester-series resin (such as a polyester resin, a polyester carbonate resin, a polycarbonate resin, and a polythioester resin), a polyether-series resin, a polysulfide-series resin (such as a polyaddition product by thiol-ene reaction), and a polyurethane-series resin (such as a polyurethane resin and a polythiourethane resin).

## Claims

1. A naphthalene compound represented by the following formula (1): 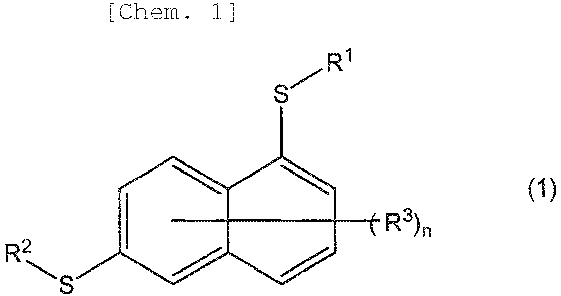
wherein R¹ and R² independently represent a hydrogen atom or a substituent, and
R³ represents a substituent, and n denotes an integer of 0 to 6,
wherein at least one of R¹ and R² represents the substituent.

2. The naphthalene compound according to claim 1, wherein, in the formula (1), each of R¹ and R² represents at least one member selected from the group consisting of a hydrogen atom, a substituted or unsubstituted hydrocarbon group, and a group containing an element in group 16 of the Periodic Table, and
R³ represents a hydrocarbon group, and n denotes an integer of 0 to 3.

3. The naphthalene compound according to claim 1 or 2, wherein, in the formula (1), at least one of R¹ and R² represents at least one member selected from the group consisting of the groups defined by the following formulae (2B) to (2G): 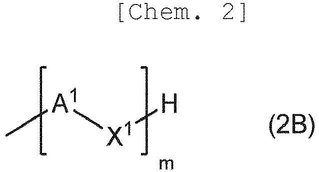
wherein A¹ represents an alkylene group, X¹ represents an oxygen atom or a sulfur atom, and m denotes 0 or an integer of not less than 1; 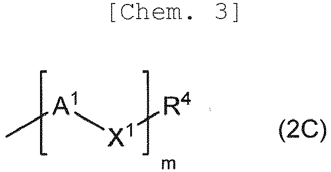
wherein A¹ represents an alkylene group, X¹ represents an oxygen atom or a sulfur atom, and m denotes 0 or an integer of not less than 1, and
R⁴ represents a saturated hydrocarbon group which may have a halogen atom; 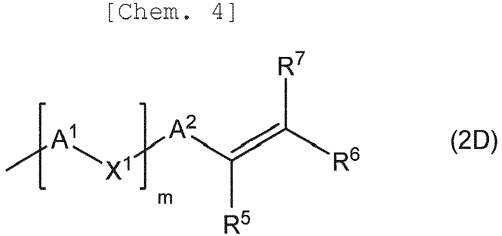
wherein A⁴ represents an alkylene group, X⁴ represents an oxygen atom or a sulfur atom, and m denotes 0 or an integer of not less than 1,
A² represents a direct bond or an alkylene group, and
R⁵, R⁶ and R⁷ independently represent a hydrogen atom, or a substituted or unsubstituted hydrocarbon group; 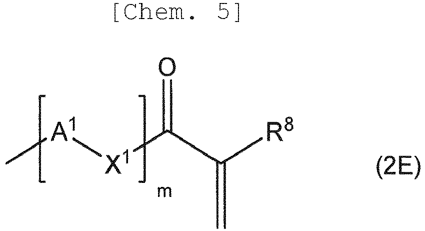
wherein A¹ represents an alkylene group, X¹ represents an oxygen atom or a sulfur atom, and m denotes 0 or an integer of not less than 1, and
R⁸ represents a hydrogen atom or a methyl group; 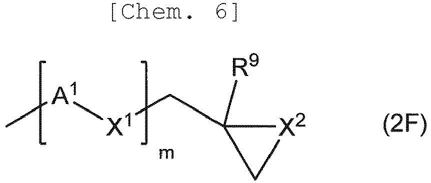
wherein A¹ represents an alkylene group, X¹ represents an oxygen atom or a sulfur atom, and m denotes 0 or an integer of not less than 1,
X² represents an oxygen atom or a sulfur atom, and
R⁹ represents a hydrogen atom or a methyl group; 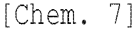 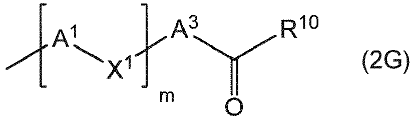
wherein A¹ represents an alkylene group, X¹ represents an oxygen atom or a sulfur atom, and m denotes 0 or an integer of not less than 1,
A³ represents an alkylene group, and
R¹⁰ represents a hydroxyl group, a group [-OR¹¹] (wherein R¹¹ represents a substituted or unsubstituted hydrocarbon group), or a halogen atom.

4. The naphthalene compound according to claim 1 or 2, wherein, in the formula (1), at least one of R¹ and R² represents at least one member selected from the group consisting of a hydrogen atom, an alkyl group, a haloalkyl group, an alkenyl group, (meth)acryloyl group, a (meth)acryloyloxyalkyl group, a glycidyl group, a β-methylglycidyl group, a glycidyloxyalkyl group, a β-methylglycidyloxyalkyl group, a hydroxyalkyl group, a carboxyalkyl group, an alkoxycarbonylalkyl group, and a halocarbonylalkyl group, and
R³ represents an alkyl group, and n denotes an integer of 0 to 2.

5. The naphthalene compound according to claim 1 or 2, which has a refractive index of not less than 1.6 at a temperature of 25°C and a wavelength of 589 nm.

6. The naphthalene compound according to claim 1 or 2, which is dissolvable at a concentration of not less than 20% by mass at a temperature of 25°C in at least one organic solvent selected from the group consisting of toluene, tetrahydrofuran, propylene glycol monomethyl ether acetate, ethyl acetate, and N,N-dimethylformamide.

7. A process for producing a naphthalene compound recited in claim 1 or 2, the process comprising a step of replacing, with the substituent, at least one hydrogen atom of mercapto groups on 1-position and 6-position of a naphthalene compound represented by the following formula (1A): 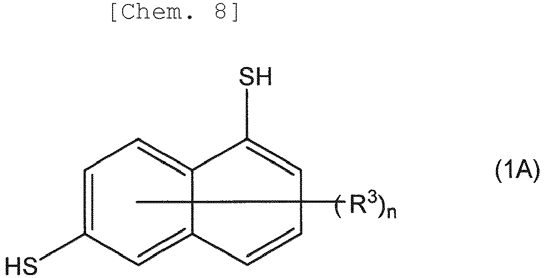 wherein R³ and n each have the same meanings as the formula (1).

8. A mixture containing a naphthalene compound represented by the following formula (1): 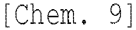 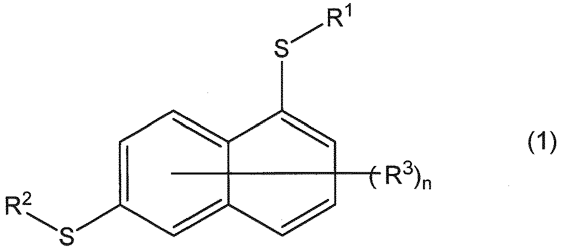
wherein R¹ and R² independently represent a hydrogen atom or a substituent, and
R³ represents a substituent, and n denotes an integer of 0 to 6,
and an organic solvent.

9. The mixture according to claim 8, wherein the organic solvent contains at least one member selected from the group consisting of a hydrocarbon, a halogenated hydrocarbon, an alcohol, an ether, a glycol ether acetate, an ester, an amide, and a urea.

10. The mixture according to claim 8 or 9, wherein the naphthalene compound has a concentration of not less than 15% by mass relative to the whole mixture.

11. A curable composition containing a naphthalene compound represented by the following formula (1): 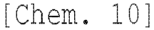 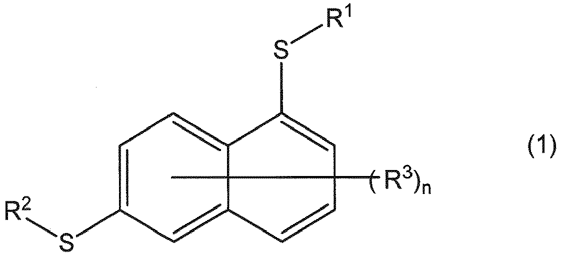
wherein R¹ and R² independently represent a hydrogen atom or a substituent, and
R³ represents a substituent, and n denotes an integer of 0 to 6.

12. The curable composition according to claim 11, which contains the naphthalene compound in which at least one of R¹ and R² in the formula (1) represents a group defined by a formula (2D), (2E), or (2F) recited in claim 3.

13. The curable composition according to claim 11 or 12, which contains
the naphthalene compound in which at least one of R¹ and R² in the formula (1) represents at least one member selected from the group consisting of an alkenyl group, (meth)acryloyl group, a (meth)acryloyloxyalkyl group, a glycidyl group, a β-methylglycidyl group, a glycidyloxyalkyl group, and a β-methylglycidyloxyalkyl group, and
a polymerizable compound having at least one ethylenically unsaturated bond.

14. The curable composition according to claim 11, which at least contains
the naphthalene compound in which both R¹ and R² in the formula (1) represent a group defined by a formula (2B) or formula (2G) recited in claim 3, and
an epoxy resin.

15. The curable composition according to any one of claims 11, 12, and 14, wherein the naphthalene compound has a concentration of not less than 25% by mass relative to the whole curable composition.

16. A cured product of a curable composition recited in any one of claims 11, 12, and 14.

17. A cured product of a curable composition recited in claim 13.

18. A curing agent for curing an epoxy resin, which is represented by the following formula (1): 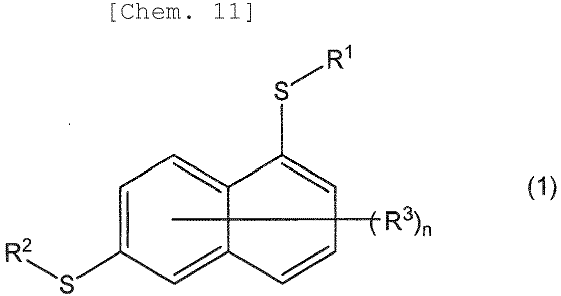
wherein R¹ and R² independently represent a group defined by a formula (2B) or formula (2G) recited in claim 3, and
R³ represents a substituent, and n denotes an integer of 0 to 6.

19. A resin at least containing a constituent unit represented by the following formula (1P): 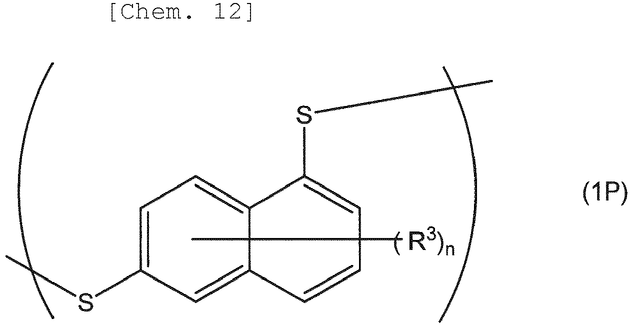 wherein R³ represents a substituent, and n denotes an integer of 0 to 6.

20. The resin according to claim 19, which is a thermoplastic resin.

21. The resin according to claim 19 or 20, which is a polymer of a polymerizable component at least containing a compound represented by the following formula (1): 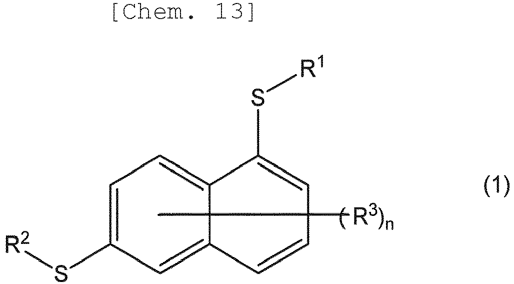
wherein R¹ and R² independently represent a group defined by a formula (2B), (2D), (2E), (2F), or (2G) recited in claim 3, and
R³ represents a substituent, and n denotes an integer of 0 to 6,
wherein the polymerizable component is any of the following polymerizable components (a) to (c):
(a) a polymerizable component containing a dithiol component and a component having two ethylenically unsaturated bonds;
wherein the dithiol component contains the compound in which each of R¹ and R² in the formula (1) represents a group defined by the formula (2B), where X¹ represents a sulfur atom, and/or
the component having two ethylenically unsaturated bonds contains the compound in which each of R¹ and R² in the formula (1) represents a group defined by the formula (2D) or (2E),
(b) a polymerizable component containing a diol component and/or a dithiol component, and at least one component selected from the group consisting of a dicarboxylic acid component, a diisocyanate component, and a carbonyl component;
wherein the diol component or the dithiol component contains the compound in which each of R¹ and R² in the formula (1) represents a group defined by the formula (2B), and/or
the dicarboxylic acid component contains the compound in which each of R¹ and R² in the formula (1) represents a group defined by the formula (2G),
(c) a polymerizable component containing
a diol component and/or a dithiol component containing the compound in which each of R¹ and R² in the formula (1) represents a group defined by the formula (2B), and
an epihalohydrin component, or
a polymerizable component containing
an epoxy component containing the compound in which each of R¹ and R² in the formula (1) represents a group defined by the formula (2F), and
a bisphenol component.

22. A method for improving a dissolubility or compatibility of a naphthalene compound in an organic compound, the naphthalene compound having sulfur atoms directly bonded to a naphthalene ring thereof at least at two positions, wherein the improvement comprises including the sulfur atoms at least at the 1,6-positions.

23. A resin composition at least containing a compound represented by the following formula (1): 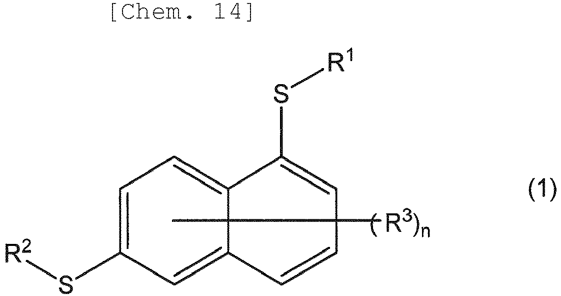
wherein R¹ and R² independently represent a hydrogen atom or a substituent, and
R³ represents a substituent, and n denotes an integer of 0 to 6,
and a resin.

24. A refractive index increasing agent for increasing a refractive index, which is represented by the following formula (1): 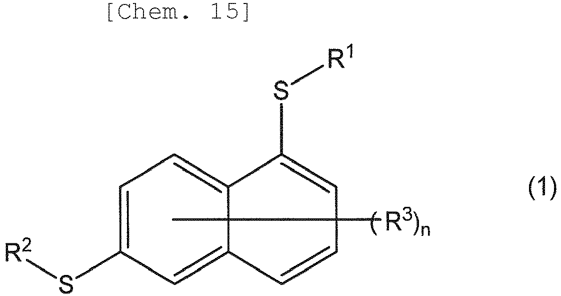
wherein R¹ and R² independently represent a hydrogen atom or a substituent, and
R³ represents a substituent, and n denotes an integer of 0 to 6.

25. A method for increasing a refractive index, which comprises adding a refractive index increasing agent recited in claim 24 to a resin.
